# EUROPÄISCHE PATENTSCHRIFT

(11) **EP 1 751 118 B1**
(45) Veröffentlichungstag und Bekanntmachung des Hinweises auf die Patenterteilung: **03.12.2014**
(21) Anmeldenummer: 05747883.6
(22) Anmeldetag: 13.05.2005
(51) Int. Cl.: C07D 239/48, A61K 31/505, A61P 29/00, A61P 31/12, A61P 35/00, A61P 37/00

(54) **PYRIMIDINE ALS PLK INHIBITOREN**
PYRIMIDINES FOR USE AS PLK INHIBITORS
PYRIMIDINES COMME INHIBITEURS DE PLK

(30) Priorität: 19.05.2004 EP 04011911
(43) Veröffentlichungstag der Anmeldung: 14.02.2007
(73) Patentinhaber: Boehringer Ingelheim International GmbH, 55216 Ingelheim (DE); Boehringer Ingelheim Pharma GmbH & Co. KG, 55216 Ingelheim (DE)
(72) Erfinder: STADTMUELLER, Heinz, 1120 Wien (AT); ENGELHARDT, Harald, A-2483 EBREICHSDORF (AT); REISER, Ulrich, A-1130 WIEN (AT); ZAHN, Stephan, Karl, A-1130 WIEN (AT); HAUPTMANN, Rudolf, A-2483 EBREICHSDORF (AT); STEEGMAIER, Martin, 72762 Reutlingen (DE); GUERTLER, Ulrich, A-1120 WIEN (AT); HOFFMANN, Matthias, 88441 Mittelbiberach (DE); GRAUERT, Matthias, 88400 Biberach (DE)
(74) Vertreter: Simon, Elke Anna Maria
(86) Internationale Anmeldenummer: PCT/EP2005/052214
(87) Internationale Veröffentlichungsnummer: WO 2005/113515

(56) Entgegenhaltungen:
- WO-A-00/12485
- WO-A-00/27825
- WO-A1-2004/080980
- US-B1- 6 593 326

## Beschreibung

Die vorliegende Erfindung betrifft neue Pyrimidine der allgemeinen Formel (1) 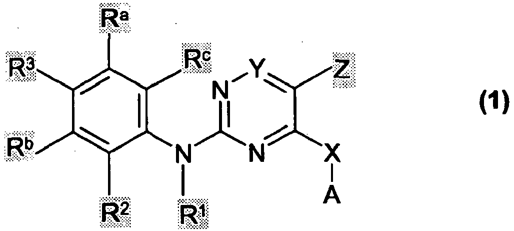 wobei die Reste A, X, Y, Z, R^{a}, R^{b}, R^{c}, R¹, R² und R³ die in den Ansprüchen und der Beschreibung genannte Bedeutung haben, deren Isomere, Verfahren zur Herstellung dieser Pyrimidine sowie deren Verwendung als Arzneimittel.

### Hintergrund der Erfindung

Tumorzellen entziehen sich teilweise oder völlig der Regulation und Kontrolle durch den Organismus und zeichnen sich durch ein unkontrolliertes Wachstum aus. Dies beruht einerseits auf dem Verlust von Kontroll-Proteinen, wie z.B. Rb, p16, p21 und p53 als auch auf der Aktivierung von so genannten Beschleunigern des Zellzykluses, den cyclin-abhängigen Kinasen (CDK's).

Studien in Modellorganismen wie *Schizosaccharomyces pombe, Drosophila melanogaster* oder *Xenopus* sowie Untersuchungen in menschlichen Zellen haben gezeigt, dass der Übergang von der G2 Phase zur Mitose durch die CDK1/Cyclin B Kinase reguliert wird (Nurse, 1990). Diese Kinase, die auch als "mitosis promoting factor" (MPF) bezeichnet wird, phosphoryliert und reguliert eine Vielzahl von Proteinen, wie z.B. nukleäre Lamine, Kinesin-ähnliche Motorproteine, Kondensine und Golgi Matrix Proteine, die eine wichtige Rolle beim Abbau der Kernhülle, bei der Zentrosomen Separation, dem Aufbau des mitotischen Spindelapparates, der Chromosomen Kondensation und Abbau des Golgi Apparates spielen (Nigg, 2001). Eine murine Zell-Linie mit einer temperatursensitiven CDK-1 Kinasemutante zeigt nach Temperaturerhöhung einen raschen Abbau der CDK-1 Kinase und einen darauf folgenden Arrest in der G2/M Phase (Th'ng et al., 1990). Die Behandlung von humanen Tumorzellen mit Inhibitoren gegen CDK1/Cyclin B, wie z.B. Butyrolacton, führt zu einem Arrest in der G2/M Phase und anschließender Apoptose (Nishio, et al. 1996).

Darüber hinaus wird auch für die Proteinkinase Aurora B eine essentielle Funktion beim Eintritt in die Mitose beschrieben. Aurora B phosphoryliert Histon H3 an Ser10 und leitet damit die Chromosomenkondensation ein (Hsu, J.Y. et al., 2000). Ein spezifischer Zellzyklusarrest in der G2/M Phase kann aber auch z.B. durch Inhibition von spezifischen Phosphatasen wie z.B. Cdc25C (Russell and Nurse, 1986) ausgelöst werden. Hefen mit defektem Cdc25 Gen arretieren in der G2 Phase, während eine Überexpression von Cdc25 zu einem verfrühten Eintritt in die Mitosephase führt (Russell und Nurse, 1987). Desweiteren kann ein Arrest in der G2/M Phase auch durch Inhibition von bestimmten Motorproteinen, den so genannten Kinesinen wie z.B. Eg5 (Mayer et al., 1999), oder durch Mikrotubuli stabilisierende oder destabilisierende Agentien (z.B. Colchicin, Taxol, Etoposid, Vinblastin, Vincristin) ausgelöst werden (Schiff und Horwitz, 1980).

Neben den Cyclin-abhängigen und den Aurora Kinasen spielen des weiteren die so genannten Polo-like Kinasen (PLK), eine kleine Familie von Serin/Threonin-Kinasen, eine wichtige Rolle bei der Regulation des eukaryontischen Zellzykluses. Besonders für PLK-1 wurde eine zentrale Rolle in der Regulation der Mitosephase gezeigt. PLK-1 ist für die Reifung der Zentrosomen, für die Aktivierung der Phosphatase Cdc25C, sowie für die Aktivierung des Anaphase Promoting Complex verantwortlich (Glover et al., 1998, Qian, et al., 2001). Die Injektion von PLK-1 Antikörpern führt zu einem G2 Arrest in nicht transformierten Zellen, während Tumorzellen in der Mitosephase arretieren (Lane und Nigg, 1996). Überexpression von PLK-1 konnte für verschiedene Tumorarten, wie nicht kleinzelliges Lungenkarzinom, Plattenepithelkarzinom, Brust- und kolorektales Karzinom gezeigt werden. Daher stellt diese Klasse von Proteinen ebenfalls einen interessanten Angriffspunkt zur therapeutischen Intervention proliferativer Krankheiten dar (Descombes und Nigg, Embo J, 17; 1).

Pyrimidine sind allgemein als Inhibitoren von Kinasen bekannt. So werden z.B. Pyrimidine als aktive Komponente mit Antikrebswirkung in der Internationalen Patentanmeldung WO 00/53595, in der die Verwendung von 2,4,5-substituierten Pyrimidinen mit einem heterozyklischen Rest in 4-Position und einem Anilinorest in 2-Position, der seinerseits eine Seitenkette mit der Länge mindestens eines n-Propylrests aufweist, beschrieben.

Weiterhin wird in der Internationalen Patentanmeldung WO 00/39101 die Verwendung von 2,4,5-substituierten Pyrimidinen als Verbindungen mit Antikrebs Wirkung vorgeschlagen, welche in der 2- und 4-Position mit einem aromatischen oder heteroaromatischen Ring verknüpft sind, von denen mindestens einer eine Seitenkette mit der Länge mindestens eines n-Propylrests aufweist.

Die Internationale Patentanmeldung WO 97/19065 schlägt weiterhin die Verwendung von 2,4,5-substituierten Pyrimidinen mit einem 3,4-Dialkoxyanilinorest in Position 2 als Kinaseinhibitoren vor.

Die Internationale Patentanmeldung WO 02/04429 beschreibt 2,4,5-substituierte Pyrimidine mit einer Cyanogruppe in Position 5 und deren Zellzyklus inhibierenden Effekt.

In der Internationalen Patentanmeldung WO 03/063794 wird die Verwendung von 2,4-Pyrimidindiaminen als Inhibitoren der IgE und/oder IgG Rezeptor Signalkaskade beschrieben.

Antivirale 2,4,5-substituierte Pyrimidine, in denen die Reste R^{c} und R^{d} an dem Stickstoff der 4- Position einen heteroaromatischen Fünfring bilden, sind aus der Internationalen Patentanmeldung WO 99/41253 bekannt.

Für 2,4,5-substituierte Pyrimidine, die in Position 2 und 4 (Hetero-)Aryle tragen (WO00/27825), sowie für 2, 4, 5-substituierten Pyrimidinen, die in Position 2 oder 4 einen mit einer Nitrilgruppe funktionalisierten (Hetero-)Arylrest tragen (EP 0 945 443 A1), wird eine antivirale Wirkung beschrieben.

Der vorliegenden Erfindung liegt die Aufgabe zugrunde, neue Wirkstoffe aufzuzeigen, welche zur Vorbeugung und/oder Behandlung von Krankheiten, die durch exzessive oder anomale Zellproliferation charakterisiert sind, eingesetzt werden können.

### Detaillierte Beschreibung der Erfindung

Es wurde nun überraschenderweise gefunden, dass Verbindungen der allgemeinen Formel **(1),** worin die Reste **A, X, Y, R^{a}**, **R^{b}**, **R^{c}**, **R¹**, **R²** und **R³** die nachstehend genannten Bedeutungen haben, als Inhibitoren spezifischer Zellzykluskinasen wirken. Somit können die erfindungsgemäßen Verbindungen beispielsweise zur Behandlung von Erkrankungen, die mit der Aktivität spezifischer Zellzykluskinasen in Zusammenhang stehen und durch exzessive oder anomale Zellproliferation charakterisiert sind, verwendet werden.

Die vorliegende Erfindung betrifft Verbindungen der allgemeinen Formel (1) 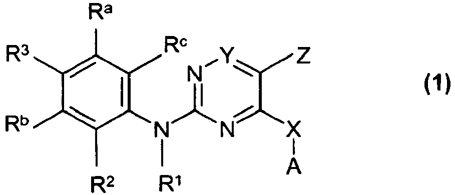 worin
- **X**: -NR^{1a}, O oder S, und
- **Y**: CH und
- **Z**: C₁₋₃-Fluoralkyl- und
- **A**: ausgewählt ist aus den Formeln **(i)** oder **(ii)** 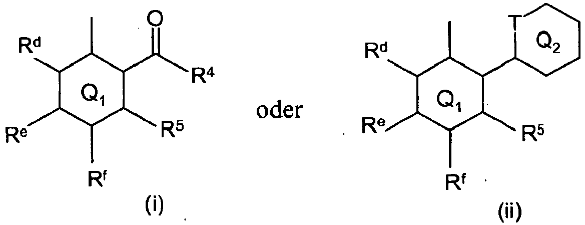 und
- **Q₁**: mono- oder bizyklische Arylverbindungen; und
- **Q₂**: mono- oder bizyklische Heteroarylverbindungen; und
- T: N, O oder S, und
- **R¹** und **R^{1a}**: Wasserstoff oder Methyl, und
- **R²**: ein Rest ausgewählt aus der Gruppe bestehend aus -Cl, -Br, -I, -OR⁶, -C(=O)R⁶, -C(=O)NR⁶R⁷, -NR⁶R⁷, -NR⁶C(=O)R⁷, -NR⁶SO₂R⁷, -N=CR⁶R⁷, -SR⁶, -SOR⁶, -SO₂R⁶, -SO₂NR⁶R⁷ und Pseudohalogen, oder ein gegebenenfalls einfach oder mehrfach substituierter Rest ausgewählt aus der Gruppe bestehend aus C₁₋₆-Alkyl, C₂₋₆-Alkenyl, C₂₋₆-Alkinyl, C₃₋₆-Cycloalkyl, Aryl, Heterocyclyl und Heteroaryl, wobei der/die Substituenten gleich oder verschieden sein können und ausgewählt werden aus der Gruppe bestehend aus Halogen, -NO₂, -OR⁶, -C(=O)R⁶, -C(=O)OR⁶, -C(=O)NR⁶R⁷, -NR⁶R⁷, -NR⁶C(=O)R⁷, -NR⁶C(=O)OR⁷, -NR⁶C(=O)NR⁷R⁸, -NR⁶SO₂R⁷, -N=CR⁶R⁷, -SR⁶, -SOR⁶, -SO₂R⁶, -SO₂NR⁶R⁷, -NR⁶SO₂NR⁷R⁸, -OSO₂NR⁷R⁸ und Pseudohalogen; und
- **R^{a}**, **R^{b}**, **R^{c}, R^{d}, R^{e}** und **R^{f}**: jeweils unabhängig voneinander ein Rest ausgewählt aus der Gruppe bestehend aus Wasserstoff, Halogen, -NO₂, -OR⁶, -C(=O)R⁶, -C(=O)OR⁶, -C(=O)NR⁶R⁷, -NR⁶R⁷, -NR⁶C(=O)R⁷, -NR⁶C(=O)OR⁷, -NR⁶C(=O)NR⁷R⁸, -NR⁶SO₂R⁷, -N=CR⁶R⁷, -SR⁶, -SOR⁶, -SO₂R⁶, -SO₂NR⁶R⁷, -NR⁶SO₂NR⁷R⁸, -OSO₂NR⁷R⁸ und Pseudohalogen;
oder ein gegebenenfalls einfach oder mehrfach substituierter Rest ausgewählt aus der Gruppe bestehend aus C₁₋₆-Alkyl, C₂₋₆-Alkenyl, C₂₋₆-Alkinyl, C₃₋₆-Cycloalkyl, Aryl, Heterocyclyl und Heteroaryl, wobei der/die Substituenten gleich oder verschieden sein können und ausgewählt werden aus der Gruppe bestehend aus Wasserstoff, Halogen, -NO₂, -OR⁶, -C(=O)R⁶, -C(=O)OR⁶, -C(=O)NR⁶R⁷, -NR⁶R⁷, -NR⁶C(=O)R⁷, -NR⁶C(=O)OR⁷, -NR⁶C(=O)NR⁷R⁸, -NR⁶SO₂R⁷, -N=CR⁶R⁷, -SR⁶, -SOR⁶, -SO₂R⁶, -SO₂NR⁶R⁷, -NR⁶SO₂NR⁷R⁸, -OSO₂NR⁷R⁸ und Pseudohalogen; und
- **R³**: ausgewählt aus den Formeln **(iii)** - **(ix),** 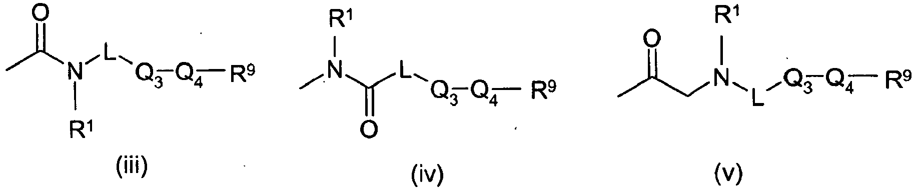 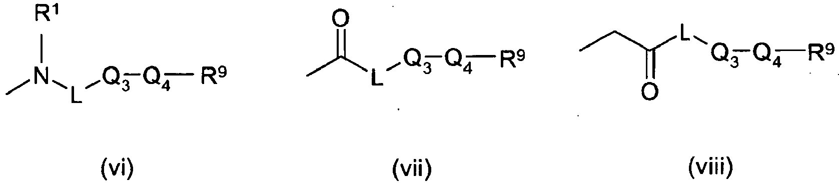 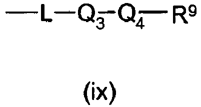 und
- **R⁴**: ein Rest ausgewählt aus der Gruppe bestehend aus Wasserstoff, -OR⁶, -C(=O)R⁶, -C(=O)OR⁶, -C(=O)NR⁶R⁷, -NR⁶R⁷, -NR⁶C(=O)R⁷, -NR⁶C(=O)OR⁷, -NR⁶C(=O)NR⁷R⁸, -NR⁶SO₂R⁷, -N=CR⁶R⁷, -SR⁶, -SOR⁶, -SO₂R⁶, -SO₂NR⁶R⁷, -NR⁶SO₂NR⁷R⁸, -OSO₂NR⁷R⁸ und Pseudohalogen, oder ein Rest ausgewählt aus der Gruppe bestehend aus gegebenenfalls einfach oder mehrfach substituiertem C₁₋₈-Alkyl, C₂₋₁₀-Alkenyl, C₂₋₁₀-Alkinyl, C₃₋₈-Cycloalkyl, Aryl, Heterocyclyl und Heteroaryl, wobei der/die Substituent(en) gleich oder verschieden sein können und ausgewählt werden aus der Gruppe bestehend aus C₁₋₈-Alkyl, Halogen, -NO₂, -OR⁶, -C(=O)R⁶, -C(=O)OR⁶, -C(=O)NR⁶R⁷, -NR⁶R⁷, -NR⁶C(=O)R⁷, -NR⁶C(=O)OR⁷, -NR⁶C(=O)NR⁷R⁸, -NR⁶SO₂R⁷, -N=CR⁶R⁷, -SR⁶, -SOR⁶, -SO₂R⁶, -SO₂NR⁶R⁷, -NR⁶SO₂NR⁷R⁸, -OSO₂NR⁷R⁸ und Pseudohalogen; und
- **R⁵**: Wasserstoff, Halogen, -CF₃, C₁₋₃-Alkyl oder -OR⁶; und
- **R⁶**, **R⁷** und **R⁸**: jeweils unabhängig voneinander Wasserstoff oder ein Rest ausgewählt aus der Gruppe bestehend aus gegebenenfalls einfach oder mehrfach substituiertem C₁₋₅-Alkyl, C₂₋₅-Alkenyl, C₂₋₅-Alkinyl, C₃₋₁₀-Cycloalkyl, Aryl, Heterocyclyl und Heteroaryl, wobei der/die Substituenten gleich oder verschieden sein können und ausgewählt werden aus der Gruppe bestehend aus C₃₋₁₀-Cycloalkyl, Aryl, Heterocyclyl, Heteroaryl, Halogen, -NO₂, -OR¹⁰, -C(=O)R¹⁰, -C(=O)OR¹⁰, -C(=O)NR¹⁰R¹¹, -NR¹⁰R¹¹, -NR¹⁰C(=O)R¹¹, -NR¹⁰C(=O)OR¹¹, -NR¹⁰C(=O)NR¹¹R¹², -NR¹⁰C(=O)ONR¹¹R¹², -NR¹⁰SO₂R¹¹, -N=CR¹⁰R¹¹, -SR¹⁰, -SOR¹⁰, -SO₂R¹⁰, -SO₂NR¹⁰R¹¹, -NR¹⁰SO₂NR¹¹R¹², -OSO₂NR¹⁰R¹¹ und Pseudohalogen; und
- **L**: eine Bindung oder ein Rest ausgewählt aus der Gruppe bestehend aus gegebenenfalls einfach oder mehrfach substituiertem C₁₋₁₆-Alkyl, C₂₋₁₆-Alkenyl, C₂₋₁₆-Alkinyl, C₃₋₁₀-Cycloalkyl, Aryl, Heterocyclyl und Heteroaryl, wobei der/die Substituent(en) gleich oder verschieden sein können und ausgewählt werden aus der Gruppe bestehend aus Halogen, -NO₂, -OR¹⁰, -C(=O)R¹⁰, -C(=O)OR¹⁰, -C(=O)NR¹⁰R¹¹, -NR¹⁰R¹¹, -NR¹⁰COR¹¹, -NR¹⁰C(=O)OR¹¹, -NR¹⁰C(=O)NR¹¹R¹², -NR¹⁰C(=O)ONR¹¹R¹², -NR¹⁰SO₂R¹¹, -N=CR¹⁰R¹¹, -SR¹⁰, -SOR¹⁰, -SO₂R¹⁰, -SO₂NR¹⁰R¹¹, -NR¹⁰SO₂NR¹¹R¹², -OSO₂NR¹⁰R¹¹ und Pseudohalogen; und
- **Q₃** und **Q₄**: unabhängig voneinander eine Bindung oder ein Rest ausgewählt aus der Gruppe bestehend aus gegebenenfalls einfach oder mehrfach substitutierten mono-oder bizyklische Heterocyclyl wobei der/die Substituent(en) gleich oder verschieden sein können und ausgewählt werden aus der Gruppe bestehend aus Methyl, Ethyl, Halogen, -NH₂, -OH und Pseudohalogen; und
- **R⁹**: ein Rest ausgewählt aus der Gruppe bestehend aus gegebenenfalls einfach oder mehrfach substituiertem C₁₋₁₆-Alkyl, C₂-₁₆-Alkenyl, C₂₋₁₆-Alkinyl, C₃₋₁₀-Cycloalkyl, Aryl, Heterocyclyl und Heteroaryl, wobei der/die Substituent(en) gleich oder verschieden sein können und ausgewählt werden aus der Gruppe bestehend aus Halogen-, -NO₂, -OR¹⁰, -C(=O)R¹⁰, -C(=O)OR¹⁰, -C(=O)NR¹⁰R¹¹, -NR¹⁰R¹¹, -NR¹⁰COR¹¹, -NR¹⁰C(=O)OR¹¹, -NR¹⁰C(=O)NR¹¹R¹², -NR¹⁰C(=O)ONR¹¹R¹², -NR¹⁰SO₂R¹¹, -N=CR¹⁰R¹¹, -SR¹⁰, -SOR¹⁰, -SO₂R¹⁰, -SO₂NR¹⁰R¹¹, -NR¹⁰SO₂NR¹¹R¹², -OSO₂NR¹⁰R¹¹ und Pseudohalogen; und
- **R¹⁰**, **R¹¹** und **R¹²**: jeweils unabhängig voneinander Wasserstoff oder ein Rest ausgewählt aus der Gruppe bestehend aus gegebenenfalls einfach oder mehrfach substituiertem C₁₋₈-Alkyl, C₂₋₈-Alkenyl, C₂₋₈-Alkinyl, C₃₋₁₀-Cycloalkyl, Aryl, Heterocyclyl und Heteroaryl, wobei der/die Substituent(en) gleich oder verschieden sein können und ausgewählt werden aus der Gruppe bestehend aus Halogen, -NH₂, -OH und Pseudohalogen;
wobei sich Aryl auf monozyklische oder bizyklische Ringe mit 6-12 Kohlenstoffatomen bezieht;
wobei unter Heteroaryl mono- oder bizyklische Ringe zu verstehen sind, welche anstelle eines oder mehrerer Kohlenstoffatome ein oder mehrere, gleich oder verschiedene Heteroatome, ausgewählt aus Stickstoff- Schwefel- und Sauerstoffatomen, enthalten;
wobei sich Heterocyclyl auf 5-12 Kohlenstoffatome umfassende gesättigte oder ungesättigte, nicht aromatische mono-, bizyklische oder überbrückte bizyklische Ringe bezieht, welche anstelle eines oder mehrerer Kohlenstoffatome Heteroatome ausgewählt aus Stickstoff- Schwefel- und Sauerstoffatomen, enthalten;
gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze, bedeuten.

Ein Aspekt der Erfindung sind Verbindungen der allgemeinen Formel (1), worin
- **X**: -NR^{1a} oder Sauerstoff, und
- **A**: ausgewählt ist aus den Formeln (i) oder **(ii)** 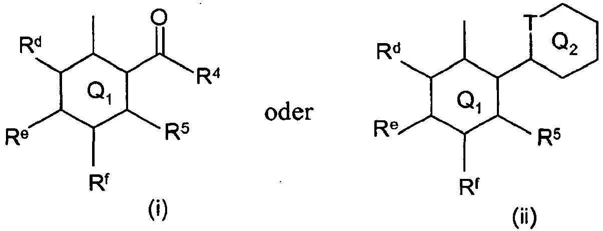 und
- **Q₁**: mono- oder bizyklische Arylverbindungen; und
- **Q₂**: monozyklische Heteroarylverbindungen; und
- **T**: N, O oder S, und
- **R¹** und **R^{1a}**: Wasserstoff; und
- **R³**: Formel **(iii),** 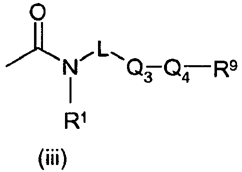
bedeuten und die übrigen Reste wie vorstehend erwähnt definiert sind.

Ein weiterer Aspekt der Erfindung sind Verbindungen der allgemeinen Formel (1), worin
- **Y**: CH; und
- **Q₁**: monozyklische Arylverbindungen bedeuten und die übrigen Reste wie vorstehend erwähnt definiert sind.

Ein anderer Aspekt der Erfindung sind Verbindungen der allgemeinen Formel (1), worin
- **R^{c}**: ein Rest ausgewählt aus der Gruppe bestehend aus Wasserstoff, -F, -Cl, Methyl und Ethyl
bedeutet und die übrigen Reste wie vorstehend erwähnt definiert sind.

Ein zusätzlicher Aspekt dieser Erfindung sind Verbindungen der allgemeinen Formel (1), worin
- **R^{a}** und **R^{b}**: jeweils unabhängig voneinander Wasserstoff oder ein gegebenenfalls einfach oder mehrfach substituierter Rest ausgewählt aus der Gruppe bestehend aus C₁₋₂-Alkyl, C₂-Alkenyl, C₂-Alkinyl, C₃₋₆-Cycloalkyl, Aryl, Heterocyclyl und Heteroaryl, wobei der/die Substituenten gleich oder verschieden sein können und ausgewählt werden aus der Gruppe bestehend aus Wasserstoff, Halogen, -NO₂, -OR⁶, -C(=O)R⁶, -C(=O)OR⁶, -C(=O)NR⁶R⁷, -NR⁶R⁷, -NR⁶C(=O)R⁷, -NR⁶C(=O)OR⁷, -NR⁶C(=O)NR⁷R⁸, -NR⁶SO₂R⁷, -N=CR⁶R⁷, -SR⁶, -SOR⁶, -SO₂R⁶, -SO₂NR⁶R⁷, -NR⁶, -SO₂NR⁷R⁸, -OSO₂NR⁷R⁸ und Pseudohalogen;
bedeuten und die übrigen Reste wie vorstehend erwähnt definiert sind.

Ein Aspekt der Erfindung sind auch Verbindungen der allgemeinen Formel (1), worin
- **R^{a}** und **R^{b}**: Wasserstoff bedeuten und die übrigen Reste wie vorstehend erwähnt definiert sind.

Ein Aspekt der Erfindung sind Verbindungen der allgemeinen Formel (1), oder deren pharmazeutisch wirksamen Salze, zur Verwendung als Arzneimittel.

Ein weiterer Aspekt der Erfindung sind Verbindungen der allgemeinen Formel (1), oder deren pharmazeutisch wirksamen Salze, zur Herstellung eines Arzneimittels mit antiproliferativer Wirkung.

Ein wesentlicher Aspekt der Erfindung sind Verbindungen der allgemeinen Formel (1), oder deren pharmazeutisch wirksamen Salze, zur Verwendung als Arzneimittel mit antiproliferativer Wirkung.

Ferner sind erfindungsgemäß Verbindungen der allgemeinen Formel (1), oder deren pharmazeutisch wirksamen Salze, zur Verwendung als Arzneimittel mit antiproliferativer Wirkung mit einem selektiven kinaseinhibierenden Wirkmechanismus umfasst.

Ein Aspekt der Erfindung ist die Verwendung von Verbindungen der allgemeinen Formel (1), oder deren pharmazeutisch wirksamen Salze, zur Herstellung eines Arzneimittels mit antiproliferativer Wirkung mit einem PLK inhibierendem Wirkmechanismus.

Ein weiterer Aspekt der Erfindung sind pharmazeutische Zubereitungen, enthaltend als Wirkstoff eine oder mehrere Verbindungen der allgemeinen Formel (1), oder deren physiologisch verträgliche Salze gegebenenfalls in Kombination mit üblichen Hilfs-und/oder Trägerstoffen.

Ein zusätzlicher Aspekt der Erfindung ist die Verwendung einer oder mehrerer Verbindungen der allgemeinen Formel (1) zur Herstellung eines Arzneimittels zur Behandlung und/oder Prävention von Krebs, Infektionen, Entzündungs- und Autoimmunerkrankungen.

Ein anderer Aspekt der Erfindung ist eine pharmazeutische Präperation enthaltend eine Verbindung der allgemeinen Formel (1) 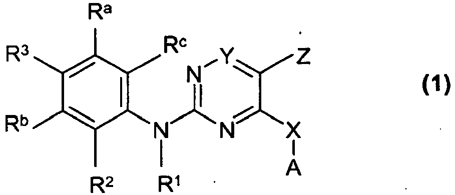 worin
- **X**: -NR^{1a}, O oder S, und
- **Y**: CH und
- **Z**: C₁₋₃-Fluoralkyl und
- **A**: ausgewählt ist aus den Formeln (i) oder **(ii)** 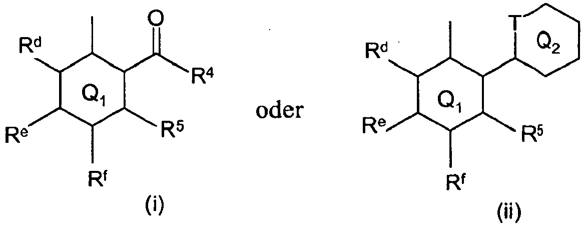 und
- **Q₁**: mono- oder bizyklische Arylverbindungen; und
- **Q₂**: mono- oder bizyklische Heteroarylverbindungen; und
- **T**: N, O oder S, und
- **R¹** und **R^{1a}**: Wasserstoff oder Methyl, und
- **R²**: ein Rest ausgewählt aus der Gruppe bestehend aus -Cl, -Br, -I, -OR⁶, -C(=O)R⁶, -C(=O)NR⁶R⁷, -NR⁶R⁷, -NR⁶C(=O)R⁷, -NR⁶SO₂R⁷, -N=CR⁶R⁷, -SR⁶, -SOR⁶, -SO₂R⁶, -SO₂NR⁶R⁷ und Pseudohalogen, oder ein gegebenenfalls einfach oder mehrfach substituierter Rest ausgewählt aus der Gruppe bestehend aus C₁₋₆-Alkyl, C₂₋₆-Alkenyl, C₂₋₆-Alkinyl, C₃₋₆-Cycloalkyl, Aryl, Heterocyclyl und Heteroaryl, wobei der/die Substituenten gleich oder verschieden sein können und ausgewählt werden aus der Gruppe bestehend aus Halogen, -NO₂, -OR⁶, -C(=O)R⁶, -C(=O)OR⁶, -C(=O)NR⁶R⁷, -NR⁶R⁷, -NR⁶C(=O)R⁷, -NR⁶C(=O)OR⁷, -NR⁶C(=O)NR⁷R⁸, -NR⁶SO₂R⁷, -N=CR⁶R⁷, -SR⁶, -SOR⁶, -SO₂R⁶, -SO₂NR⁶R⁷, -NR⁶SO₂NR⁷R⁸, -OSO₂NR⁷R⁸ und Pseudohalogen; und
- **R^{a}, R^{b}, R^{c}, R^{d}, R^{e}** und **R^{f}**: jeweils unabhängig voneinander ein Rest ausgewählt aus der Gruppe bestehend aus Wasserstoff, Halogen, -NO₂, -OR⁶, -C(=O)R⁶, -C=O)OR⁶, -C(=O)NR⁶R⁷, -NR⁶R⁷, -NR⁶C(=O)R⁷, -NR⁶C(=O)OR⁷, -NR⁶C(=O)NR⁷R⁸, -NR⁶SO₂R⁷, -N=CR⁶R⁷, -SR⁶, -SOR⁶, -SO₂R⁶, -SO₂NR⁶R⁷, -NR⁶SO₂NR⁷R⁸, -OSO₂NR⁷R⁸ und Pseudohalogen;
oder ein gegebenenfalls einfach oder mehrfach substituierter Rest ausgewählt aus der Gruppe bestehend aus C₁₋₆-Alkyl, C₂₋₆-Alkenyl, C₂₋₆-Alkinyl, C₃₋₆-Cycloalkyl, Aryl, Heterocyclyl und Heteroaryl, wobei der/die Substituenten gleich oder verschieden sein können und ausgewählt werden aus der Gruppe bestehend aus Wasserstoff, Halogen, -NO₂, -OR⁶, -C(=O)R⁶, -C(=O)OR⁶, -C(=O)NR⁶R⁷, -NR⁶R⁷, -NR⁶C(=O)R⁷, -NR⁶C(=O)OR⁷, -NR⁶C(=O)NR⁷R⁸, -NR⁶SO₂R⁷, -N=CR⁶R⁷, -SR⁶, -SOR⁶, -SO₂R⁶, -SO₂NR⁶R⁷, -NR⁶SO₂NR⁷R⁸, -OSO₂NR⁷R⁸ und Pseudohalogen; und
- **R³**: ausgewählt aus den Formeln **(iii)** - (ix), 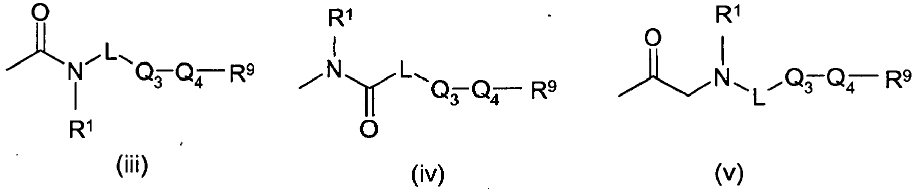 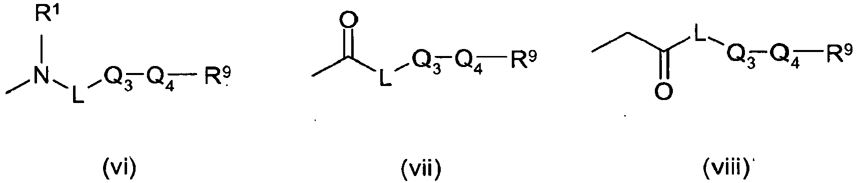 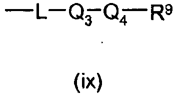 und
- **R⁴**: ein Rest ausgewählt aus der Gruppe bestehend aus Wasserstoff, -OR⁶, -C(=O)R⁶, -C(=O)OR⁶, -C(=O)NR⁶R⁷, -NR⁶R⁷, -NR⁶C(=O)R⁷, -NR⁶C(=O)OR⁷, -NR⁶C(=O)NR⁷R⁸, -NR⁶SO₂R⁷, -N=CR⁶R⁷, -SR⁶, -SOR⁶, -SO₂R⁶, -SO₂NR⁶R⁷, -NR⁶SO₂NR⁷R⁸, -OSO₂NR⁷R⁸ und Pseudohalogen, oder ein Rest ausgewählt aus der Gruppe bestehend aus gegebenenfalls einfach oder mehrfach substituiertem C₁₋₈-Alkyl, C₂₋₁₀-Alkenyl, C₂₋₁₀-Alkinyl, C₃₋₈-Cycloalkyl, Aryl, Heterocyclyl und Heteroaryl, wobei der/die Substituent(en) gleich oder verschieden sein können und ausgewählt werden aus der Gruppe bestehend aus C₁₋₈-Alkyl, Halogen, -NO₂, -OR⁶, -C(=O)R⁶, -C(=O)OR⁶, -C(=O)NR⁶R⁷, -NR⁶R⁷, -NR⁶C(=O)R⁷, -NR⁶C(=O)OR⁷, -NR⁶C(=O)NR⁷R⁸, -NR⁶SO₂R⁷, -N=CR⁶R⁷, -SR⁶, -SOR⁶, -SO₂R⁶, -SO₂NR⁶R⁷, -NR⁶SO₂NR⁷R⁸, -OSO₂NR⁷R⁸ und Pseudohalogen; und
- **R⁵**: Wasserstoff, Halogen, -CF₃, C₁₋₃-Alkyl oder -OR⁶; und
- **R⁶, R⁷** und **R⁸**: jeweils unabhängig voneinander Wasserstoff oder ein Rest ausgewählt aus der Gruppe bestehend aus gegebenenfalls einfach oder mehrfach substituiertem C₁₋₈-Alkyl, C₂₋₅-Alkenyl, C₂₋₅-Alkinyl, C₃₋₁₀-Cycloalkyl, Aryl, Heterocyclyl und Heteroaryl, wobei der/die Substituenten gleich oder verschieden sein können und ausgewählt werden aus der Gruppe bestehend aus C₃₋₁₀-Cycloalkyl, Aryl, Heterocyclyl, Heteroaryl, Halogen, -NO₂, -OR¹⁰, -C(=O)R¹⁰, -C(=O)OR¹⁰, -C(=O)NR¹⁰R¹¹, -NR¹⁰R¹¹, -NR¹⁰C(=O)R¹¹, -NR¹⁰C(=O)OR¹¹, -NR¹⁰C(=O)NR¹¹R¹², -NR¹⁰C(=O)ONR¹¹R¹², -NR¹⁰SO₂R¹¹, -N=CR¹⁰R¹¹, -SR¹⁰, -SOR¹⁰, -SO₂R¹⁰, -SO₂NR¹⁰R¹¹, -NR¹⁰SO₂NR¹¹R¹², -OSO₂NR¹⁰R¹¹ und Pseudohalogen; und
- **L**: eine Bindung oder ein Rest ausgewählt aus der Gruppe bestehend aus gegebenenfalls einfach oder mehrfach substituiertem C₁₋₁₆-Alkyl, C₂₋₁₆-Alkenyl, C₂₋₁₆-Alkinyl, C₃₋₁₀-Cycloalkyl, Aryl, Heterocyclyl und Heteroaryl, wobei der/die Substituent(en) gleich oder verschieden sein können und ausgewählt werden aus der Gruppe bestehend aus Halogen, -NO₂, -OR¹⁰, -C(=O)R¹⁰, -C(=O)OR¹⁰, -C(=O)NR¹⁰R¹¹, -NR¹⁰R¹¹, -NR¹⁰COR¹¹, -NR¹⁰C(=O)OR¹¹, -NR¹⁰C(=O)NR¹¹R¹², -NR¹⁰C(=O)ONR¹¹R¹², -NR¹⁰SO₂R¹¹, -N=CR¹⁰R¹¹, -SR¹⁰, -SOR¹⁰, -SO₂R¹⁰, -SO₂NR¹⁰R¹¹, -NR¹⁰SO₂NR¹¹R¹², -OSO₂NR¹⁰R¹¹ und Pseudohalogen; und
- **Q₃** und **Q₄**: unabhängig voneinander eine Bindung oder ein Rest ausgewählt aus der Gruppe bestehend aus gegebenenfalls einfach oder mehrfach substitutierten mono-oder bizyklische Heterocyclyl wobei der/die Substituent(en) gleich oder verschieden sein können und ausgewählt werden aus der Gruppe bestehend aus Methyl, Ethyl, Halogen, -NH₂, -OH und Pseudohalogen; und
- **R⁹**: ein Rest ausgewählt aus der Gruppe bestehend aus gegebenenfalls einfach oder mehrfach substituiertem C₁₋₁₆-Alkyl, C₂₋₁₆-Alkenyl, C₂₋₁₆-Alkinyl, C₃₋₁₀-Cycloalkyl, Aryl, Heterocyclyl und Heteroaryl, wobei der/die Substituent(en) gleich oder verschieden sein können und ausgewählt werden aus der Gruppe bestehend aus Halogen-, -NO₂, -OR¹⁰, -C(=O)R¹⁰, -C(=O)OR¹⁰, -C(=O)NR¹⁰R¹¹, -NR¹⁰R¹¹, -NR¹⁰COR¹¹, -NR¹⁰C(=O)OR¹¹, -NR¹⁰C(=O)NR¹¹R¹², -NR¹⁰C(=O)ONR¹¹R¹², -NR¹⁰SO₂R¹¹, -N=CR¹⁰R¹¹, -SR¹⁰, -SOR¹⁰, -SO₂R¹⁰, -SO₂NR¹⁰R¹¹, -NR¹⁰SO₂NR¹¹R¹², -OSO₂NR¹⁰R¹¹ und Pseudohalogen; und
- **R¹⁰, R¹¹** und **R¹²**: jeweils unabhängig voneinander Wasserstoff oder ein Rest ausgewählt aus der Gruppe bestehend aus gegebenenfalls einfach oder mehrfach substituiertem C₁₋₈-Alkyl, C₂₋₈-Alkenyl, C₂₋₈-Alkinyl, C₃₋₁₀-Cycloalkyl, Aryl, Heterocyclyl und Heteroaryl, wobei der/die Substituent(en) gleich oder verschieden sein können und ausgewählt werden aus der Gruppe bestehend aus Halogen, -NH₂, -OH und Pseudohalogen;
wobei sich Aryl auf monozyklische oder bizyklische Ringe mit 6-12 Kohlenstoffatomen bezieht;
wobei unter Heteroaryl mono- oder bizyklische Ringe zu verstehen sind, welche anstelle eines oder mehrerer Kohlenstoffatome ein oder mehrere, gleich oder verschiedene Heteroatome, ausgewählt aus Stickstoff- Schwefel- und Sauerstoffatomen, enthalten;
wobei sich Heterocyclyl auf 5-12 Kohlenstoffatome umfassende gesättigte oder ungesättigte, nicht aromatische mono-, bizyklische oder überbrückte bizyklische Ringe bezieht, welche anstelle eines oder mehrerer Kohlenstoffatome Heteroatome ausgewählt aus Stickstoff- Schwefel- und Sauerstoffatomen, enthalten;
gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze, und
mindestens eine weitere Wirksubstanz ausgewählt aus der Gruppe bestehend aus zytostatische Wirksubstanzen, zytotoxische Wirksubstanzen, Steroide und Antikörper, gegebenenfalls in Form ihrer Tautomeren, ihrer Racemate, ihrer Enantiomere, ihrer

Diastereomere und ihrer Gemische, sowie gegebenenfalls ihrer pharmakologisch unbedenklichen Säureadditionssalze.
genannten Eigenschaften.

### DEFINITIONEN

Wie hierin verwendet treffen folgenden Definitionen zu, falls nicht anders beschrieben.

Unter Alkyl-Substitutenten sind jeweils gesättigte, geradkettige oder verzweigte aliphatische Kohlenwasserstoffreste (Alkylrest) zu verstehen.

Die Alkenyl-Substituenten sind jeweils geradkettige oder verzweigte, ungesättigte Alkylrest, die mindestens eine Doppelbindung aufweisen.

Unter Alkinyl-Substituenten sind jeweils geradkettige oder verzweigte, ungesättigte Alkylreste, die mindestens eine Dreifachbindung aufweisen, zu verstehen.

Halogenalkyl bezieht sich auf Alkylreste, in denen ein oder mehrere Wasserstoffatome durch Halogenatome ersetzt sind. Halogenalkyl umfasst sowohl gesättigte Alkylreste als auch ungesättigte Alkenyl- und Alkinylreste, wie beispielsweise -CF₃, -CHF₂, -CH₂F, -CF₂CF₃,-CHFCF₃, -CH₂CF₃, -CF₂CH₃, -CHFCH₃, -CF₂CF₂CF₃, -CF₂CH₂CH₃, -CF=CF₂, -CCl=CH₂, -CBr=CH₂, -CI=CR₂, -C≡C-CR₃, -CHFCH₂CH₃ und -CHFCH₂CF₃.

Halogen bezieht sich auf Fluor-, Chlor-, Brom- und/oder Jodatome.

Unter Pseudohalogen sind folgende Reste zu verstehen: -OCN, -SCN, -CF3 und -CN.

Unter Cycloalkyl ist ein mono- oder bizyklischer Ring zu verstehen, wobei das Ringsystem ein gesättigter Ring aber auch ein ungesättigter, nichtaromatischer Ring sein kann, welcher gegebenenfalls auch Doppelbindungen enthalten kann, wie zum Beispiel Cyclopropyl, Cyclopropenyl, Cyclobutyl, Cyclobutenyl, Cyclopentyl, Cyclopentenyl, Cyclohexyl, Cyclohexenyl, Norbornyl und Norbornenyl.

Aryl bezieht sich auf monozyklische oder bizyklische Ringe mit 6 -12 Kohlenstoffatomen wie beispielsweise Phenyl und Naphthyl.

Unter Heteroaryl sind mono- oder bizyklische Ringe zu verstehen, welche anstelle eines oder mehrere Kohlenstoffatome ein oder mehrere, gleich oder verschiedene Heteroatome enthalten, wie z.B. Stickstoff-, Schwefel- oder Sauerstoffatome. Beispielsweise genannt seien Furyl, Thienyl, Pyrrolyl, Oxazolyl, Thiazolyl, Isoxazolyl, Isothiazolyl, Pyrazolyl, Imidazolyl, Triazolyl, Tetrazolyl, Oxadiazolyl, Thiadiazolyl, Pyridyl, Pyrimidyl, Pyridazinyl, Pyrazinyl und Triazinyl. Beispiele für bizyklische Heteroarylreste sind Indolyl, Isoindolyl, Benzofuranyl, Benzothienyl, Benzoxazolyl, Benzothiazolyl, Benzisoxazolyl, Benzisothiazolyl, Benzimidazolyl, Indazolyl, Isoquinolinyl, Quinolinyl, Quinoxalinyl, Cinnolinyl, Phthalazinyl, Quinazolinyl und Benzotriazinyl, Indolizinyl, Oxazolopyridinyl, Imidazopyridinyl, Naphthyridinyl, Indolinyl, Isochromanyl, Chromanyl, Tetrahydroisochinolinyl, Isoindolinyl, Isobenzotetrahydrofuranyl, Isobenzotetrahydrothienyl, Isobenzothienyl, Benzoxazolyl, Pyridopyridinyl, Benzotetrahydrofuranyl, Benzotetrahydrothienyl, Purinyl, Benzodioxolyl, Triazinyl, Phenoxazinyl, Phenothiazinyl, Pteridinyl, Benzothiazolyl, Imidazopyridinyl, Imidazothiazolyl, Dihydrobenzisoxazinyl, Benzisoxazinyl, Benzoxazinyl, Dihydrobenzisothiazinyl, Benzopyranyl, Benzothiopyranyl, Coumarinyl, Isocoumarinyl, Chromonyl, Chromanonyl, Pyridinyl-*N*-oxid, Tetrahydroquinolinyl, Dihydroquinolinyl, Dihydroquinolinonyl, Dihydroisoquinolinonyl, Dihydrocoumarinyl, Dihydroiso-coumarinyl, Isoindolinonyl, Benzodioxanyl, Benzoxazolinonyl, Pyrrolyl-*N*-oxid, Pyrimidinyl-*N*-oxid, Pyridazinyl-*N*-oxid, Pyrazinyl-*N*-oxid, Quinolinyl-*N*-oxid, Indolyl-*N-*oxid, Indolinyl-*N*-oxid, Isoquinolyl-*N*-oxid, Quinazolinyl-*N*-oxid, Quinoxalinyl-*N*-oxid, Phthalazinyl-*N*-oxid, Imidazolyl-*N-*oxid, Isoxazolyl-*N*-oxid, Oxazolyl-*N*-oxid, Thiazolyl-N-oxid, Indolizinyl-*N*-oxid, Indazolyl-*N*-oxid, Benzothiazolyl-*N*-oxid, Benzimidazolyl-*N* oxid, Pyrrolyl-*N*-oxid, Oxadiazolyl-*N*-oxid, Thiadiazolyl-*N*-oxid, Triazolyl-*N*-oxid, Tetrazolyl-*N*-oxid, Benzothiopyranyl-*S*-oxid und Benzothiopyranyl-*S,S*-dioxid.

Heterocyclyl bezieht sich auf 5 - 12 Kohlenstoffatome umfassende gesättigte oder ungesättigte, nicht aromatische mono-, bizyklische oder überbrückte bizyklische Ringe, welche anstelle eines oder mehrere Kohlenstoffatome Heteroatome, wie Stickstoff, Sauerstoff oder Schwefel, tragen. Beispiele für solche Heterocylylreste sind Tetrahydrofuranyl, Pyrrolidinyl, Pyrrolinyl, Imidazolidinyl, Imidazolinyl, Pyrazolidinyl, Pyrazolinyl, Piperidyl, Piperazinyl, Indolinyl, Isoindolinyl, Morpholinyl, Thiomorpholinyl, Homomorpholinyl, Homopiperidyl, Homopiperazinyl, Thiomorpholinyl-*S*-oxid, Thiomorpholinyl-*S*,*S*-dioxid, Pyrrolidinyl, Tetrahydropyranyl, Piperidinyl, Tetrahydrothienyl, Homopiperidinyl, Homothiomorpholinyl-*S,S*-Dioxid, Oxazolidinonyl, Dihydropyrazolyl, Dihydropyrrolyl, Dihydropyrazinyl, Dihydropyridinyl, Dihydropyrimidinyl, Dihydrofuryl, Dihydropyranyl, Tetrahydrothienyl-*S*-oxid, Tetrahydrothienyl-*S,S*-dioxid, Homothiomorpholinyl-*S*-oxid, 2-Oxa-5-azabicyclo[2.2.1]heptan, 8-Oxa-3-aza-bicyclo[3.2.1]octan, 3,8-Diaza-bicyclo[3.2.1]octan, 2,5-Diaza-bicyclo[2.2.1]heptan, 3,8-Diaza-bicyclo[3.2.1]octan, 3,9-Diaza-bicyclo[4.2.1]nonan und 2,6-Diaza-bicyclo[3.2.2]nonan.

Die nachfolgenden Beispiele illustrieren die vorliegende Erfindung ohne sie jedoch in ihrem Umfang einzuschränken:

### Herstellung der erfindungsgemäßen Verbindungen:

Die Herstellung der erfindungsgemäßen Verbindungen kann nach den, im Folgenden beschriebenen Syntheseverfahren Abis C erfolgen, wobei die Substituenten der allgemeinen Formeln **(I** bis **XVI)** die zuvor genannten Bedeutungen haben.

### Chromatographie:

Für die Mitteldruck Chromatographie (MPLC) wird Kieselgel der Firma Millipore (Bezeichnung: Granula Silica Si-60A 35-70µm) oder C-18 RP-Kieselgel der Firma Macherey Nagel (Bezeichnung: Polygoprep 100-50 C18) eingesetzt.
Für die Hochdruck Chromatographie (HPLC) werden Säulen der Firma Waters (Bezeichnung: XTerra Prep. MS C18, 5 µM, 30*100 mm oder Symmetrie C18, 5 µm, 19*100) verwendet.

### Massenspektroskopie / UV-Spektrometer:

Diese Daten werden mit Hilfe einer HPLC-MS Anlage (high performance liquid chromatography mit Massendetektor) der Firma Agilent erzeugt.
Die Anlage ist so aufgebaut, dass anschließend an die Chromatographie (Säule: Zorbax SB-C8, 3,5 µm, 2,1*50, Fa. Agilent) ein Diodenarry-Detektor (G1315B von Fa. Agilent) und ein Massendetektor (1100 LS-MSD SL; G1946D; Fa. Agilent) in Reihe geschalten sind.
Die Anlage wird mit einem Fluß von 0,6 ml/min betrieben. Für einen Trennvorgang wird ein Gradient innerhalb von 3,5 min durchlaufen (Gradient Anfang: 95% Wasser und 5% Acetonitril; Gradient Ende: 5% Wasser und 95% Acetonitril; den beiden Lösungsmitteln wird jeweils 0,1%Ameisensäure beigemischt).

Soweit die Herstellung der Ausgangsverbindungen nicht beschrieben wird, sind diese bekannt, kommerziell erhältlich oder analog zu bekannten Verbindungen oder hier beschriebenen Verfahren herstellbar. Falls nicht anders erwähnt, liegen die Verbindungen als freie Base vor. In den Tabellen stellen X₁ und X₂ den Anknüpfungspunkt des jeweiligen Strukturfragmentes an die generische Struktureinheit dar.

### Verfahren A

### Stufe 1A

Die Herstellung der Zwischenverbindung **III** erfolgt durch Substitution einer Abgangsgruppe LG, beispielsweise Halogen, Pseudohalogen, Methoxy, vorzugsweise Chlor, an einem heteroaromatischem System I durch ein Nukleophil **II.**

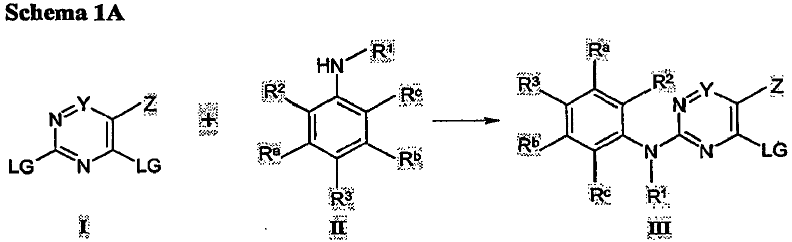

Es werden 1 Äquivalent der Verbindung I und 1 bis 1,5 Äquivalente der Verbindung **II,** in einem Lösungsmittel, beispielsweise 1,4-Dioxan, Tetrahydrofuran, *N*,*N-*Dimethylformamid oder *N,N*-Dimethylacetamid gerührt.
Bei einer Temperatur von 15 bis 25 °C werden 2 bis 2,5 Äquivalente einer Base, beispielsweise Kaliumcarbonat, Natriumcarbonat, Cäsiumcarbonat, *N*-Ethyl-*N,N-*diisopropylamin oder Triethylamin zugegeben. Das Reaktionsgemisch wird 12 bis 72 h bei einer Temperatur von 15 bis 25 °C weitergerührtDanach wird das Lösungsmittel abdestilliert und der Rückstand mit Wasser versetzt, welches mit einer Mineralsäure, beispielsweise Salzsäure oder Schwefelsäure auf einen pH-Wert zwischen 1 - 4 eingestellt wird. Dieses Gemisch wird zwei bis dreimal mit einem organischen Lösungsmittel beispielsweise Diethylether, Ethylacetat oder Dichlormethan extrahiert.
Die vereinigten organischen Extrakte werden getrocknet und das Lösungsmittel abdestilliert. Der Rückstand wird chromatographisch gereinigt.

### Stufe 2A

Die Herstellung der Endverbindung V oder VII erfolgt durch Substitution einer Abgangsgruppe LG, beispielsweise Halogen, SCN, Methoxy, vorzugsweise Chlor, an einem heteroaromatischem System **III** durch ein Nukleophil IV oder VI.

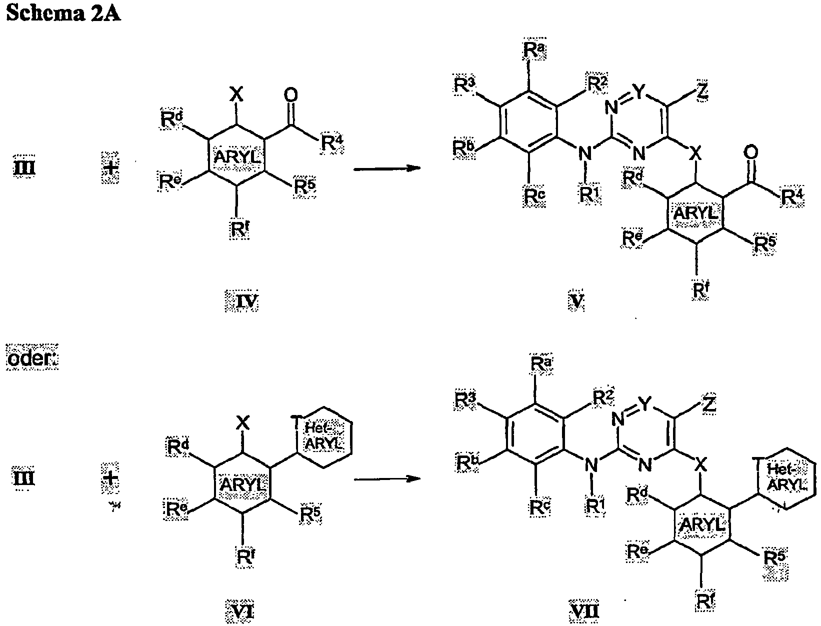

Es werden 1 Äquivalent der Verbindung **III** und 1 bis 3 Äquivalente der Verbindung IV oder **VI** in einem Lösungsmittel, beispielsweise 1,4-Dioxan, *N,N*-Dimethylformamid, *N*,*N-*Dimethylacetamid oder *N*-Methyl-2-pyrrolidinon gerührt.
Bei einer Temperatur von 15 bis 40 °C werden 1 bis 2 Äquivalente einer Mineralsäure, beispielsweise Schwefelsäure oder Salzsäure zugegeben. Das Reaktionsgemisch wird 12 bis 72 h bei einer Temperatur von 20 bis 100 °C weitergerührt. Danach wird das Lösungsmittel abdestilliert und der Rückstand chromatographisch gereinigt.

### Verfahren B

### Stufe 1B

Die Herstellung der Zwischenverbindung IX erfolgt durch Substitution einer Abgangsgruppe LG, beispielsweise Halogen, SCN, Methoxy, vorzugsweise Chlor, an einem heteroaromatischem System I durch ein Nukleophil **VIII.**

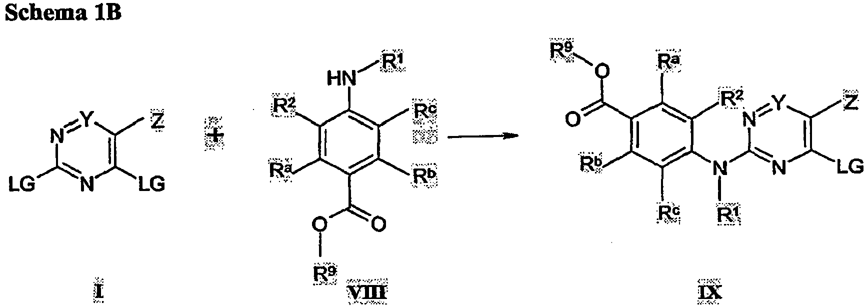

Es werden 1 Äquivalent der Verbindung I und 1 bis 1,5 Äquivalente der Verbindung **VIII** in einem Lösungsmittel, beispielsweise 1,4-Dioxan, Tetrahydrofuran, *N*,*N-*Dimethylformamid oder *N,N-*Dimethylacetamid gerührt.
Bei einer Temperatur von 15 bis 25 °C werden 2 bis 2,5 Äquivalente einer Base, beispielsweise Kaliumcarbonat, Natriumcarbonat, Cäsiumcarbonat, Kaliumhydrogenphosphat, *N*-Ethyl-*N,N*-diisopropylamin oder Triethylamin zugegeben. Das Reaktionsgemisch wird 2 bis 8 h bei einer Temperatur von 50 bis 120 °C weitergerührt. Das Reaktionsgemisch wird mit Wasser versetzt, welches mit einer anorganischen Base, beispielsweise Natriumhydrogencarbonat oder Kaliumcarbonat auf einen pH-Wert von 8 bis 9 eingestellt wird. Dieses Gemisch wird zwei bis dreimal mit einem organischen Lösungsmittel, beispielsweise Diethylether oder Ethylacetat extrahiert. Die vereinigten organischen Extrakte werden getrocknet und das Lösungsmittel abdestilliert. Der Rückstand wird durch mehrmalige Kristallisation gereinigt.

### Stufe 2B

Die Herstellung der Zwischenverbindung X oder **XI** erfolgt durch Substitution einer Abgangsgruppe LG, beispielsweise Halogen, SCN, Methoxy, vorzugsweise Chlor, an einem heteroaromatischem System **IX** durch ein Nukleophil IV oder VI.

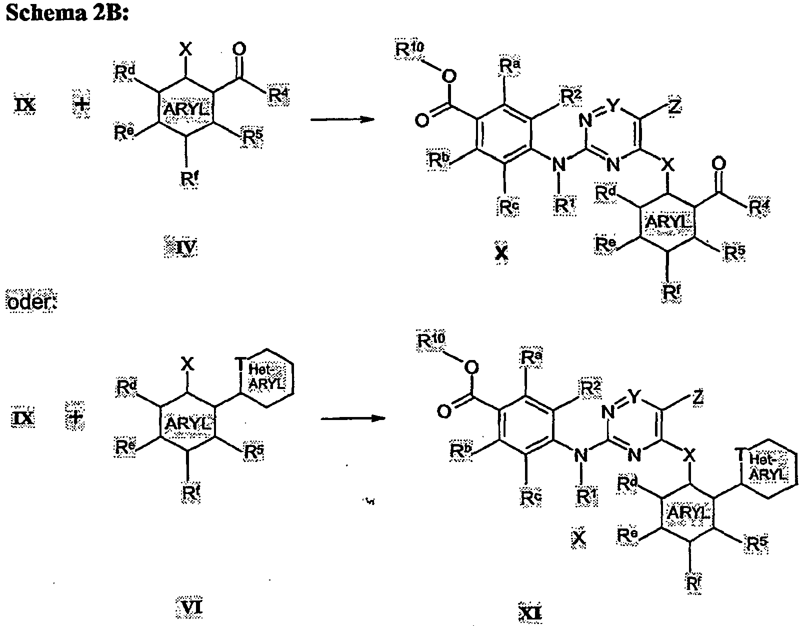

Es werden 1 Äquivalent der Verbindung **IX** und 1 bis 1,5 Äquivalente der Verbindung IV oder VI in einem Lösungsmittel, beispielsweise 1,4-Dioxan, *N,N-*Dimethylformamid, *N,N-*Dimethylacetamid oder *N*-Methyl-2-pyrrolidinon gerührt.
Bei einer Temperatur von 15 bis 40 °C werden 0,2 bis 1 Äquivalent einer Säure, beispielsweise Schwefelsäure oder Salzsäure zugegeben. Das Reaktionsgemisch wird 12 bis 72 h bei einer Temperatur von 20 bis 100 °C weitergerührt. Das Reaktionsgemisch wird in Wasser eingerührt und der entstehende Niederschlag abfiltriert und getrocknet. Der Niederschlag kann durch Chromatographie oder Kristallisation gereinigt oder als Rohprodukt in der nächsten Stufe eingesetzt werden.

### Stufe 3B

Alle Verbindungen X oder **XI** mit einem Rest R¹⁰ ungleich Wasserstoff müssen vor der eigentlichen Stufe 3B durch literaturbekannte Verfahren in Verbindungen, bei denen der Rest R¹⁰ Wasserstoff repräsentiert, überführt werden. Verbindungen **X** oder **XI** deren Rest R¹⁰ Wasserstoff darstellt können direkt zur Herstellung der Endverbindungen **XIII** oder **XIV** eingesetzt werden, wobei eine Verbindung **XII** mit einer Verbindung **X** oder XI umgesetzt wird.

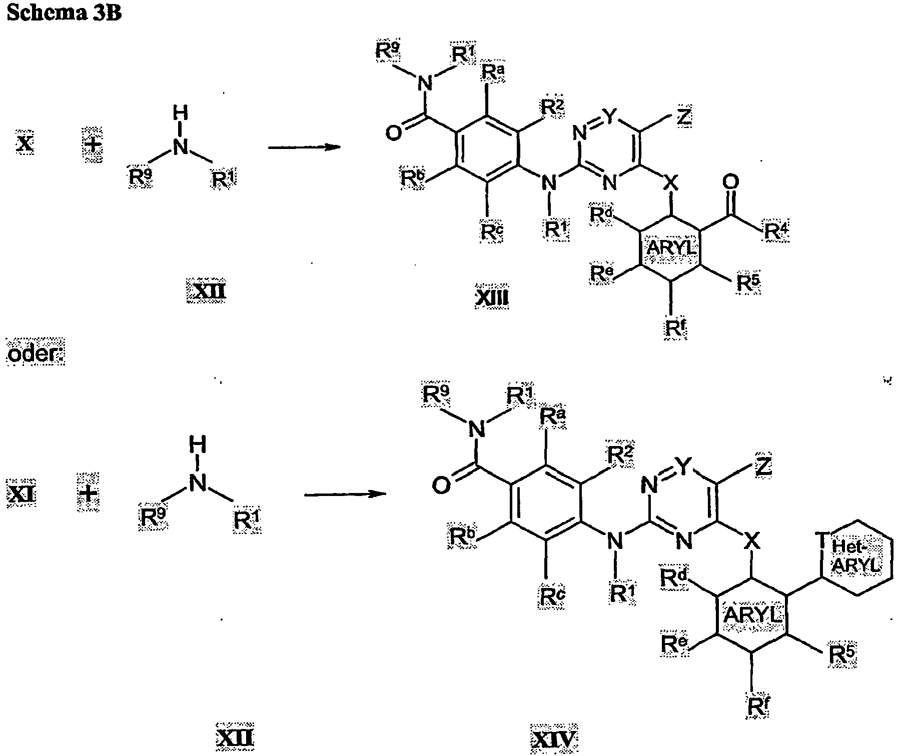

Es werden 1 Äquivalent der Verbindung **X** oder XI, 1 bis 1,5 Äquivalente der Verbindung **XII** und 1 bis 3 Äquivalente einer Base, beispielsweise Triethylamin oder Ethyldiisopropylamin in einem Lösungsmittel, beispielsweise 1,4-Dioxan, *N,N-*Dimethylformamid, *N,N*-Dimethylacetamid oder *N*-Methyl-2-pyrrolidinon gerührt.

Bei einer Temperatur von 15 bis 25 °C werden 1 bis 1,5 Äquivalente eines Kupplungsreagenzes, beispielsweise *N,N*-Dicyclohexylcarbodiimid, *N,N*-Diisopropyl-carbodiimid, *O*-(Benzotriazol-1-yl)-*N,N,N',N'*-tetramethyluronium-tetrafluoroborat oder 1-(3*-N,N-*Dimethylaminopropyl)-3-ethylcarbodiimid zugegeben. Das Reaktionsgemisch wird 4 bis 24 h bei einer Temperatur von 15 bis 25 °C weitergerührt. Danach wird das Lösungsmittel abdestilliert und der Rückstand chromatographisch gereinigt.

### Verfahren C

### Stufe 1C

Die Herstellung der Zwischenverbindung XV oder **XVI** erfolgt durch Substitution einer Abgangsgruppe LG, beispielsweise Halogen, SCN, Methoxy, vorzugsweise Chlor, an einem heteroaromatischem System I durch ein Nukleophiel IV oder VI.

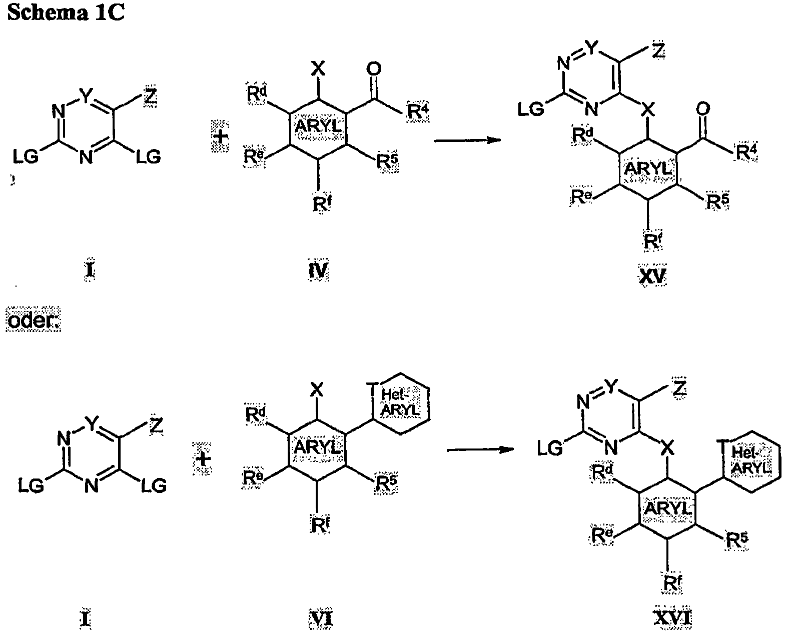

Es werden 1 Äquivalent der Verbindung I und 1 bis 3 Äquivalente einer Base, beispielsweise Triethylamin oder Ethyldiisopropylamin einem Lösungsmittel, beispielsweise 1,4-Dioxan, Tetrahydrofuran, *N,N*-Dimethylformamid oder *N*,*N-*Dimethylacetamid gerührt. Bei einer Temperatur von -60 bis 0 °C werden 0,8 bis 1,5 Äquivalente einer Verbindung IV oder **VI** zugegeben. Das Reaktionsgemisch wird 12 bis 72 h bei einer Temperatur von 15 bis 25 °C weitergerührt. Danach wird das Lösungsmittel abdestilliert und der Rückstand chromatographisch gereinigt.

### Stufe 2C

Die Herstellung der Endverbindung V oder VII erfolgt durch Substitution einer Abgangsgruppe LG, beispielsweise Halogen, SCN, Methoxy, vorzugsweise Chlor, an einem heteroaromatischem System XV oder XVI durch ein Nukleophil **II**.

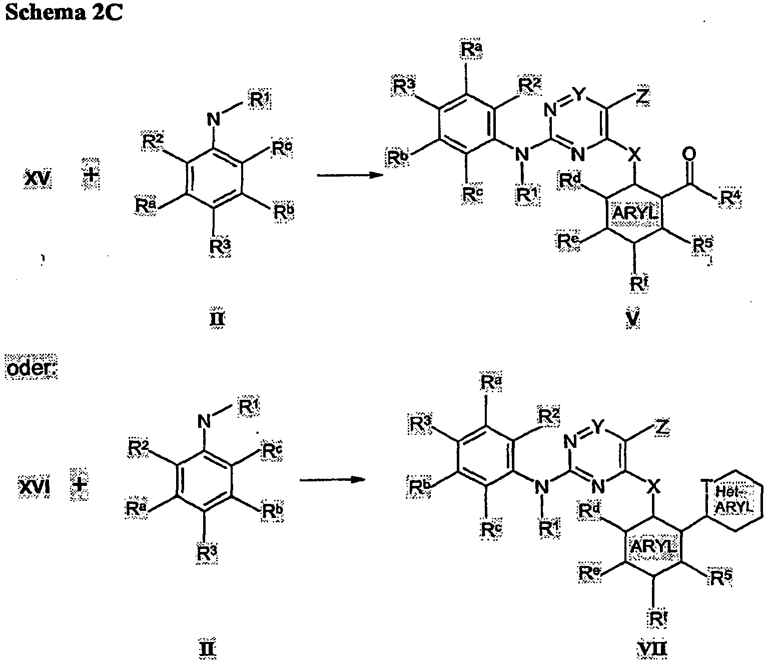

Es werden 1 Äquivalent der Verbindung XV oder **XVI** und 1 bis 1,5 Äquivalente der Verbindung **II** in einem Lösungsmittel, beispielsweise 1,4-Dioxan, *N,N*-Dimethyl-formamid, *N,N-*Dimethylacetamid oder *N*-Methyl-2-pyrrolidinon gerührt.

Bei einer Temperatur von 15 bis 40 °C werden 1 bis 2 Äquivalente einer Säure, beispielsweise Schwefelsäure oder Salzsäure zugegeben. Das Reaktionsgemisch wird 12 bis 72 h bei einer Temperatur von 20 bis 100 °C weitergerührt. Danach wird das Lösungsmittel abdestilliert und der Rückstand chromatographisch gereinigt.

### Beispiel 1

### 2-(2-Methoxy-4-N-propylcarbamoyl-phenylamino)-4-(2-carboxy-3-fluor-phenylamino)-5-trifluormethyl-pyrimidin

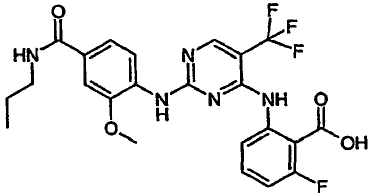

165 mg (0,424 mmol) 2-(2-Methoxy-4-propylcarbamoyl-phenylamino)-4-chlor-5-trifluor-methyl-pyrimidin (Methode 1) werden in 400 µl 1,4-Dioxan gelöst und mit 72 mg (0,466 mmol) 2-Amino-6-fluor-benzoesäure versetzt. Diesem Reaktionsgemisch werden 106 µl einer 4 molaren Lösung von HCl (0,424 mmol) in 1,4-Dioxan zudosiert.
Nach einem Tag bei 50 °C wird das Lösungsmittel im Vakuum entfernt. Das Rohprodukt wird durch Säulenchromatographie gereinigt. Als Trägermaterial dient Kieselgel und als Eluent wird Dichlormethan, dem 18% einer Mischung aus 90% Methanol und 10% gesättigter wässriger Ammoniak-Lösung zugefügt sind, verwendet Ausbeute: 212 mg (0,418 mmol; 98 %) eines weißen Feststoffs
UV max: 318 nm
MS (ESI): 508 (M+H)⁺

### Beispiele 2 - 10

Die folgenden Verbindungen sind über ein analoges Verfahren, wie in Beispiel 1 beschrieben, hergestellt. Dabei wird 2-(2-Methoxy-4-propylcarbamoyl-phenylamino)-4-chlor-5-trifluormethylpyrimidin und ein entsprechendes kommerziell erhältliches Anilin verwendet. Als Lösungsmittel wird 1,4-Dioxan, *N*-Methyl-2-pyrrolidinon oder *N,N-*Dimethylacetamid verwendet

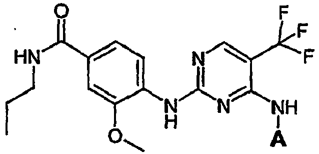

| **#** | **A** | **UV max [nm]** | **MS (ESI) (M+H)⁺** |
|---|---|---|---|
| 2 | 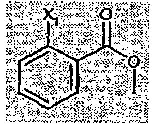 | 236/284 | 504 |
| 3 | 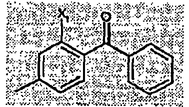 | 270 | 564 |
| 4 | 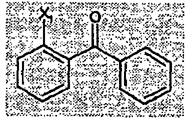 | 322 | 550 |
| 5 | 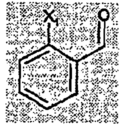 | 322 | 474 |
| 6 | 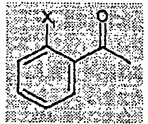 | 322 | 488 |
| 7 | 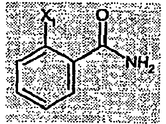 | 320 | 489 |
| 8 | 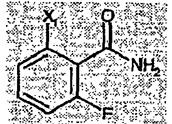 | 314 | 507 |
| 9 | 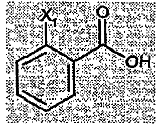 | 322 | 490 |
| 10 | 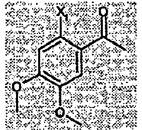 | 270 | 548 |

### Beispiele 11 - 19

Die folgenden Verbindungen sind über ein analoges Verfahren, wie in Beispiel 1 beschrieben, hergestellt.

Die Herstellung des entsprechenden Anilins ist in Tetrahedron 2000, 56(37), 7245, Tetrahedron Letters 1992, 33(43), 6453, US 4,307,113 oder in Methode 2 - 5 beschrieben. Als Lösungsmittel wird 1,4-Dioxan oder *N,N*-Dimethylacetamid verwendet.

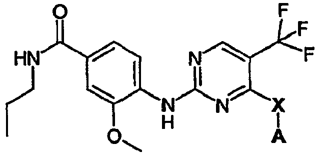

| **#** | **X-A** | **UV max [nm]** | **MS (ESI) (M+H)⁺** |
|---|---|---|---|
| 11 | 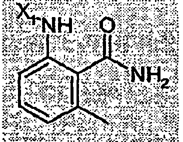 | 0 | 503 |
| 12 | 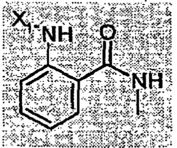 | 234 | 503 |
| 13 | 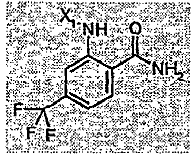 | 318 | 557 |
| 14 | 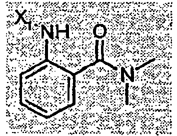 | 318 | 517 |
| 15 | 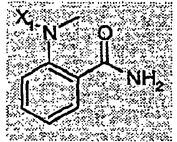 | 318 | 503 |
| 16 | 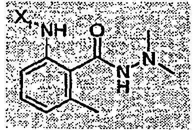 | 317 | 546 |
| 17 | 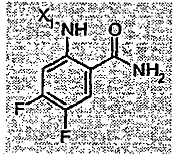 | 282 | 525 |
| 18 | 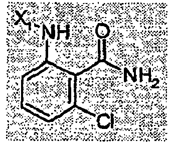 | 318 | 523 |
| 19 | 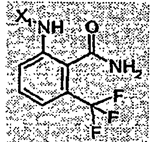 | 318 | 557 |

### Beispiele 20 - 33

Die folgenden Verbindungen sind über ein analoges Verfahren, wie in Beispiel 1 beschrieben, hergestellt. Dabei wird 2-(2-Methoxy-4-propylcarbamoyl-phenylamino)-4-chlor-5-trifluormethylpyrimidin und ein entsprechendes Anilin, welches in Methode 6 beschrieben ist, verwendet.

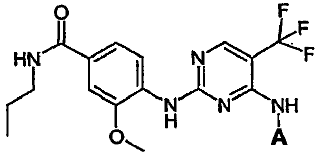

| **#** | **A** | **UV max [nm]** | **MS (ESI) (M+H)⁺** |
|---|---|---|---|
| 20 | 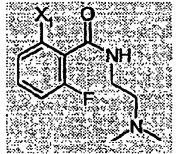 | 320 | 578 |
| 21 | 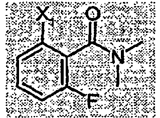 | 275 | 535 |
| 22 | 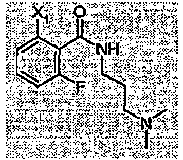 | 320 | 592 |
| 23 | 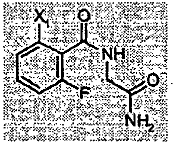 | 315 | 546 |
| 24 | 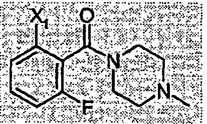 | 320 | 591 |
| 25 | 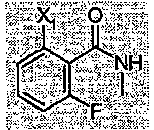 | 315 | 521 |
| 26 | 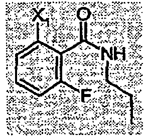 | 318 | 549 |
| 27 | 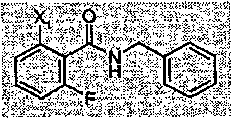 | 315 | 597 |
| 28 | 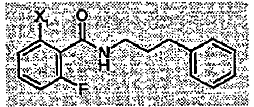 | 315 | 625 |
| 29 | 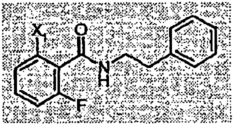 | 318 | 612 |
| 30 | 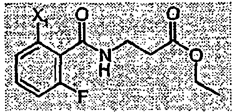 | 315 | 607 |
| 31 | 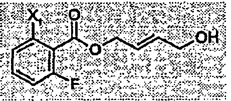 | 318 | 578 |
| 32 | 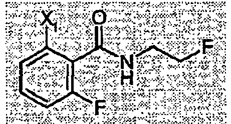 | 316 | 553 |
| 33 | 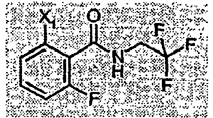 | 230 | 589 |

### Beispiele 34 - 41

Die folgenden Verbindungen sind über ein analoges Verfahren, wie in Beispiel 1 beschrieben, hergestellt. Dabei wird 2-(2-Methoxy-4-propylcarbamoyl-phenylamino)-4-chlor-5-trifluormethylpyrimidin und ein entsprechendes Anilin, welches in Methode 7 beschrieben ist, verwendet.

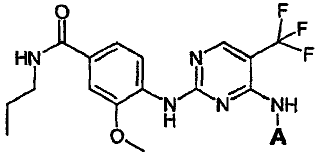

| **#** | **A** | **UV max [nm]** | **MS (ESI) (M+H)⁺** |
|---|---|---|---|
| 34 | 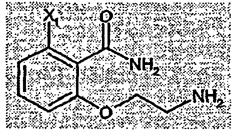 | 282/318 | 548 |
| 35 | 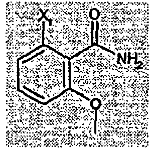 | 314 | 519 |
| 36 | 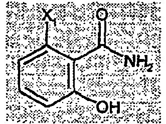 | 314 | 505 |
| 37 | 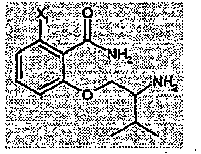 | 230/282 /318 | 590 |
| 38 | 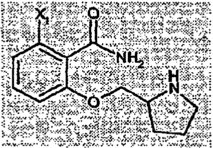 | 230/282 /318 | 588 |
| 39 | 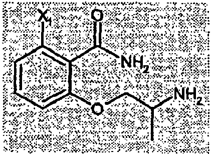 | 282/318 | 562 |
| 40 | 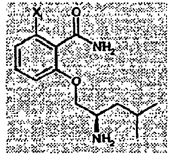 | 226/282 /318 | 604 |
| 41 | 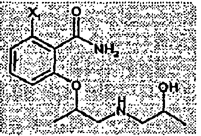 | 226/282 /318 | 620 |

### Beispiele 42 - 45

Die folgenden Verbindungen sind über ein analoges Verfahren, wie in Beispiel 1 beschrieben, hergestellt. Dabei wird 2-(2-Methoxy-4-propylcarbamoyl-phenylamino)-4-chlor-5-trifluormethylpyrimidin und ein entsprechendes Anilin, welches in Methode 6 beschrieben ist, verwendet.

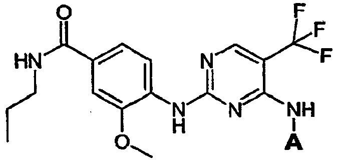

| **#** | **A** | **UV max [nm]** | **MS (ESI) (M+H)⁺** |
|---|---|---|---|
| 42 | 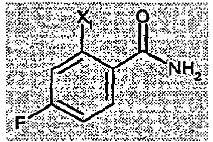 | 315 | 507 |
| 43 | 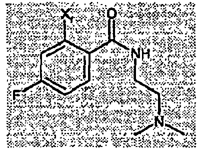 | 285/320 | 578 |
| 44 | 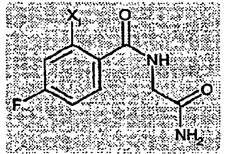 | 230/285 /320 | 564 |
| 45 | 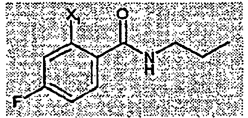 | 318 | 549 |

### Beispiele 46 - 48

Die folgenden Verbindungen sind über ein analoges Verfahren, wie in Beispiel 1 beschrieben, hergestellt Dabei wird 2-(2-Methoxy-4-propylcarbamoyl-phenylamino)-4-chlor-5-trifluormethylpyrimidin und ein entsprechendes Anilin, welches in Methode 8 beschrieben ist, verwendet.

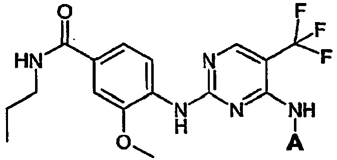

| **#** | **A** | **UV max [nm]** | **MS (ESI) (M+H)⁺** |
|---|---|---|---|
| 46 | 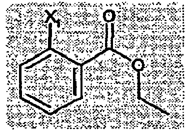 | 238/286 | 518 |
| 47 | 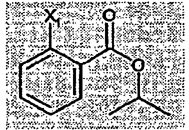 | 230/ 282/306 | 532 |
| 48 | 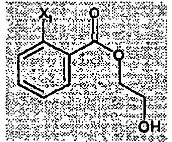 | 282/318 | 534 |

### Beispiele 49 - 52

Die folgenden Verbindungen sind über ein analoges Verfahren, wie in Beispiel 1 beschrieben, hergestellt. Dabei wird 2-(2-Methoxy-4-propylcarbamoyl-phenylamino)-4-chlor-5-trifluormethylpyrimidin und ein entsprechendes Anilin, welches in Methode 9 beschrieben ist, verwendet.

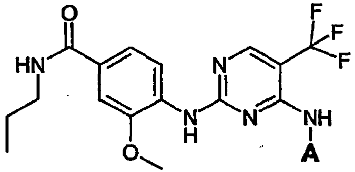

| **#** | **A** | **UV max [nm]** | **MS (ESI) (M+H)⁺** |
|---|---|---|---|
| 49 | 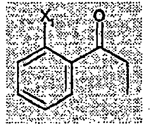 | 280 | 502 |
| 50 | 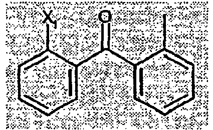 | 230-330 | 564 |
| 51 | 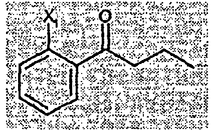 | 230 | 516 |
| 52 | 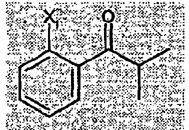 | 270 | 516 |

### Beispiele 53 - 56

Die folgenden Verbindungen sind über ein analoges Verfahren, wie in Beispiel 1 beschrieben, hergestellt. Dabei wird 2-(2-Methoxy-4-propylcarbamoyl-phenylamino)-4-chlor-5-trifluormethylpyrimidin und ein entsprechendes Anilin, welches kommerziell erhältlich oder in Journal of the Chemical Society, Perkin Transactions 1: 1979, (9), 2203 oder in Journal of Medicinal Chemistry 1963, 6(5), 471-80 beschrieben ist, verwendet.

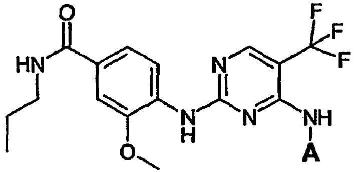

| **#** | **A** | **UV max [nm]** | **MS (ESI) (M+H)⁺** |
|---|---|---|---|
| 53 | 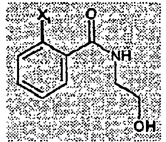 | 266 | 533 |
| 54 | 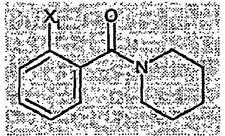 | 316 | 557 |
| 55 | 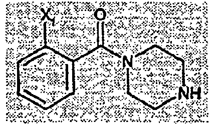 | 313 | 556 (M-H) |
| 56 | 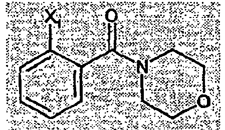 | 241,250 | 559 |

### Beispiele 57

### 2-[2-(2-Chloro-phenoxy)-4-N-propylcarbamoyl-phenylamino]-4-(2-acetyl-phenylamino)-5-trifluormethyl-pyrimidin

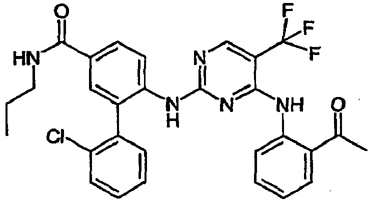

50 mg (0,16 mmol) 4-(2-Acetyl-phenylamino)-2-chlor-5-trifluormethyl-pyrimidin (Methode 10) werden in 0,2 ml 1,4-Dioxan gelöst, mit 60 mg (0,17 mmol) 4-Amino-3-(2-chlor-phenoxy)-*N*-propyl-benzamid hydrochlorid (Methode 11) versetzt und 3 Tage bei 50 °C gerührt. Anschließend wird das Lösungsmittel im Vakuum entfernt und das Rohprodukt durch Säulenchromatographie gereinigt, wobei als Trägermaterial C18 RP-Gel verwendet wird.Das Produkt wird mit einem Gradient eluiert, dessen Startbedingungen aus Wasser :
Acetonitril = 95% : 5% bestehen und der sich innerhalb von 20 min auf die
Endbedingungen Wasser : Acetonitril = 5% : 95% ändert.
Ausbeute: 64 mg (0,088 mmol; 55 %) eines gelben Feststoffs
MS (ESI): 584/586 (M+H)⁺ Isomerenmuster ³⁵Cl / ³⁷Cl
UV max: 282 nm

### Beispiele 58 - 63

Die folgenden Verbindungen sind über ein analoges Verfahren, wie in Beispiel 57 beschrieben, hergestellt. Dabei wird 4-(2-Acetyl-phenylamino)-2-chlor-5-trifluormethyl-pyrimidin und ein entsprechendes Anilin, welches in Methode 11 beschrieben ist, verwendet.

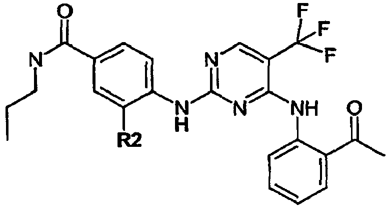

| **#** | **R2** | **UV max [nm]** | **MS (ESI) (M+H)⁺** |
|---|---|---|---|
| 58 | 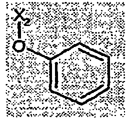 | 278 | 550 |
| 59 | 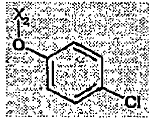 | 282 | 584 |
| 60 | 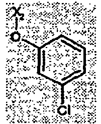 | 243 | 584 |
| 61 | 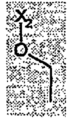 | 318 | 502 |
| 62 | 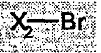 | 286 | 536 |

### Beispiel 63

### 2-[2-Methoxy-4-(4-morpholin-4-yl-(1,4-trans-cyclohexyl)carbamoyl)-phenylamino]-4-(2-carbamoyl-3-fluor-phenylamino)-5-trifluormethyl-pyrimidin

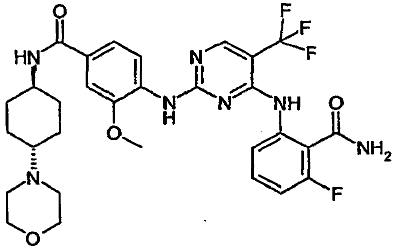

200 mg (0,49 mmol) 2-(4-Carboxyamino-2-methoxy-phenylamino)-4-chlor-5-trifluormethyl-pyrimidin (Methode 12) werden in 0,5 ml *N*-Methyl-2-pyrrolidinon gelöst und mit 83mg (0,54 mmol) 2-Amino-6-fluor-benzamid versetzt. Diesem Reaktionsgemisch werden 120 µl einer 4 M Lösung von HCl (0,49 mmol) in 1,4-Dioxan zudosiert.

Nach 16 h bei 90 °C wird das Reaktionsgemisch in 150 ml einer wässrigen 1 N Salzsäure eingerührt. Der Niederschlag wird abfiltriert und im Vakuum getrocknet. 50 mg (0,11 mmol) dieses Niederschlages, 94 µl (0,54 mmol) *N*-Ethyldiisopropylamin, 45 mg (0,13 mmol) TBTU und 30 mg (0,16 mmol) 4-(4-Amino-cyclohexyl)-morpholin (Methode 13) werden in 0,4 ml Tetrahydrofuran gelöst.

Nach 15 h bei Raumtemperatur wird das Lösungsmittel im Vakuum entfernt. Das Rohprodukt wird durch Säulenchromatographie gereinigt. Als Trägermaterial dient Kieselgel und als Eluent wird Dichlormethan, dem 5% einer Mischung aus 90% Methanol und 10% gesättigter wässriger Ammoniak-Lösung zugefügt sind, verwendet.
Ausbeute: 43 mg (0,068 mmol; 62 %) eines hellgelben Feststoffs
MS (ESI): 633 (M+H)⁺

### Beispiele 64 - 71

Die folgenden Verbindungen sind über ein analoges Verfahren, wie in Beispiel 63 beschrieben, hergestellt. Das entsprechende Anilin ist in Methode 6,14 oder 15 beschrieben. Das für die Darstellung des Amids eingesetzte Amin ist kommerziell erhältlich oder ist in Methode 13 beschrieben.

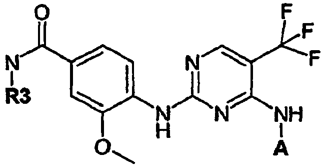

| **#** | **A** | **R3** | **UV max [nm]** | **MS (ESI) (M+H)⁺** |
|---|---|---|---|---|
| 64 | 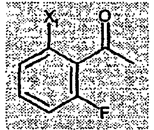 | 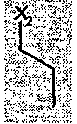 | 284,246 | 506 |
| 65 | 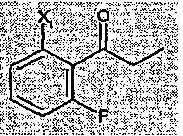 | 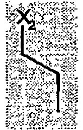 | 243 | 520 |
| 66 | 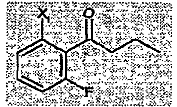 | 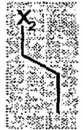 | 319 | 534 |
| 67 | 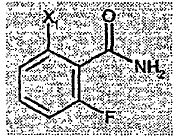 | 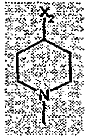 | 320 | 562 |
| 68 | 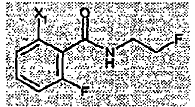 | 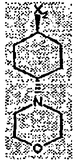 | 318 | 678 |
| 69 | 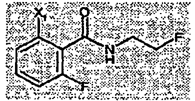 | 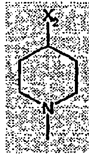 | 318 | 608 |
| 70 | 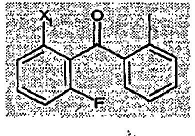 | 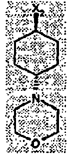 | 314 | 707 |
| 71 | 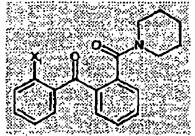 | 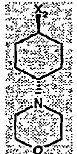 | 318 | 786 |

### Beispiele 72 - 76

Die folgenden Verbindungen sind über ein analoges Verfahren, wie in Beispiel 1 beschrieben, hergestellt. Dabei wird 2-(2-Methoxy-4-propylcarbamoyl-phenylamino)-4-chlor-5-trifluormethylpyrimidin und ein Anilin, welches entweder kommerziell erhältlich oder in Methode 6, 16 beziehungsweise 17 beschrieben ist, verwendet.

Als Lösungsmittel wird 1,4-Dioxan, *N*-Methyl-2-pyrrolidinon oder *N,N-*Dimethylacetamid verwendet

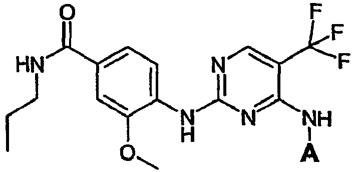

| **#** | **A** | **Salzform** | **UV max [nm]** | **MS (ESI) (M+H)⁺** |
|---|---|---|---|---|
| 72 | 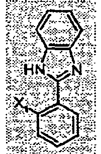 | Base | 282, 322 | 562 |
| 73 | 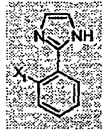 | Base | 226, 278, 318 | 512 |
| 74 | 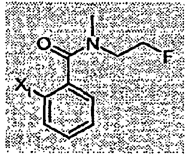 | Base | 314, 283, 230 | 549 |
| 75 | 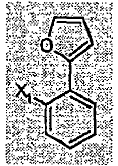 | Base | | 512 |
| 76 | 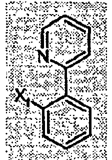 | Base | 234, 274, 318 | 523 |

### Beispiele 77 -112

Die folgenden Verbindungen sind über ein analoges Verfahren, wie in Beispiel 63 beschrieben, hergestellt. Das entsprechende Anilin ist kommerziell erhältlich. Das für die Darstellung des Amids eingesetzte Amin ist kommerziell erhältlich oder in Methode 13 beziehungsweise 18 beschrieben.

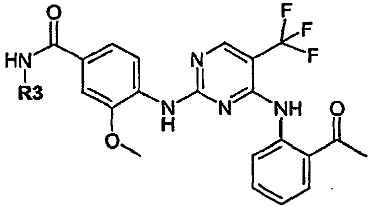

| **#** | **R3** | **Salzform** | **UV max [nm]** | **MS (ESI) (M+H)⁺** |
|---|---|---|---|---|
| 77 | 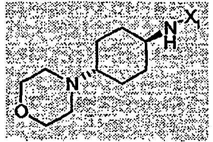 | Dihydro-chlorid | 282, 318 | 613 |
| 78 | 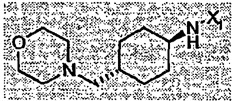 | Dihydro-chlorid | 282, 318 | 627 |
| 79 | 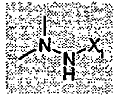 | Dihydro-chlorid | 286, 322 | 489 |
| 80 | 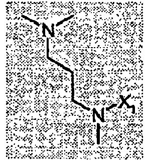 | Base | | 545 |
| 81 | 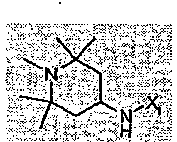 | Base | | 599 |
| 82 | 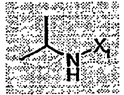 | Base | | 488 |
| 83 | 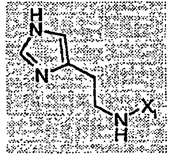 | Base | | 540 |
| 84 | 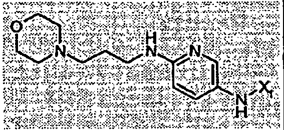 | Base | | 665 |
| 85 | 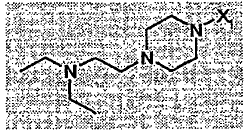 | Base | | 614 |
| 86 | 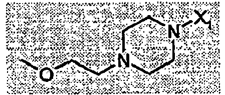 | Base | | 573 |
| 87 | 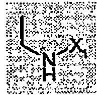 | Base | | 474 |
| 88 | 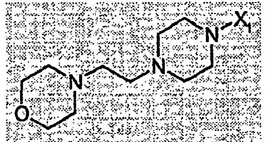 | Base | | 628 |
| 89 | 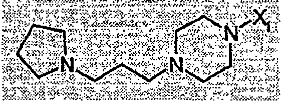 | Base | | 626 |
| 90 | 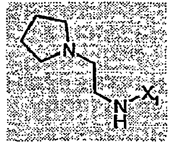 | Base | | 543 |
| 91 | 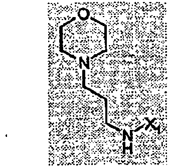 | Base | | 573 |
| 92 | 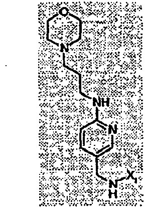 | Base | | 679 |
| 93 | 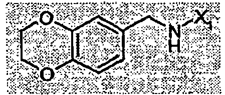 | Base | | 594 |
| 94 | 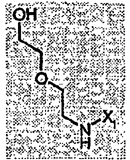 | Base | | 534 |
| 95 | 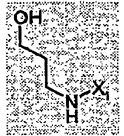 | Base | | 504 |
| 96 | 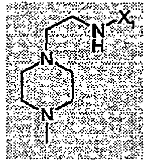 | Base | | 572 |
| 97 | 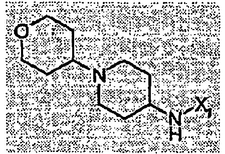 | Base | | 613 |
| 98 | 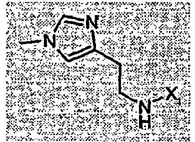 | Base | | 554 |
| 99 | 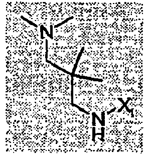 | Base | | 559 |
| 100 | 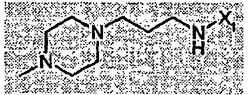 | Base | | 586 |
| 101 | 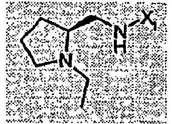 | Base | | 557 |
| 102 | 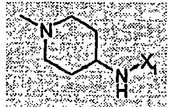 | Base | | 543 |
| 103 | 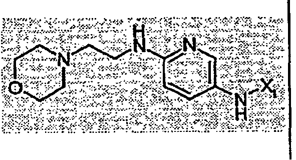 | Base | | 651 |
| 104 | 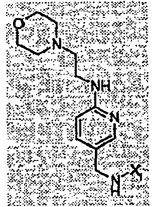 | Base | | 665 |
| 105 | 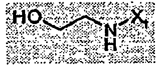 | Base | | 490 |
| 106 | 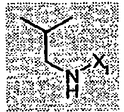 | Base | | 502 |
| 107 | 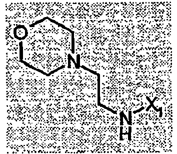 | Base | | 559 |
| 108 | 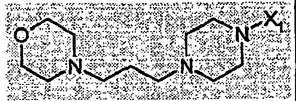 | Base | | 642 |
| 109 | 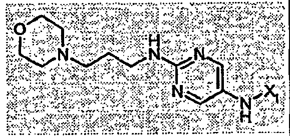 | Base | | 666 |
| 110 | 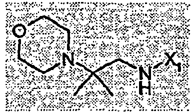 | Base | | 587 |
| 111 | 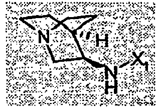 | Base | | 555 |
| 112 | 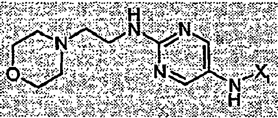 | Base | | 652 |

### Beispiele 113 - 119

Die folgenden Verbindungen sind über ein analoges Verfahren, wie in Beispiel 57 beschrieben, hergestellt. Dabei wird 4-(2-Acetyl-phenylamino)-2-chlor-5-trifluormethyl-pyrimidin und ein entsprechendes Anilin, welches in Methode 11 beziehungsweise 19 beschrieben ist, verwendet.

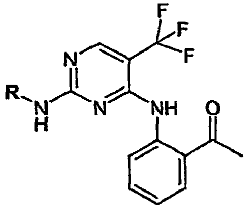

| **#** | **R** | **Salzform** | **UV max [nm]** | **MS (ESI) (M+H)⁺** |
|---|---|---|---|---|
| 113 | 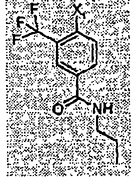 | Base | 246 | 526 |
| 114 | 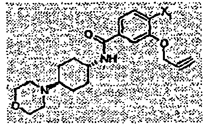 | Dihydro-chlorid | 226, 246, 282, 314 | 637 |
| 115 | 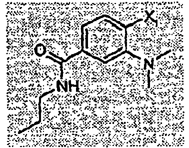 | Base | 302 | 501 |
| 116 | 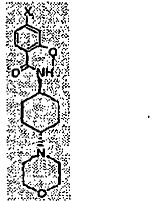 | Diformiat | 314 | 613 |
| 117 | 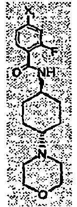 | Diformiat | 298 | 601 |
| 118 | 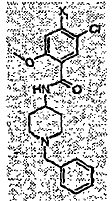 | Base | 286 | 653 |
| 119 | 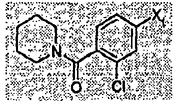 | Base | | 518 |

### Beispiele 120 - 144

Die folgenden Verbindungen sind über ein analoges Verfahren, wie in Beispiel 63 beschrieben, hergestellt. Das entsprechende Anilin ist kommerziell erhältlich oder ist in Methode 6 beziehungsweise 20 beschrieben. Das für die Darstellung des Amids eingesetzte Amin ist kommerziell erhältlich oder ist in Methode 13 beschrieben.

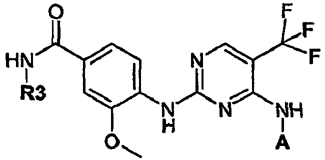

| **#** | **A** | **R3** | **Salzform** | **UV max [nm]** | **MS (ESI) (M+H)⁺** |
|---|---|---|---|---|---|
| 120 | 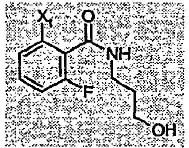 | 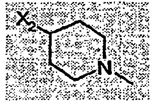 | Base | 316, 282, 234 | 620 |
| 121 | 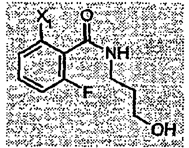 | 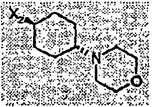 | Base | 316, 282, 234 | 690 |
| 122 | 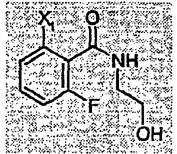 | 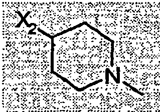 | **Base** | 314, 278, 235 | 606 |
| 123 | 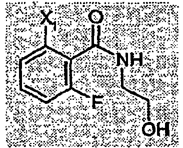 | 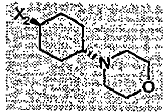 | Base | 314, 278, 238 | 676 |
| 124 | 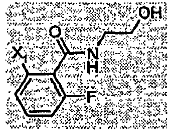 | 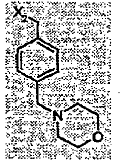 | Base | 318, 282, 226 | 698 |
| 125 | 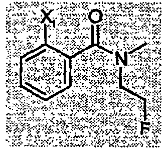 | 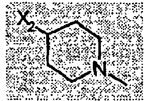 | Hydro-chlorid | 316 | 604 |
| 126 | 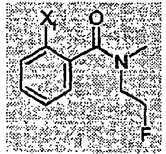 | 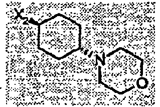 | Dihydrochlorid | 314 | 674 |
| 127 | 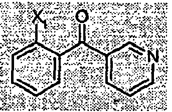 | 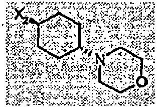 | Base | 330 | 676 |
| 128 | 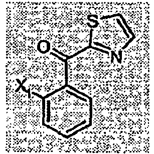 | 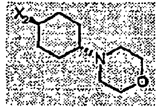 | Base | 314 | 683 |
| 129 | 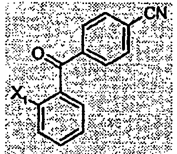 | 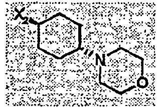 | Base | 254 | 700 |
| 130 | 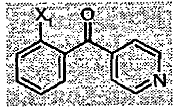 | 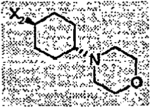 | Trihydro-chlorid | 316 | 676 |
| 131 | 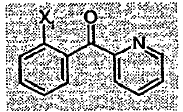 | 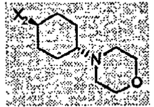 | Base | 313 | 671 (M-H)⁻ |
| 132 | 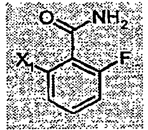 | 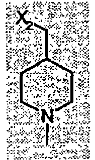 | Dihydrochlorid | 230, 282, 318 | 576 |
| 133 | 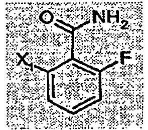 | 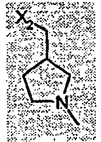 | Dihydro-chlorid | 278,314 | 562 |
| 134 | 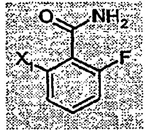 | 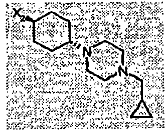 | Trihydro-chlorid | 230, 282, 318 | 685 |
| 135 | 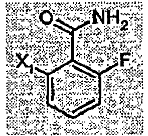 | 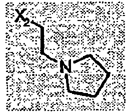 | Dihydrochlorid | 282, 318 | 562 |
| 136 | 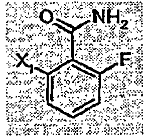 | 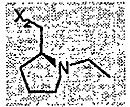 | Dihydro-chlorid | 282,318 | 576 |
| 137 | 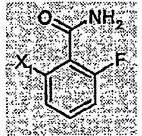 | 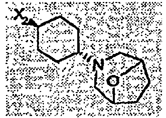 | Dihydro-chlorid | 282, 318 | 658 |
| 138 | 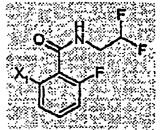 | 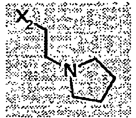 | Dihydro-chlorid | 226, 282, 318 | 626 |
| 139 | 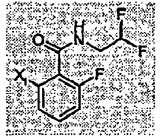 | 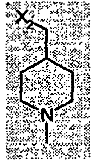 | Dihydro-chlorid | 230, 282, 318 | 640 |
| 140 | 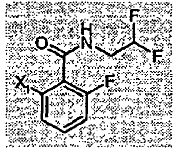 | 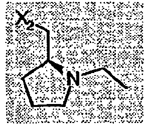 | Dihydrochlorid | 230,282, 322 | 640 |
| 141 | 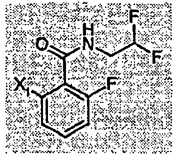 | 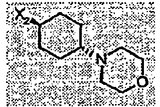 | Dihydrochlorid | 230, 282, 318 | 696 |
| 142 | 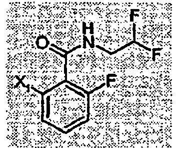 | 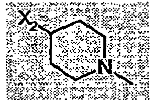 | Dihydro-chlorid | 230, 282, 318 | 626 |
| 143 | 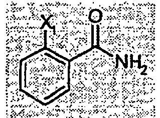 | 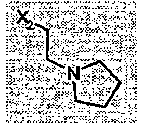 | Dihydro-chlorid | 284, 325 | 544 |
| 144 | 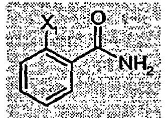 | 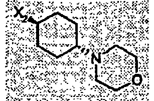 | Dihydro-chlorid | 283, 317 | 614 |

### Methode 1

### 2-(2-Methoxy-4-propylcarbamoyl-phenylamino)-4-chlor-5-trifluormethyl-pyrimidin

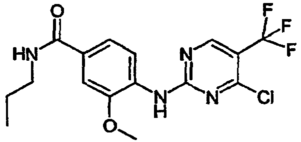

5 g (21,9 mmol) 2,4-Dichlor-5-trifluormethyl-pyrimidin werden in 50 ml 1,4-Dioxan gelöst und mit 5,50 g (21,9 mmol) 4-Amino-3-methoxybenzoesäure-propylamid Hydrochlorid (Journal of Pharmaceutical Sciences 1989, 78(10), 829-32) versetzt. Diesem Reaktionsgemisch werden 7,50 ml (43,8 mmol) Ethyldiisopropylamin zugesetzt und 2 Tage bei Raumtemperatur gerührt. Anschließend wird das Reaktionsgemisch mit 250 ml Ethylacetat verdünnt und zunächst mit 300 ml wässriger 10%-iger KHSO₄-Lösung, dann mit 300 ml gesättigter, wässriger NaCl-Lösung gewaschen. Die organische Phase wird mit MgSO₄ getrocknet und das Lösungsmittel im Vakuum entfernt.
Das Rohprodukt wird durch Säulenchromatographie gereinigt. Als Trägermaterial dient Kieselgel und als Eluent wird ein Gemisch bestehend aus Cyclohexan: Ethylacetat (75:25) verwendet.
Ausbeute: 2,30 g (5,9 mmol; 27 %)

### Methode 2

### 2-Amino-6-methyl-benzamid

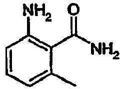

5,25 g (28,1 mmol) 2-Methyl-6-nitrobenzoesäure werden zu 250 ml Thionylchlorid gegeben und unter Rückfluss 3 h erhitzt. Anschließend wird das Thionylchlorid im Vakuum entfernt. Vom dem Rückstand werden 2,93 g (14,7 mol) in 50 ml THF gelöst, auf 0 °C heruntergekühlt und mit 44 ml einer wässrigen, 32%-igen Ammoniak-Lösung versetzt. Dieses Reaktionsgemisch wird über Nacht unter Rühren auf Raumtemperatur erwärmt. Anschließend wird das Reaktionsgemisch mit 100 ml Ethylacetat verdünnt und dreimal mit je 50 ml einer gesättigten, wässrigen NaCl-Lösung gewaschen. Die organische Phase wird mit MgSO₄ getrocknet und das Lösungsmittel im Vakuum entfernt.
2,44 g (13,5 mmol) dieses Reaktionsproduktes werden in 100 ml THF gelöst und mit 200 mg Pd auf Kohle (10% Pd) versetzt. Das Reaktionsgemisch wird 16 h bei 3 bar H₂-Druck und Raumtemperatur hydriert. Danach wird der Katalysator abfiltriert und das Lösungsmittel im Vakuum entfernt.

| | |
|---|---|
| Ausbeute: | 2,03 g (13,5 mol; 48%) |

Analog zu dieser Methode werden folgende Verbindungen hergestellt:

| | **MS (ESI) (M+H)⁺** | | **MS (ESI) (M+H)⁺** |
|---|---|---|---|
| 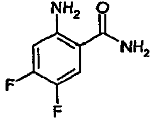 | 173 | 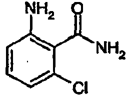 | 171 |

### Methode 3

### 2-Methylamino-benzamid

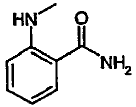

2 g (16,5 mmol) 2-Fluorbenzonitril werden mit 10 ml (80 mmol) einer 8 M MethylaminLösung in Ethanol versetzt und 20 h bei 100 °C in einem Druckgefäß erhitzt. Anschließend wird das Reaktionsgemisch mit 200 ml Ethylacetat verdünnt und dreimal mit je 160 ml einer wässrigen 10%-igen NaCl-Lösung gewaschen. Die organische Phase wird mit MgSO₄ getrocknet und das Lösungsmittel im Vakuum entfernt.

1 g (7,6 mmol) dieses Reaktionsproduktes werden mit 20 ml einer 20%-igen wässrigen Schwefelsäure versetzt und 3 h bei 80 °C gerührt. Anschließend wird das Reaktionsgemisch mit 200 ml Ethylacetat verdünnt und dreimal mit je 160 ml einer wässrigen 10%-igen NaCl-Lösung gewaschen. Danach wird die organische Phase mit MgSO₄ getrocknet, das Lösungsmittel im Vakuum entfernt Das Rohprodukt wird durch Säulenchromatographie gereinigt. Als Trägermaterial dient Kieselgel und als Eluent wird Dichlormethan, dem 15% einer Mischung aus 90% Methanol und 10% gesättigter wässriger Ammoniak-Lösung zugefügt sind, verwendet.
Ausbeute: 777 mg (5,2 mmol; 32%)
MS (ESI): 151 (M+H)⁺

### Methode 4

### 2-Amino-6-methyl-benzoesäure-N'-N'-dimethyl-hydrazid

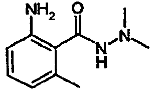

2-Dimethylamino-7-nitro-2,3-dihydro-isoindol-1-on ist analog zu den Verbindungen, die in Journal of the Chemical Society of Pakistan 1985, 7(1), 69-70, WO 03/14315 und US 5716993 beschrieben sind, hergestellt.
240 mg (1,1 mmol) 2-Dimethylamino-7-nitro-2,3-dihydro-isoindol-1-on werden in 70 ml Dimethylformamid und 50 ml Methanol gelöst und mit 100 mg Pd auf Kohle (10% Pd) versetzt. Das Reaktionsgemisch wird 16 h bei 3 bar H₂-Druck und Raumtemperatur hydriert. Anschließend wird der Katalysator abfiltriert und das Lösungsmittel im Vakuum entfernt.

| | |
|---|---|
| Ausbeute: | 204 mg (1,0 mmol; 91%) |
| MS (ESI): | 194 (M+H)⁺ |

### Methode 5

### 2-Amino-6-trifluormethyl-benzamid

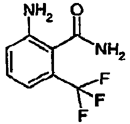

100 mg (0,5 mmol) 2-Fluor-6-trifluormethyl-benzonitril werden mit 1 ml einer 7 M Ammoniak-Lösung in Methanol versetzt und 20 min. bei 100 °C in der Mikrowelle erhitzt. Anschließend wird das Lösungsmittel im Vakuum entfernt, das Rohprodukt mit 1 ml konz. H₂SO₄ versetzt und 2,5 h bei 80 °C erhitzt Danach wird das Reaktionsgemisch in Eiswasser eingerührt, mit 1 M NaOH neutralisiert und zweimal mit je 150 ml Dichlormethan und dreimal mit je 160 ml Ethylacetat extrahiert Die organische Phase wird mit MgSO₄ getrocknet, das Lösungsmittel im Vakuum entfernt Das Rohprodukt wird durch Säulenchromatographie gereinigt. Als Trägermaterial dient Kieselgel und als Eluent wird Dichlormethan, dem 4% einer Mischung aus 90% Methanol und 10% gesättigter wässriger Ammoniak-Lösung zugefügt sind, verwendet

| | |
|---|---|
| Ausbeute: | 31 mg (0,152mmol; 30 %) |
| MS (ESI): | 205 (M+H)⁺ |

### Methode 6

### 2-Amino-N-(2-dimethylamino-ethyl)-6-fluor-benzamid

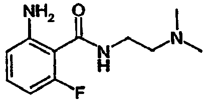

500 mg (3,2 mmol) 2-Amino-6-fluorbenzoesäure, 284 µl (3,2 mmol) 2-*N,N-*Dimethylaminoethylamin und 563 µl (3,2 mmol) Diisopropylethylamin werden in 2 ml Tetrahydrofuran gelöst und mit 1,08 ml (3,2 mmol) TBTU versetzt. Diese Reaktionsmischung wird 2,5 h bei Raumtemperatur gerührt. Anschließend wird die Reaktionsmischung mit 100 ml 10 %iger wässrigen Kaliumhydrogencarbonat-Lösung versetzt und sechsmal mit je 100 ml Ethylacetat extrahiert. Danach wird die organische Phase mit MgSO₄ getrocknet, das Lösungsmittel im Vakuum entfernt.

Das Rohprodukt wird durch Säulenchromatographie gereinigt. Als Trägermaterial dient Kieselgel und als Eluent wird Dichlormethan, dem 8% einer Mischung aus 90% Methanol und 10% gesättigter wässriger Ammoniak-Lösung zugefügt sind, verwendet.
Ausbeute: 154 mg (21 %)
MS (ESI): 226 (M+H)⁺

Analog zu dieser Methode werden folgende Verbindungen hergestellt Dabei wird 2-Amino-6-fluorbenzoesäure oder 2-Amino-benzoesäure und ein entsprechendes kommerziell erhältliches Amin verwendet.

| | **MS (ESI) (M+H)⁺** | | **MS (ESI) (M+H)⁺** |
|---|---|---|---|
| 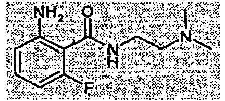 | 226 | 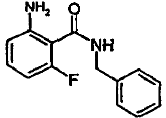 | |
| 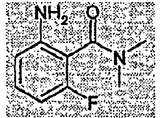 | 183 | 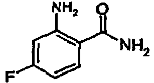 | |
| 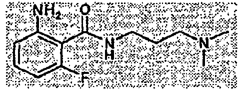 | 240 | 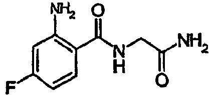 | |
| 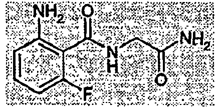 | 212 | 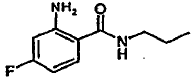 | |
| 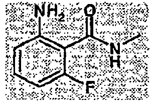 | 169 | 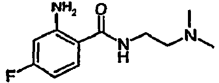 | |
| 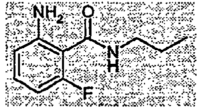 | 197 | 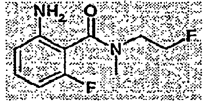 | 215 |
| 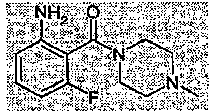 | 238 | 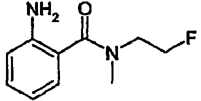 | 197 |
| 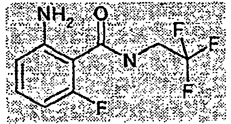 | 237 | 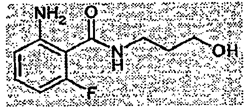 | 213 |
| 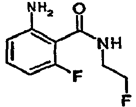 | 201 | 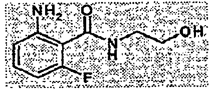 | 199 |
| 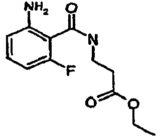 | | 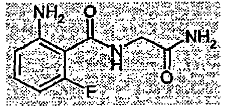 | 212 |
| 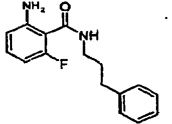 | | 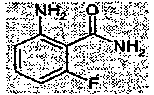 | 155 |
| 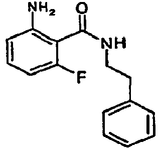 | 245 | 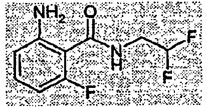 | 219 |

### Methode 7

### 2-Amino-6-(2-amino-ethoxy)-benzamid

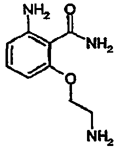

444 µl (7,4 mmol) Ethanolamin werden in 4 ml 1,4-Dioxan gelöst, mit 312 mg (7,8 mmol) Natriumhydrid versetzt und 30 min bei Raumtemperatur gerührt. Diesem Reaktionsgemisch werden 1 g (7,4 mmol) 2-Amino-6-fluorbenzonitril zudosiert und 6 Tage bei Raumtemperatur gerührt. Anschließend wird das Lösungsmittel im Vakuum entfernt. Das Rohprodukt wird durch Säulenchromatographie gereinigt. Als Trägermaterial dient Kieselgel und als Eluent wird Dichlormethan, dem 10% einer Mischung aus 90% Methanol und 10% gesättigter wässriger Ammoniak-Lösung zugefügt sind, verwendet. 434 mg (2,5 mmol) des gereinigten Zwischenproduktes werden in 5 ml einer 20%-igen Kaliumhydroxid-Lösung in Ethanol gelöst und 2 Tage bei 90 °C gerührt. Anschließend wird das Lösungsmittel im Vakuum entfernt.
Das Rohprodukt wird durch Säulenchromatographie gereinigt. Als Trägermaterial dient Kieselgel und als Eluent wird Dichlormethan, dem 20% einer Mischung aus 90% Methanol und 10% gesättigter wässriger Ammoniak-Lösung zugefügt sind, verwendet.
Ausbeute: 164 mg (0,841 mmol; 11 %)
MS (ESI): 196 **(M+H)⁺**

Analog zu dieser Methode werden folgende Verbindungen hergestellt Dabei wird 2-Amino-6-fluorbenzonitril und ein entsprechendes kommerziell erhältlicher Alkohol verwendet.

| | **MS (ESI) (M+H)⁺** | | **MS (ESI) (M+H)⁺** |
|---|---|---|---|
| 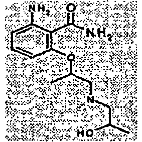 | 268 | 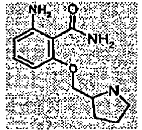 | 236 |
| 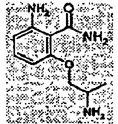 | 210 | 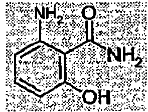 | 153 |
| 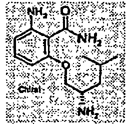 | 252 | 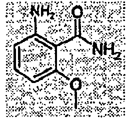 | |
| 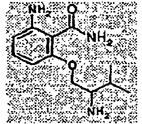 | 238 | | |

### Methode 8

### 2-Amino-benzoesäure-isopronylester

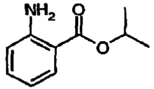

1,0 g (7,3 mmol) Anthranilsäure werden in 5 ml Propan-2-ol gelöst, mit 795 µl (10,9 mmol) Thionylchlorid versetzt und 3 Tage unter Rückfluss erhitzt. Anschließend wird Thionylchlorid im Vakuum entfernt, der Rückstand in 50 ml dest Wasser aufgenommen und dreimal mit je 30 ml Ethylacetat extrahiert. Die organische Phase wird mit 20 ml einer gesättigten wässrigen NaHCO₃-Lösung gewaschen, mit MgSO₄ getrocknet und das Lösungsmittel im Vakuum entfernt.

| | |
|---|---|
| Ausbeute: | 156 mg (0,872 mmol; 12 %) |
| MS (ESI): | 180 (M+H)⁺ |

Analog zu dieser Methode werden folgende Verbindungen hergestellt. Dabei wird Anthranilsäure und ein entsprechendes kommerziell erhältlicher Alkohol verwendet.

| | **MS (ESI) (M+H)⁺** | | **MS (ESI) (M+H)⁺** |
|---|---|---|---|
| 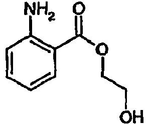 | 182 | 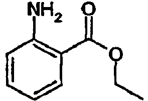 | 166 |

### Methode 9

### 1-(2-Amino-phenyl)-propan-1-on

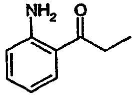

100 mg (0,6 mmol) *N*-(2-Formyl-phenyl)-acetamid (Angew. Chem., Int. Ed. 2002, 41(16), 3028-31) werden in 4 ml Tetrahydrofuran gelöst und auf -78 °C abgekühlt. Anschließend werden bei dieser Temperatur 406 µl (1,2 mmol) einer 3 M Lösung von Ethylmagnesiumbromid in Diethylether zugegeben. Dieses Reaktionsgemisch läßt man über Nacht unter Rühren auf Raumtemperatur kommen. Danach wird das Reaktionsgemisch in 30 ml dest Wasser eingerührt und dreimal mit je 10 ml Ethylacetat extrahiert. Die organische Phase wird mit MgSO₄ getrocknet, das Lösungsmittel im Vakuum entfernt Das Rohprodukt wird durch Säulenchromatographie gereinigt. Als Trägermaterial dient Kieselgel und als Eluent wird ein Gemisch bestehend aus Cyclohexan:Ethylacetat (1:1) verwendet.
93 mg (0,5 mmol) dieses Zwischenproduktes werden in 6 ml Dichlormethan gelöst, mit 209 mg (2,4 mmol) Mangan(IV)-oxid versetzt und 3 Tage bei Raumtemperatur gerührt. Anschließend wird das überschüssiges Mangan(IV)-oxid über Celite abfiltriert und das Lösungsmittel im Vakuum entfernt. Das Rohprodukt wird durch Säulenchromatographie gereinigt Als Trägermaterial dient Kieselgel und als Eluent wird ein Gemisch bestehend aus Cyclohexan: Ethylacetat (85:15) verwendet.
44 mg (0,23 mmol) dieses Zwischenproduktes werden in 2 ml Ethanol gelöst, mit 4 ml einer wässrigen 1 N Salzsäure versetzt und 2 Tage bei 40 °C gerührt. Anschließend wird das Reaktionsgemisch in 30 ml dest Wasser eingerührt, mit Na₂CO₃ auf pH 7 eingestellt und dreimal mit je 10 ml Ethylacetat extrahiert Die organische Phase mit MgSO₄ getrocknet und das Lösungsmittel im Vakuum entfernt.

| | |
|---|---|
| Ausbeute: | 25 mg (0,168 mmol; 28 %) |
| MS (ESI): | 150 (M+H)⁺ |

Analog zu dieser Methode werden folgende Verbindungen hergestellt Dabei geht man von *N*-(2-Formyl-phenyl)-acetamid und einer entsprechenden kommerziell erhältlichen Grignad-Verbindung aus.

| | **MS (ESI) (M+H)⁺** | | **MS (ESI) (M+H)⁺** |
|---|---|---|---|
| 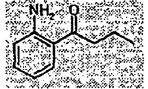 | 164 | 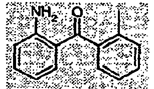 | 212 |
| 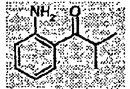 | 164 | | |

### Methode 10

### 4-(2-Acetyl-phenylamino)-2-chlor-5-trifluormethyl-pyrimidin

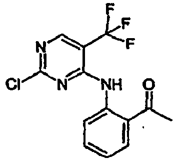

4,23 g (18,5 mmol) 2,4-Dichlor-5-trifluor-methyl-pyrimidin (J. Org. Chem. 1965, 30(3), 835) werden in 2 ml Tetrahydrofuran gelöst, auf - 40 °C abgekühlt und mit 2,20 g (16,0 mmol) 2-Aminoacetophenon und 7,77 ml (44,5 mmol) *N*-Ethyldiisopropylamin versetzt. Nach 3 Tagen bei Raumtemperatur wird das Lösungsmittel im Vakuum entfernt.
Das Rohprodukt wird durch Säulenchromatographie gereinigt. Als Trägermaterial dient Kieselgel und als Eluent wird Dichlormethan verwendet.
Ausbeute: 1,38 g (4,4 mmol; 24 %)
MS (ESI): 316/318 (M+H)⁺ Isotopenverteilung ^{3s}Cl/³⁷Cl

### Methode 11

### 4-Amino-3-(2-chlor-phenoxy)-N-propyl-benzamid

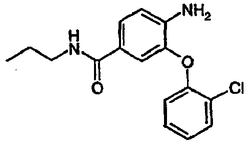

8,4 g (65,3 mmol) Chlorphenol werden in 60 ml *N,N*-Dimethylformamid gelöst und mit 5 g (36,2 mmol) Kaliumcarbonat versetzt. Man erwärmt auf 55° C und tropft 15 g (66,3 mmol) 3-Fluoro-4-nitro-*N*-propyl-benzamide, welches in 20 ml *N,N-*Dimethylformamid gelöst ist, zu und rührt 16 h bei 55 °C. Das Lösungsmittel wird im Vakuum entfernt und der Rückstand mit 100 ml Wasser versetzt. Die entstehenden Kristalle werden abgesaugt und getrocknet.
14 g (41,8 mmol) dieses Zwischenproduktes werden in 150 ml THF gelöst und mit 1 g Raney-Nickel versetzt. Die Reaktionsmischung wird 16 h bei RT und 3 bar Wasserstoff-Druck gerührt. Anschließend wird vom Katalysator abfiltriert und das Lösungsmittel im Vakuum entfernt. Der Rückstand wird in Butylmethylether aufgenommen und mit 16 ml einer 5 molaren Lösung von HCl (80 mmol) in Isopropanol versetzt. Die Kristalle werden abgesaugt und getrocknet.

| | |
|---|---|
| Ausbeute: | 13,5 g (44,4 mmol; 68 %) |
| MS (ESI): | 305/307 (M+H)⁺Isotopenverteilung ³⁵Cl/³⁷Cl |

Analog zu dieser Methode werden folgende Verbindungen hergestellt

| | **MS (ESI) (M+H)⁺** | | **MS (ESI) (M+H)⁺** |
|---|---|---|---|
| 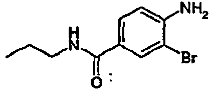 | 257/259 | 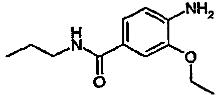 | |
| 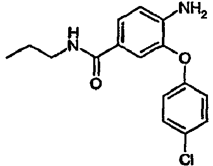 | | 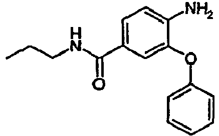 | |
| 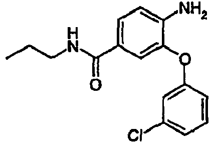 | | | |

### Methode 12

### 2-(4-Carboxyamino-2-methoxy-phenylamino)-4-chlor-5-trifluormethyl-pyrimidin

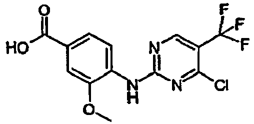

7,36 g (44 mmol) 4-Amino-3-methoxybenzoesäure werden in 80 ml einer wässrigen Phosphatpuffer-Lösung (pH 6,3) suspendiert und mit 9,5 g (44 mmol) 2,4-Dichlor-5-trifluor-methyl-pyrimidin, welches in 240 ml 1,4-Dioxan gelöst ist, versetzt. Nach 4 h bei 100 °C wird das Reaktionsgemisch bei 0 °C zur Auskristallisation gebracht Der Niederschlag wird abfiltriert, das Filtrat wird mit 150 ml Ethylacetat versetzt und zweimal mit je 200 ml einer gesättigten wässrigen Natriumhydrogencarbonat-Lösung gewaschen. Die organische Phase wird mit MgSO₄ getrocknet und das Lösungsmittel im Vakuum entfernt. Das Rohprodukt wird in 10 ml n-Hexan suspendiert und unter Rückfluss erhitzt.
Der Niederschlag wird abfiltriert, in 48 ml einer gesättigten wässrigen Natriumhydrogencarbonat-Lösung suspendiert und 1 h bei 65 °C erhitzt. Anschließend wird die Lösung bei 0 °C zur Auskristallisation gebracht. Der Niederschlag wird abfiltriert, das Filtrat wird mit 1 N wässriger Salzsäure angesäuert und mit 100 ml Essigester versetzt. Die organische Phase wird abgetrennt, mit Magnesiumsulfat getrocknet und das Lösungsmittel im Vakuum entfernt. Der Rückstand wird in Ethylacetat umkritallisiert.
Ausbeute: 330 mg (0,95 mmol, 2 %)
MS (ESI): 348 **(M+H)⁺**

### Methode 13

### 4-(4-Amino-cyclohexyl)-morpholin

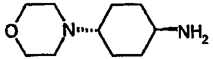

### Dibenzyl-4-morpholino-cyclohexylamin

3,9 g (30 mmol)) 4-Dibenzylcyclohexanon werden in 100 mL Dichlormethan gelöst und mit 3,9 g (45 mmol) Morpholin und 9,5 g (45 mmol) Natriumtriacetoxyborhydrid 12 h bei RT gerührt. Anschließend wird mit Wasser und Kaliumcarbonat versetzt, die organische Phase abgetrennt, getrocknet und im Vakuum das Lösungsmittel abgezogen.
Das Rohprodukt wird durch Säulenchromatographie gereinigt. Als Trägermaterial dient Kieselgel und als Eluent wird Essigester, dem 10% einer Mischung aus 90% Methanol und 10% gesättigter wässriger Ammoniak-Lösung zugefügt sind, verwendet. Die geeigneten Fraktionen werden im Vakuum eingeengt.

| | |
|---|---|
| Ausbeute: | 6,6 g (18 mmol, 60%) cis-Isomer |
| | 2 g (5,4 mmol, 18%) trans-Isomer |

### trans-4-Morpholino-cyclohexylamin

7,2 g (16,4 mmol) trans-Dibenzyl-4-morpholino-cyclohexylamin werden in 100 mL MeOH gelöst und an 1,4 g Pd/C (10%) bei 30-50 °C hydriert Das Lösungsmittel wird im Vakuum abgezogen und der Rückstand aus Ethanol und konz. HCl kristallisiert.
Ausbeute: 3,9 g (15,2 mmol, 93%)
Schmelzpunkt: 312 °C

Analog zu Methode 13 werden folgende Amine hergestellt:

| | **MS (ESI) (M+H)⁺** | | **MS** (**ESI**) **(M+H)⁺** |
|---|---|---|---|
| 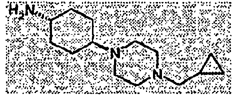 | 238 | 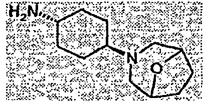 | 211 |

### Methode 14

### (2-Amino-6-fluor-phenyl)-o-tolyl-methanon

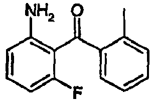

### 2-Amino-6-fluor-benzoesäuremethylester

5 g (31,6 mmol) 2-Amino-6-fluorbenzoesäure werden in 50 ml Methanol gelöst und mit 7,9 ml (31,6 mmol) einer 4 M Lösung von HCL in 1,4-Dioxan versetzt und 10 min. bei Raumtemperatur gerührt. Das Reaktionsgemisch wird tropfenweise mit 3,45 ml (47,4 mmol) Thionylchlorid versetzt und unter Rückfluss 3 h erhitzt. Anschließend wird das Reaktionsgemisch mit 150 ml einer gesättigten, wässrigen NaHCO₃-Lösung neutralisiert und viermal mit je 100 ml Ethylacetat extrahiert. Die organische Phase wird mit MgSO₄ getrocknet und das Lösungsmittel im Vakuum entfernt.

| | |
|---|---|
| Ausbeute: | 1,57 g (9,3 mmol, 29 %) |

### (2-Amino-6-fluor-phenyl)-methanol

676,3 mg (17,8 mmol) Lithiumaluminiumhydrid werden unter N₂-Atmospäre in 65 ml Diethylether vorgelegt und auf 0 °C abgekühlt. Dazu werden langsam 2,01 g (11,9 mmol) 2-Amino-6-fluor-benzoesäuremethylester, gelöst in 65 ml Diethylether, getropft und 2 h bei 0 °C gerührt. Anschließend wird das Reaktionsgemisch bei 0 °C tropfenweise mit 100 ml dest. H₂O versetzt. Die wässrige Phase wird zweimal mit je 100 ml Diethylether extrahiert. Die vereinigten organischen Phasen werden einmal mit 100 ml einer gesättigten, wässrigen NaCl-Lösung gewaschen, mit MgSO₄ getrocknet und das Lösungsmittel im Vakuum entfernt.
Ausbeute: 1,44 g (10,2 mmol, 86 %)

### 2-Amino-6-fluor-benzaldehyd

1,44 g (10,2 mmol) (2-Amino-6-fluor-phenyl)-methanol werden in 120 ml Chloroform gelöst, mit 4,43 g (51 mmol) Mangan(IV)-oxid versetzt und 2 Tage bei Raumtemperatur gerührt. Anschließend wird überschüssiges Mangan(IV)-oxid über Celite abfiltriert und das Lösungsmittel im Vakuum entfernt.

| | |
|---|---|
| Ausbeute: | 1,36 g (9,78 mmol, 96 %) |

### N-(3-Fluor-2-formyl-phenyl)-acetamid

1,37 g (9,85 mmol) 2-Amino-6-fluor-benzaldehyd werden mit 20 ml Essigsäureanhydrid versetzt und 4 h bei 70 °C erhitzt. Anschließend wird das Reaktionsgemisch in 200 ml dest Wasser eingerührt, mit Na₂CO₃ auf pH 7 eingestellt und dreimal mit je 50 ml Ethylacetat extrahiert. Danach wird die organische Phase mit MgSO₄ getrocknet, das Lösungsmittel im Vakuum entfernt. Das Rohprodukt wird durch Säulenchromatographie gereinigt. Als Trägermaterial dient Kieselgel und als Eluent wird ein Gemisch bestehend aus Cyclohexan : Ethylacetat (80:20) verwendet

| | |
|---|---|
| Ausbeute: | 1,40 g (7,73 mmol, 79 %) |

### N-[3-Fluor-2-(hydroxyl-o-tolyl-methyl)-phenyl]-acetamid

100 mg (0,83 mmol) *N*-(3-Fluor-2-formyl-phenyl)-acetamid werden in 4 ml Tetrahydrofuran gelöst und auf -78 °C abgekühlt. Anschließend werden bei dieser Temperatur 1,66 ml (3,3 mmol) einer 2 M Lösung von *o*-Tolylmagnesiumbromid in Diethylether zugegeben. Dieses Reaktionsgemisch wird über Nacht unter Rühren auf Raumtemperatur aufgetaut. Danach wird das Reaktionsgemisch in 30 ml dest. Wasser eingerührt und dreimal mit je 10 ml Ethylacetat extrahiert. Die organische Phase wird mit MgSO₄ getrocknet, das Lösungsmittel im Vakuum entfernt. Das Rohprodukt wird durch Säulenchromatographie gereinigt Als Trägermaterial dient Kieselgel und als Eluent wird ein Gemisch bestehend aus Cyclohexan: Ethylacetat (80:20) verwendet

| | |
|---|---|
| Ausbeute: | 205 mg (0,75 mmol, 90 %) |

### N-[3-Fluor-2-(2-methyl-benzoyl)-phenyl]-acetamid

205 mg (0,75 mmol) *N*-[3-Fluor-2-(hydroxyl-*o*-tolyl-methyl)-phenyl]-acetamid werden in 9 ml Chloroform gelöst, mit 652 mg (7,5 mmol) Mangan(IV)-oxid versetzt und 3 d bei Raumtemperatur gerührt. Anschließend wird überschüssiges Mangan(IV)-oxid über Celite abfiltriert, das Lösungsmittel im Vakuum entfernt Das Rohprodukt wird durch Säulenchromatographie gereinigt. Als Trägermaterial dient Kieselgel und als Eluent wird ein Gemisch bestehend aus Cyclohexan: Ethylacetat (80:20) verwendet.

| | |
|---|---|
| Ausbeute: | 142 mg (0,52 mmol, 70 %) |

### (2-Amino-6-fluor-phenyl)-o-tolyl-methanon

142 mg (0,52 mmol) *N*-[3-Fluor-2-(2-methyl-benzoyl)-phenyl]-acetamid werden in 2 ml Ethanol gelöst, mit 2 ml konz. Salzsäure versetzt und 4 h bei 70 °C gerührt. Anschließend wird das Reaktionsgemisch in 30 ml dest Wasser eingerührt, mit Natriumcarbonat auf pH 7 eingestellt und dreimal mit je 10 ml Ethylacetat extrahiert. Danach wird die organische Phase mit MgSO₄ getrocknet und das Lösungsmittel im Vakuum entfernt.

| | |
|---|---|
| Ausbeute: | 1,40 g (7,73 mmol, 79 %) |
| MS (ESI): | 230 (M+H)⁺ |

Analog zu dieser Methode werden folgende Verbindungen hergestellt.

| | **MS (ESI) (M+H)⁺** | | **MS (ESI) (M+H)⁺** |
|---|---|---|---|
| 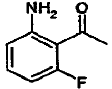 | 154 | 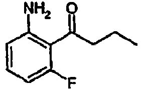 | 182 |
| 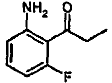 | 168 | | |

### Methode 15

### (2-Amino-phenyl)-[2-(piperidin-yl-1-carbonyl)-phenyl]-methanon

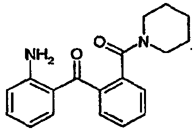

200 mg (0,81 mmol) 2-Aminobenzophenon-2-carbonsäure, 81 µl (0,81 mmol) Piperidin, 425 µl (2,43 mmol) *N*-Ethyldiisopropylamin werden in 1 ml Tetrahydrofuran gelöst. Diesem Reaktionsgemisch werden 265 mg (0,81 mmol) TBTU zugesetzt und über Nacht bei Raumtemperatur gerührt. Anschließend wird das Reaktionsgemisch in 20 ml dest Wasser eingerührt und dreimal mit je 5 ml Ethylacetat extrahiert. Danach wird die organische Phase über Alox B filtriert mit MgSO₄ getrocknet, das Lösungsmittel im Vakuum entfernt. Das Rohprodukt wird durch Säulenchromatographie gereinigt. Als Trägermaterial dient Kieselgel und als Eluent wird Dichlormethan, dem 2% einer Mischung aus 90% Methanol und 10% gesättigter wässriger Ammoniak-Lösung zugefügt sind, verwendet.

| | |
|---|---|
| Ausbeute: | 89 mg (0,29 mmol, 36 %) |
| MS (ESI): | 309 (M+H)⁺ |

### Methode 16

### 2-(1H-Imidazol-2-yl)-phenylamin:

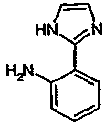

Durch katalytische Hydrierung, bei der Palladium auf Kohle oder Raney Nickel als Katalysator einsetzt wird, wird 2-(2-Nitro-phenyl)-1*H*-imidazol, welches kommerziell erhältlich ist, in 2-(1*H*-Imidazol-2-yl)-phenylamin überführt (US 6376664).

### Methode 17

### 2-Furan-2-yl-phenylamin

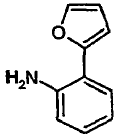

500 mg (2,43 mmol) 2-Bromnitrobenzol werden unter Schutzgasatmosphäre in 7,5 ml 1,4-Dioxan gelöst und mit 2,5 ml einer 2 M Natriumcarbonat-Lösung versetzt. Anschließend wird 560 mg (4,86 mmol) Furan-2-boronsäure und 283 mg (0,243 mmol) Tetrakis-(triphenylphosphin)-palladium(0) zugegeben. Die Reaktionsmischung wird 24 h unter Rückfluss Bedingungen gerührt. Die Reaktionsmischung wird auf Raumtemperatur abgekühlt und auf 100 ml Wasser gegeben. Diese Mischung wird dreimal mit 30 ml Essigester extrahiert, die vereinigten organischen Phasen werden mit Magnesiumsulfat getrocknet und das Lösungsmittel im Vakuum entfernt. Das Rohprodukt wird durch Säulenchromatographie gereinigt Als Trägermaterial dient Kieselgel und als Eluent wird ein Gemisch bestehend aus Cyclohexen: Ethylacetat (95:5) verwendet.

| | |
|---|---|
| Ausbeute: | 120 mg (0,63 mmol; 26 %) |
| MS(ESI): | 190 (M+H)⁺ |

113 mg (0,6 mmol) 2-(2-Nitro-phenyl)-furan werden in 2 ml Ethanol gelöst und mit 6 mg Palladium auf Aktivkohle (10% Pd) versetzt. Zu dieser Suspension gibt man 110 µl einer 35% wässrigen Hydrazin-Lösung und rührt die Reaktionsmischung unter Refluxbedingungen 20 h lang. Der Katalysator wird abgesaugt, das Filtrat wird mit 25 ml Wasser versetzt und auf pH 5 eingestellt. Diese Mischung wird dreimal mit je 10 ml Essigester extrahiert. Die vereinigten organischen Phasen werden getrocknet und das Lösungsmittel im Vakuum entfernt.

| | |
|---|---|
| Ausbeute: | 90 mg (0,57 mmol; 94 %) |
| MS (ESI): | 160 (M+H)⁺ |

Analog zu dieser Methode wird 2-Pyridin-2-yl-phenylamin hergestellt

### Methode 18

### 4-Morpholin-4-ylmethyl-cyclohexylamin

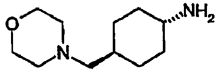

2,5 g (11,0 mmol) (4-Formyl-cyclohexyl)-carbaminsäure *tert*-butylester werden in 25 ml DMA gelöst mit 1 ml (11 mmol) Morpholin und 0,7 ml Essigsäure versetzt. Zu dieser Mischung gibt man 2,40 g (11,3 mmol) Natriumtriacetoxyborhydrid, welches in 12,5 ml DMA gelöst ist.
Die Reaktionsmischung wird 16 h bei Raumtemperatur gerührt. Anschließend gibt man die Reaktionsmischung auf 250 ml 10% Kaliumhydrogencarbonat Lösung und extrahiert diese Mischung dreimal mit je 100 ml Essigester. Die organischen Phasen werden vereinigt, getrocknet und anschließend wird das Lösungsmittel im Vakuum entfernt.
Der Rückstand wird in 20 ml Dichlormethan und 20 ml Trifluoressigsäue aufgenommen und eine 1 h bei Raumtemperatur gerührt. Die Lösungsmittel werden im Vakuum entfernt.

| | |
|---|---|
| Ausbeute: | 4,22 g (9,9 mmol; 90 %) (zweifaches Trifluoressigsäure Salz) |
| MS (ESI): | 199 (M+H)⁺ |

### Methode 19

### 4-Amino-N-(4-morpholin-4-yl-cyclohexyl)-3-prop-2-ynyloxy-benzamid

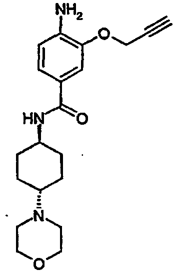

6,09 g (30 mmol) 3-Fluoro-4-nitro-benzoesäurechlorid werden in 36 ml Dichlormethan gelöst und auf 0 °C abgekühlt. Zu dieser Lösung gibt man bei 0 °C 5,52 g (30 mmol) 4-(4-Aminocyclohexyl)-morpholin, welches in 10 ml Dichlormethan gelöst und mit 4,65 g (45 mmol) Triethylamin versetzt ist Nach 16 h bei 0 °C wird die Reaktionsmischung auf Wasser gegossen. Der Niederschlag wird abgesaugt und getrocknet.

| | |
|---|---|
| Ausbeute: | 4,74 g (13,5 mmol; 45 %) |
| MS (ESI): | 352 (M+H)⁺ |

17,6 g (0,05 mol) 3-Fluor-*N*-(4-morpholin-4-yl-cyclohexyl)-4-nitro-benzamid und 9 g (0,16 mol) Propagylalkohol werden in 150 ml DMF vorgelegt und mit 10 g Kaliumcarbonat versetzt. Diese Reaktionsmischung wird 35 h bei 70 °C gerührt,
Die Hälfte des Lösungsmittels wird im Vakuum entfernt. Anschließend gibt man die Reaktionsmischung auf 10% Kaliumcarbonatlösung. Der entstehende Niederschlag wird abfiltriert und getrocknet

| | |
|---|---|
| Ausbeute: | 17,5 g (13,5 mmol; 90 %) |
| MS (ESI): | 388 (M+H)⁺ |

38,7 g (0,1 mol) *N*-(4-Morpholin-4-yl-cyclohexyl)4-nitro-3-prop-2-ynyloxy-benzamid werden in 400 ml Essigester und 25 ml Methanol gelöst. Zu dieser Lösung gibt man 75 g Zinn(II)chlorid_{*}3H₂O. Man lässt 16 h bei 50 °C rühren. Anschließend kühlt man auf Raumtemperatur ab und gibt 80 ml konz. wässrigen Ammoniak zu.
Der Niederschlag wird abgesaugt und verworfen. Das Filtrat wird im Vakuum eingeengt. Dieser Rückstand wird in Acetoniril umkristallisiert (1 g : 20 ml). Dabei fällt ein Nebenprodukt aus welches ebenfalls verworfen wird. Das Filtrat wird mit 30 ml 1-Chlorbutan versetzt und auf 10 °C abgekühlt. Der entstehende Niederschlag wird abfiltriert und getrocknet

| | |
|---|---|
| Ausbeute: | 15,4 g (0,04 mmol; 43 %) |
| MS (ESI): | 358 (M+H)⁺ |

Analog zu dieser Methode werden folgende Verbindungen hergestellt. Die Reduktion der Nitrogruppe kann gegebenenfalls auch mit Pd/C oder Raney-Nickel erfolgen.

| | **MS (ESI) (M+H)⁺** | | **MS (ESI) (M+H)⁺** |
|---|---|---|---|
| 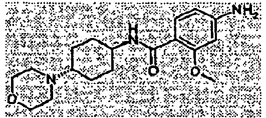 | 334 | 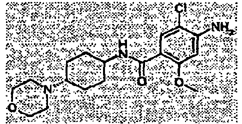 | 368/370 |
| 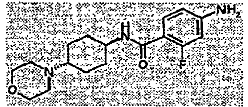 | 322 | 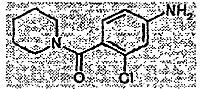 | 239/241 |

### Methode 20

### 4-(2-Amino-benzoyl)-benzonitril

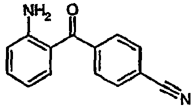

3,43 ml (5,50 mmol) einer 1,6 M Lösung von n-Butyllithium in Hexan werden mit 10 ml trockenem THF versetzt und auf -80 °C gekühlt. Eine Lösung von 1 g (5,94 mmol) 4-Brombenzonitril in 10 ml THF werden in einem Zeitraum von 30 min zu der ButyllithiumLösung getropft. Nachdem die Reaktionsmischung weitere 30 min bei -80 °C gerührt wird, gibt man tropfenweise 0,42 g (2,75 mmol) 2-Nitrobenzaldehyd gelöst in 10 ml THF zu. Nach der Zugabe rührt man weitere 2 h bei -80 °C. Anschließend gibt man gesättigte Ammoniumchlorid-Lösung zu. Die organische Phase wird abgetrennt mit Wasser gewaschen und getrocknet Das Lösungsmittel wird im Vakuum entfernt Das Rohprodukt wird durch Säulenchromatographie gereinigt. Als Trägermaterial dient Kieselgel und als Eluent wird ein Gemisch bestehend aus Cyclohexan : Ethylacetat (2:1) verwendet.

| | |
|---|---|
| Ausbeute: | 0,59 (2,32 mmol; 42 %) |
| MS (ESI): | 255 (M+H)⁺ |

Die weiteren Synthesestufen, welche zu 4-(2-Amino-benzoyl)-benzonitril führen, können analog zu Methode 9 durchgeführt werden.

Analog zu dieser Methode werden folgende Verbindungen hergestellt:

| | **MS (ESI) (M+H)⁺** | | **MS (ESI) (M+H)⁺** |
|---|---|---|---|
| 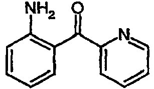 | 199 | 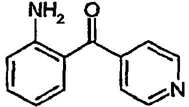 | 199 |
| 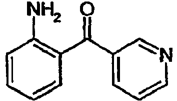 | 199 | 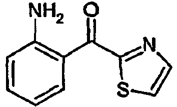 | 205 |

### Biologische Eigenschaften

Wie durch DNA-Färbung mit darauf folgender FACS Analyse gezeigt werden konnte, ist die, durch die erfindungsgemäßen Verbindungen bewirkte, Proliferationsinhibition vor allem durch einen Arrest der Zellen in der G2/M Phase des Zellzyklus vermittelt. Die Zellen arretieren abhängig von dem verwendeten Zelltyp für eine bestimmte Zeitspanne in dieser Zellzyklus Phase, bevor der programmierte Zelltod eingeleitet wird. Ein Arrest in der G2/M Phase des Zellzyklus kann z.B. durch die Inhibition spezifischer Zellzykluskinasen ausgelöst werden. Auf Grund ihrer biologischen Eigenschaften eignen sich die erfindungsgemäßen Verbindungen der allgemeinen Formel I, deren Isomere und deren physiologisch verträgliche Salze zur Behandlung von Erkrankungen, die durch exzessive oder anomale Zellproliferation charakterisiert sind.

Zu solchen Erkrankungen gehören beispielsweise: Virale Infektionen (z.B. HIV und Kaposi Sarkoma); Entzündung und Autoimmun-Erkrankungen (z.B. Colitis, Arthritis, Alzheimer Erkrankung, Glomerulonephritis und Wund-Heilung); bakterielle, fungale und/oder parasitäre Infektionen; Leukämien, Lymphome und solide Tumore; Haut-Erkrankungen (z.B. Psoriasis); Knochen-Erkrankungen; kardiovaskuläre Erkrankungen (z.B. Restenose und Hypertrophie). Ferner sind sie nützlich als Schutz von proliferierenden Zellen (z.B. Haar-, Intestinal-, Blut- und Progenitor-Zellen) gegen DNA-Schädigung durch Strahlung, UV-Behandlung und/oder zytostatischer Behandlung (Davis et al., 2001).

Beispielsweise können folgende Krebserkrankungen mit erfindungsgemäßen Verbindungen behandelt werden, ohne jedoch darauf beschränkt zu sein: Hirntumore wie beispielsweise Akustikusneurinom, Astrozytome wie pilozytisches Astrozytome, fibrilläres Astrozytom, protoplasmatisches Astrozytom, gemistozytäres Astrozytom, anaplastisches Astrozytom und Glioblastome, Hirnlymphome, Hirnmetastasen, Hypophysentumor wie Prolaktinom, HGH (human growth hormon) produzierender Tumor und ACTH produzierender Tumor (adrenocorticotropes Hormon), Kraniopharyngiome, Medulloblastome, Meningeome und Oligodendrogliome; Nerventumore (Neoplasmen) wie zum Beispiel Tumore des vegetativen Nervensystems wie Neuroblastoma sympathicum, Ganglioneurom, Paragangliom (Phäochromozytom, Chromaffinom) und Glomus-caroticum-Tumor, Tumore am peripheren Nervensystem wie Amputationsneurom, Neurofibrom, Neurinom (Neurilemmom, Schwannom) und malignes Schwannom, sowie Tumore am zentralen Nervensystem als Hirn- und Rückenmarkstumoren;Darmkrebs wie zum Beispiel Rektumkarzinom, Kolonkarzinom, Analkarzinom, Dünndarmtumore und Zwölffingerdarmtumore; Lidtumore wie Basaliom oder Basalzellkarzinom; Bauchspeicheldrüsenkrebs oder Pankreaskarzinom; Blasenkrebs oder Blasenkarzinom; Lungenkrebs (Bronchialkarzinom) wie beispielsweise kleinzellige Bronchialkarzinome (Haferzellkarzinome) und nicht-kleinzellige Bronchialkarzinome wie Plattenepithelkarzinome, Adenokarzinome und großzellige Bronchialkarzinome; Brustkrebs wie zum Beispiel Mammakarzinom wie infiltrierend duktales Karzinom, Kolloidkarzinom, lobulär invasives Karzinom, tubuläres Karzinom, adenozystisches Karzinom und papilläres Karzinom; Non-Hodgkin-Lymphomen (NHL) wie beispielsweise Burkitt-Lymphom, niedrigmaligne Non-Hodgkin-Lymphomen (NHL) und Mucosis fungoides; Gebärmutterkrebs oder Endometriumkarzinom bzw. Corpuskarzinom; CUP-Syndrom (Cancer of Unknown Primary); Eierstockskrebs oder Ovarial-Karzinom wie mucinöse, endometriale oder seröse Krebs; Gallenblasenkrebs; Gallengangskrebs wie beispielsweise Klatskin-Tumor; Hodenkrebs wie zum Beispiel Seminome und Nicht-Seminome;Lymphom (Lymphosarkom) wie zum Beispiel malignes Lymphom, Morbus Hodgkin, Non-Hodgkin-Lymphomen (NHL) wie chronisch lymphatische Leukämie, Haarzell-Leukämie, Immunozytom, Plasmozytom (multiples Myelom), Immunoblastom, Burkitt-Lymphom, T-Zonen-Mycosis fungoides, großzellig-anaplastisches Lymphoblastom und Lymphoblastom; Kehlkopfkrebs wie zum Beispiel Stimmbandtumoren, supraglottisch, glottisch und subglottisch Kehlkopflumore; Knochenkrebs wie zum Beispiel Osteochondrom, Chondrom, Chondroblastom, Chondromyxoidfibrom, Osteom, Osteoid-Osteom, Osteoblastom, eosinophiles Granulom, Riesenzell-Tumor, Chondrosarkom, Osteosarkom, Ewing-Sarkom, Retikulo-Sarkom, Plasmozytom, Riesenzell-Tumor, fibröse Dysplasie, juvenile Knochenzyste und aneurysmatische Knochenzyste; Kopf-Hals-Tumoren wie zum Beispiel Tumoren der Lippen, Zunge, Mundboden, Mundhöhle Zahlfleisch, Gaumen, Speicheldrüsen, Rachen, Nasenhöhlen, Nasennebenhöhlen, Kehlkopf und Mittelohr;Leberkrebs wie zum Beispiel das Leberzellkarzinom oder hepatozelluläres Karzinom (HCC); Leukämien wie zum Beispiel akute Leukämien wie akute lymphatische/lymphoblastische Leukämie (ALL), akute myeloische Leukämie (AML); chronische Leukämien wie chronisch lymphatische Leukämie (CLL), chronisch myeloische Leukämie (CML); Magenkrebs oder Magenkarzinom wie zum Beispiel papilläres, tubuläres und muzinöses Adenokarzinom, Siegelringzellkarzinom adenosquamöses Karzinom, kleinzelliges Karzinom und undifferenziertes Karzinom; Melanome wie zum Beispiel oberflächlich spreitendes, knotiges, lenitgo-maligna und akral-lentiginöse Melanom; Nierenkrebs wie zum Beispiel Nierenzellkarzinom oder Hypernephrom oder Grawitz-Tumor; Speiseröhrenkrebs oder Ösophaguskarzinom; Peniskrebs; Prostatakrebs; Rachenkrebs oder Pharynxkarzinome wie zum Beispiel Nasopharynxkarzinome, Oropharynxkarzinome und Hypopharynxkarzinome; Retinoblastom wie zum Beispiel Scheidenkrebs oder Vaginalkarzinom; Plattenepithelkarzinome, Adenokarzinome, in situ Karzinome, maligne Melanome und Sarkome; Schilddrüsen-Karzinome wie zum Beispiel papilläre, follikuläre und medulläre Schilddrüsen-Karzinom, sowie anaplastische Karzinome; Spinaliom, Stachelzellkarzinom und Plattenepithelkarzinom der Haut; Thymome, Urethrakrebs und Vulvakrebs.

Die neuen Verbindungen können zur Prävention, Kurz- oder Langzeitbehandlung der vorstehend erwähnten Krankheiten auch gegebenenfalls in Kombination mit anderen "State-of-art" Verbindungen, wie anderen anti-Tumor Substanzen, zytotoxische Substanzen, Zellproliferationshemmer, anti-angiogene Substanzen, Steroide oder Antikörper, verwendet werden.

Die Verbindungen der allgemeinen Formel (1) können allein oder in Kombination mit anderen erfindungsgemäßen Wirkstoffen, gegebenenfalls auch in Kombination mit weiteren pharmakologisch aktiven Wirkstoffen, zur Anwendung gelangen.

Chemotherapeutica welche in Kombination mit den erfindungsgemäßen Verbindungen verabreicht werden können, umfassen ohne darauf eingeschränkt zu sein, Hormone, Hormonanaloge und Antihormone (z.B. Tamoxifen, Toremifene, Raloxifene, Fulvestrant, Megestrol Acetat, Flutamide, Nilutamide, Bicalutamid, Aminoglutethimid, Cyproterone Acetat, Finasteride, Buserelin Acetat, Fludrocortinsone, Fluoxymesterone, Medroxyprogesterone, Octreotide), Aromatase Inhibitoren (z.B. Anastrozole, Letrozole, Liarozole, Vorozole, Exemestane, Atamestane,), LHRH Agonisten und Antagonists (z.B. Goserelin Acetat, Luprolide), Inhibitoren von Wachstumsfaktoren (growth factors wie zum Beispiel "platelet derived growth factor" und "hepatocyte growth factor", Inhibitoren sind z. B. "growth factor"-Antikörper, "growth factor receptor"-Antikörper und Tyrosinekinase Inhibitoren, wie zum Beispiel Gefitinib, Imatinib, Lapatinib und Trastuzumab); Antimetabolite (z.B. Antifolate wie Methotrexate, Raltitrexed, Pyrimidinanaloge wie 5-Fluorouracil, Capecitabin und Gemcitabin, Purin- und Adenosinanaloge wie Mercaptopurine, Thioguanine, Cladribine und Pentostatin, Cytarabine, Fludarabine); Antitumor-Antibiotica (z.B. Anthracycline wie Doxorubicin, Daunorubicin, Epirubicin und Idarubicin, Mitomycin-C, Bleomycin, Dactinomycin, Plicamycin, Streptozocin); Platinderivative (z.B. Cisplatin, Oxaliplatin, Carboplatin); Alkylierungsagentien (z.B. Estramustin, Meclorethamin, Melphalan, Chlorambucil, Busulphan, Dacarbazin, Cyclophosphamid, Ifosfamid, Temozolomid, Nitrosoharnstoffe wie zum Beispiel Carmustin und Lomustin, Thiotepa); antimitotische Agentien (z.B. Vinca-Alkaloide wie zum Beispiel Vinblastin, Vindesin, Vinorelbin und Vincristin; und Taxane wie Paclitaxel, Docetaxel); Topoisomerase Inhibitoren (z.B. Epipodophyllotoxine wie zum Beispiel Etoposid und Etopophos, Teniposid, Amsacrin, Topotecan, Irinotecan, Mitoxantron) und verschiedene Chemotherapeutica wie Amifostin, Anagrelid, Clodronat, Filgrastin, Interferon alpha, Leucovorin, Rituximab, Procarbazine, Levamisole, Mesna, Mitotane, Pamidronate und Porfimer.

### PLK-1 Kinaseassay

Rekombinantes humanes und an seinem N-terminalen Ende mit GST verbundenes PLK1 Enzym wird aus Bakulovirus infizierten Insektenzellen (Sf21) isoliert Die Reinigung erfolgt durch Affinitätschromatographie an Glutathion Sepharose Säulen.

4x10⁷ St21 Zellen (Spodoptera frugiperda) in 200 ml Sf-900 II Serum freien Insektenzellmedium (Life Technologies) werden in eine Spinnerflasche ausgesät. Nach 72 Stunden Inkubation bei 27°C und 70 rpm werden 1x10⁸ Sf21 Zellen in insgesamt 180 ml Medium in eine neue Spinnerflasche ausgesät Nach weiteren 24 Stunden werden 20 ml rekombinanter Baculovirus Stammsuspension zugesetzt und die Zellen 72 Stunden bei 27°C bei 70 rpm kultiviert. 3 Stunden vor dem Ernten wird Okadainsäure zugesetzt (Calbiochem, Endkonzentration 0,1 µM) und die Suspension weiter inkubiert. Die Zellzahl wird bestimmt, die Zellen abzentrifugiert (5 Minuten, 4°C, 800 rpm) und 1x mit PBS (8 g NaCl/1, 0,2 g KCl/1, 1,44 g Na₂HPO₄/l, 0,24 g KR₂PO4/l) gewaschen. Nach nochmaligem Abzentrifugieren wird das Pellet in flüssigem Stickstoff Schock gefroren. Danach wird das Pellet rasch aufgetaut und in eiskaltem Lysispuffer (50 mM HEPES pH 7,5, 10 mM MgCl₂, 1 mM DTT, 5 µg/ml Leupeptin, 5 µg/ml Aprotinin, 100 µM NaF, 100 µM PMSF, 10 mM ß-Glycerolphosphat, 0.1 mM Na₃VO₄, 30 mM 4-Nitrophenylphosphate) zu 1x10⁸ Zellen/17,5 ml resuspendiert. Die Zellen werden 30 Minuten auf Eis lysiert. Nach dem Entfernen der Zelltrümmer durch Zentrifugation (4000 rpm, 5 Minuten) wird der klare Überstand mit Glutathion Sepharosebeads versetzt (1 ml resuspendierte und gewaschene Beads für 50 ml Überstand) und 30 Minuten bei 4°C auf einem Rotationsbrett inkubiert. Danach werden die Beads mit Lysispuffer gewaschen und das rekombinante Protein mit 1 ml Elutionspuffer/ ml resuspendierte Beads (Elutionspuffer: 100 mM Tris/HCl pH=8,0, 120 mM NaCl, 20 mM reduziertes Glutathion (Sigma G-4251), 10 mM MgCl₂, 1 mM DTT) von den Beads eluiert. Die Proteinkonzentration wird mittels Bradford Assay bestimmt

### Assay

In einem Napf einer 96-Loch Rundbodenplatte (Fa. Greiner bio-one, PS-Microtiterplatte Nr.650101) werden folgende Komponenten zusammengefügt:
- 10 µl zu testende Verbindung in variabler Konzentration (z.B. beginnend bei 300 µM, und Verdünnung in 1:3) in 6% DMSO, 0,5 mg/ml Casein (Sigma C-5890), 60 ß-Glycerophosphat, 25 mM MOPS pH=7,0, 5 mM EGTA, 15 mM MgCl₂, 1 mM DTT
- 20 µl Substratlösung (25 mM MOPS pH=7,0, 15 mM MgCl₂, 1 mM DTT, 2,5 mM EGTA, 30 mM ß-Glycerophosphat, 0,25 mg/ml Casein)
- 20 µl Enzymverdünnung (1:100 Verdünnung des Enzymstocks in 25 mM MOPS pH=7,0, 15 mM MgCl₂, 1 mM DTT)
- 10 µl ATP Lösung (45 µM ATP mit 1,11x10⁶ Bq/ml gamma-P33-ATP).

Durch Zusatz der ATP Lösung wird die Reaktion gestartet und 45 Minuten bei 30 °C unter leichtem Schütteln (650 rpm auf IKA Schüttler MTS2) durchgeführt. Die Reaktion wird durch Zusatz von 125 µl eiskalter 5%iger TCA pro Napf gestoppt und mindestens 30 Minuten auf Eis inkubiert. Das Präziptitat wird durch Ernten auf Filterplatten (96-well-Microtiter-Filterplatte: UniFilter-96, GF/B; Fa. Packard; Nr.6005177) übertragen, dann viermal mit 1%iger TCA gewaschen und bei 60°C getrocknet. Nach Zugabe von 35 µl Szintillationslösung (Ready-Safe; Beckmann) pro Napf wird die Platte mit Sealing-tape zugeklebt und die präzipitierte Menge P33 mit dem Wallac Betacounter gemessen.

Die Messdaten werden mit der Standard Graphpad Software (Levenburg-Marquard Algorhythmus) ausgewertet

Die Wirkung der erfindungsgemäßen Verbindungen wird im Cytotoxizitätstest an kultivierten humanen Tumorzellen und/oder in einer FACS-Analyse, beispielsweise an HeLa S3-Zellen, bestimmt. Die Verbindungen zeigen in beiden Testmethoden eine gute bis sehr gute Wirksamkeit, d.h. beispielsweise einen EC50-Wert im HeLa S3-Cytotoxizitätstest kleiner 5 µmol/L, in der Regel kleiner 1 µmol/L.

### Messung der Cytotoxizität an kultivierten humanen Tumorzellen

Zur Messung der Cytotoxizität an kultivierten humanen Tumorzellen werden
Zellen der zervikalen Carcinoma Tumorzell-Linie HeLa S3 (erhalten von American Type Culture Collection (ATCC)) in Ham's F12 Medium (Life Technologies) und 10% fötalem Rinderserum (Life Technologies) kultiviert und in der log-Wachstumsphase geerntet. Anschließend werden die HeLa S3 Zellen in 96-well Platten (Costar) mit einer Dichte von 1000 Zellen pro well eingebracht und über Nacht in einem Inkubator (bei 37°C und 5 % CO2) inkubiert, wobei auf jeder Platte 6 wells nur mit Medium gefüllt werden (3 wells zur Mediumkontrolle, 3 wells zur Inkubation mit reduzierten AlamarBlue Reagenz). Die Wirksubstanzen werden in verschiedenen Konzentrationen (gelöst in DMSO; DMSO-Endkonzentration: 0.1%) zu den Zellen zugegeben (jeweils als Dreifachbestimmung). Nach 72 Stunden Inkubation werden zu jedem well 20 µl AlamarBlue Reagenz (AccuMed International) zugesetzt, und die Zellen für weitere 5-7 Stunden inkubiert. Zur Kontrolle wird zu 3 wells je 20 µl reduziertes AlamarBlue Reagenz gegeben (AlamarBlue Reagenz, das für 30 min autoklaviert wird). Nach Inkubation wird der Farbumsatz des AlamarBlue Reagenz in den einzelnen wells in einem Perkin Elmer Fluoreszenzspektrophotometer bestimmt (Exitation 530 nm, Emission 590 nm, Slits 15, Integrate time 0.1). Die Menge an umgesetzten AlamarBlue Reagenz repräsentiert die metabolische Aktivität der Zellen. Die relative Zellaktivität wird in Prozent der Kontrolle (HeLa S3 Zellen ohne Inhibitor) berechnet und die Wirkstoffkonzentration, die die Zellaktivität zu 50% hemmt (IC50) abgeleitet. Die Werte werden hierbei aus dem Mittelwert von drei Einzelbestimmungen-unter Korrektur des Leerwertes (Mediumkontrolle)- berechnet.

### FACS- Analyse

Propidium Iodid (PI) bindet stöchiometrisch an doppelsträngige DNA, und ist damit geeignet den Anteil an Zellen in der G1 S, und G2/M Phase des Zellzykluses auf der Basis des zellulären DNA Gehaltes zu bestimmen. Zellen in der G0 und G1 Phase haben einen diploiden DNA Gehalt (2N), während Zellen in der G2 oder Mitose einen 4N DNA Gehalt haben.
Für eine PI-Färbung werden beispielsweise 1x10⁶ HeLa S3 Zellen auf eine 75 cm2 Zellkulturflasche ausgesät, nach 24 h wird entweder 0.1 % DMSO als Kontrolle zugesetzt, bzw. die Substanz in verschiedenen Konzentrationen (in 0.1% DMSO). Die Zellen werden für 24 h mit der Substanz bzw. mit DMSO inkubiert, bevor die Zellen 2 x mit PBS gewaschen und dann mit Trypsin /EDTA abgelöst werden. Die Zellen werden zentrifugiert (1000 Upm, 5 min, 4°C), und das Zellpellet 2 x mit PBS gewaschen, bevor die Zellen in 0.1 ml PBS resuspendiert werden. Anschließend werden die Zellen für 16 Stunden bei 4°C oder alternativ für 2 Stunden bei -20°C mit 80% Ethanol fixiert. Die fixierten Zellen werden zentrifugiert (1000 Upm, 5min, 4°C), mit PBS gewaschen und anschließend nochmals zentrifugiert. Das Zellpellet wird in 2 ml 0.25% Triton X-100 in PBS resuspendiert, und 5 min auf Eis inkubiert, bevor 5 ml PBS zugeben werden und erneut zentrifugiert wird. Das Zellpellet wird in 350 µl PI Färbelösung (0.1 mg/ml RNase A (Sigma, No. R-4875), 10 µg/ml Prodium Iodid (Sigma, No. P-4864) in 1 x PBS) resuspendiert. Die Zellen werden für 20 min im Dunkeln mit dem Färbepuffer inkubiert, bevor sie in Probenmessgefäße für das FACS Scan überführt werden. Die DNA Messung erfolgte in einem Becton Dickinson FACS Analyzer, mit einem Argonlaser (500 mW, Emission 488 nm), und dem DNA Cell Quest Programm (BD). Die logarithmische PI Fluoreszenz wird mit einem band-pass Filter (BP 585/42) bestimmt. Die Quantifizierung der Zellpopulationen in den einzelnen Zellzyklusphasen erfolgte mit dem ModFit LT Programm von Becton Dickinson.

Entsprechend werden die erfindungsgemäßen Verbindungen auch auf weiteren Tumorzellen getestet. Beispielsweise sind diese Verbindungen auf Karzinomen verschiedenster Gewebe (z. Bsp. Brust (MCF7); Colon (HCT116), Kopf-Hals (FaDu), Leber (HepG2), Lunge (NCI-H460), Magen (NCI-N87), Pankreas (BxPC-3), Prostata (DU145)), Sarkome (z. Bsp. SK-UT-1B, Saos-2), Leukämien und Lymphome (z. Bsp. HL-60, THP-1, Raji, Jurkat, GRANTA-519) und anderen Tumoren (z. Bsp. Melanome (BRO), Gliome (U-87MG)) aktiv und könnten in solchen Indikationen eingesetzt werden. Dies belegt die breite Anwendbarkeit der erfindungsgemäßen Verbindungen zur Behandlung verschiedenster Tumortypen.
Die Verbindungen der allgemeinen Formel (I) können allein oder in Kombination mit anderen erfindungsgemäßen Wirkstoffen, gegebenenfalls auch in Kombination mit weiteren pharmakologisch aktiven Wirkstoffen, zur Anwendung gelangen.

Geeignete Anwendungsformen sind beispielsweise Tabletten, Kapseln, Zäpfchen, Lösungen, - insbesondere Lösungen zur Injektion (s.c., i.v., i.m.) und Infusion - Säfte, Emulsionen oder dispersible Pulver. Hierbei soll der Anteil der pharmazeutisch wirksamen Verbindung(en) jeweils im Bereich von 0,1 - 90 Gew.%, bevorzugt 0,5 - 50 Gew.-% der Gesamtzusammensetzung liegen, d.h. in Mengen die ausreichend sind, um den unten angegebenen Dosierungsbereich zu erreichen. Die genannten Dosen können, falls erforderlich, mehrmals täglich gegeben werden.
Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektions- und Infusionslösungen werden in üblicher Weise, z.B. unter Zusatz von Isotonantien, Konservierungsmitteln, wie p-Hydroxybenzoate, oder Stabilisatoren, wie Alkalisalzen der Ethylendiamintetraessigsäure, gegebenenfalls unter Verwendung von Emulgiermitteln und /oder Dispergiermitteln, wobei beispielsweise bei der Verwendung von Wasser als Verdünnungsmittel gegebenenfalls organische Lösemittel als Lösevermittler bzw. Hilfslösemittel eingesetzt werden können, hergestellt und in Injektionsflaschen oder Ampullen oder Infusionsflaschen abgefüllt.

Die eine oder mehrere Wirkstoffe beziehungsweise Wirkstoffkombinationen enthaltenden Kapseln können beispielsweise hergestellt werden indem man die Wirkstoffe mit inerten Trägern, wie Milchzucker oder Sorbit, mischt und in Gelatinekapseln einkapselt. Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.
Als Hilfsstoffe seien beispielsweise Wasser, pharmazeutisch unbedenkliche organische Lösemittel, wie Paraffine (z.B. Erdölfraktionen), Öle pflanzlichen Ursprungs (z.B. Erdnuss- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzucker) Emulgiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Die Applikation erfolgt in üblicher Weise, vorzugsweise oral oder transdermal, insbesondere bevorzugt oral. Im Falle der oralen Anwendung können die Tabletten selbstverständlich außer den genannten Trägerstoffen auch Zusätze, wie z.B. Natriumcitrat, Calciumcarbonat und Dikalziumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wässriger Suspensionen können die Wirkstoffe außer den oben genannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.
Die Dosierung für die intravenöse Anwendung liegt bei 1 - 1000 mg pro Stunde, vorzugsweise zwischen 5 - 500 mg pro Stunde.

Trotzdem kann es gegebenenfalls notwendig sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Formulierungsbeispiele illustrieren die vorliegende Erfindung ohne sie jedoch in ihrem Umfang zu beschränken:

### Pharmazeutische Formulierungsbeispiele

| A) | Tabletten | pro Tablette |
|---|---|---|
| | Wirkstoff | 100 mg |
| | Milchzucker | 140 mg |
| | Maisstärke | 240 mg |
| | Polyvinylpyrrolidon | 15 mg |
| | Magnesiumstearat | 5 mg |
| | | 500 mg |

Der fein gemahlene Wirkstoff, Milchzucker und ein Teil der Maisstärke werden miteinander vermischt. Die Mischung wird gesiebt, danach mit einer Lösung von Polyvinylpyrrolidon in Wasser befeuchtet, geknetet, feucht granuliert und getrocknet Das Granulat, der Rest der Maisstärke und das Magnesiumstearat werden gesiebt und miteinander vermischt. Das Gemisch wird zu Tabletten geeigneter Form und Größe verpresst.

| B) | Tabletten | pro Tablette |
|---|---|---|
| | Wirkstoff | 80 mg |
| | Milchzucker | 55 mg |
| | Maisstärke | 190 mg |
| | Mikrokristalline Cellulose | 35 mg |
| | Polyvinylpyrrolidon | 15 mg |
| | Natrium-carboxymethylstärke | 23 mg |
| | Magnesiumstearat | 2 mg |
| | | 400 mg |

Der fein gemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natriumcarboxymethylstärke und das Magnesiumstearat, vermischt und verpresst das Gemisch zu Tabletten geeigneter Größe.

| C) | Ampullenlösung | |
|---|---|---|
| | Wirkstoff | 50 mg |
| | Natriumchlorid | 50 mg |
| | Aqua pro inj. | 5 ml |

Der Wirkstoff wird bei Eigen-pH oder gegebenenfalls bei pH 5,5 - 6,5 in Wasser gelöst und mit Natriumchlorid als Isotonans versetzt. Die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.

## Patentansprüche

1. Verbindungen der allgemeinen Formel (1) 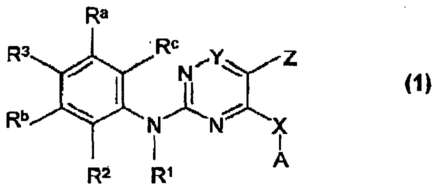 worin
**X** -NR^{1a} O oder S, und
**Y** CH und
**Z** C₁₋₃-Fluorakyl- und
**A** ausgewählt ist aus den Formeln (i) oder (ii) 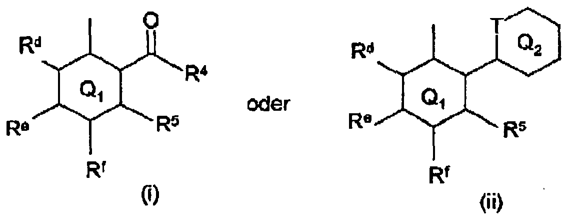 und
**Q₁** mono- oder bizyklische Arylverbindungen; und
**Q₂** mono- oder bizyklische Heteroarylverbindungen; und
**T** N, O oder S, und
**R¹ und R^{1a}** Wasserstoff oder Methyl, und
**R²** ein Rest ausgewählt aus der Gruppe bestehend aus -Cl, -Br, -I, -OR⁶, -C(=O)R⁶, -C(=O)NR⁶R⁷, -NR⁶R⁷, -NR⁶C(=O)R⁷, -NR⁶SO₂R⁷, -N=CR⁶R⁷, -SR⁶, -SOR⁶, -SO₂R⁶, -SO₂NR⁶R⁷ und Pseudohalogen, oder ein gegebenenfalls einfach oder mehrfach substituierter Rest ausgewählt aus der Gruppe bestehend aus C₁₋₆-Alkyl, C₂₋₆-Alkenyl, C₂₋₆-Alkinyl C₃₋₆-Cycloalkyl, Aryl , Heterocyclyl und Heteroaryl, wobei der/die Substituenten gleich oder verschieden sein können und ausgewählt werden aus der Gruppe bestehend aus Halogen, -NO₂, -OR⁶, -C(=O)R⁶, -C(=O)OR⁶, -C(=O)NR⁶R⁷, -NR⁶R⁷, -NR⁶C(=O)R⁷, -NR⁶C(=O)OR⁷, -NR⁶C(=O)NR⁷R⁸, -NR⁶SO₂R⁷, -N=CR⁶R⁷, -SR⁶, -SOR⁶, -SO₂R⁶, -SO₂NR⁶R⁷, -NR⁶SO₂NR⁷R⁸, -OS₂NR⁷R⁸ und Pseudohalogen; und
**R^{a}, R^{b}**, **R^{c}**, **R^{d}, R^{e}** und **R^{f}** jeweils unabhängig voneinander ein Rest ausgewählt aus der Gruppe bestehend aus Wasserstoff Halogen, -NO₂, -OR⁶, -C(=O)R⁶, -C(=O)OR⁶, -C(=O)NR⁶R⁷, -NR⁶R⁷, -NR⁶C(=O)R⁷, -NR⁶C(=O)OR⁷, -NR⁶C(=O)NR⁷R⁸, -NR⁶SO₂R⁷, -N=CR⁶R⁷, -SR⁶, -SOR⁶, -SO₂R⁶, -SO₂NR⁶R⁷, -NR⁶SO₂NR⁷R⁸, -OSO₂NR⁷R⁸ und Pseudohalogen;
oder ein gegebenenfalls einfach oder mehrfach substituierter Rest ausgewählt aus der Gruppe bestehend aus C₁₋₆-Alkyl, C₂₋₆-Alkenyl, C₂₋₆-Alkinyl, C₃₋₆-Cycloalkyl, Aryl, Heterocyclyl und Heteroaryl, wobei der/die Substituenten gleich oder verschieden sein können und ausgewählt werden aus der Gruppe bestehend aus Wasserstoff Halogen, -NO₂, -OR⁶, -C(=O)R⁶, -C(=O)OR⁶, -C(=O)NR⁶R⁷, -NR⁶R⁷, -NR⁶C(=O)R⁷, -NR⁶C(=O)OR⁷, -NR⁶C(=O)NR⁷R⁸, -NR⁶SO₂R7, -N=CR⁶R⁷, -SR⁶, -SOR⁶, -SO₂R⁶, -SO₂NR⁶R⁷, -NR⁶SO₂NR⁷R⁸, -OSO₂NR⁷R⁸ und Pseudohalogen; und
**R³** ausgewählt aus den Formeln **(iii) - (ix)**, 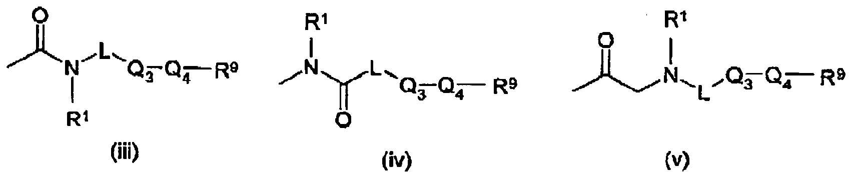 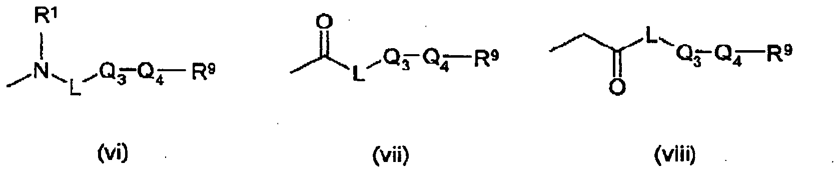 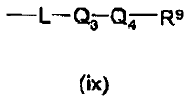 und
R⁴ ein Rest ausgewählt aus der Gruppe bestehend aus Wasserstoff, -OR⁶, -C(=O)R⁶, -C(=O)OR⁶, -C(=O)NR⁶R⁷, -NR⁶R⁷, -NR⁶C(=O)R⁷, -NR⁶C(=O)OR⁷, -NR⁶C(=O)NR⁷R⁸, -NR⁶SO₂R⁷, -N=CR⁶R⁷, -SR⁶, -SOR⁶, -SO₂R⁶, -SO₂NR⁶R⁷, -NR⁶SO₂NR⁷R⁸, -OSO₂NR⁷R⁸ und Pseudohalogen,
oder ein Rest ausgewählt aus der Gruppe bestehend aus gegebenenfalls einfach oder mehrfach substituiertem C₁₋₈-Alkyl, C₂₋₁₀-Alkenyl, C₂₋₁₀-Akinyl, C₃₋₈-Cycloalkyl-Aryl, Heterocyclyl und Heteroaryl, wobei der/die Substituent(en) gleich oder verschieden sein können und ausgewählt werden aus der Gruppe bestehend aus C₁₋₈-Alkyl, Halogen, -NO₂, -OR⁶, -C(=O)R⁶, -C(=O)OR⁶, -C(=O)NR⁶R⁷, -NR⁶R⁷, -NR⁶C(=O)R⁷, -NR⁶C(=O)OR⁷, NR⁶C(=O)NR⁷R⁸, -NR⁶SO₂R⁷, -N=CR⁶R⁷, -SR⁶, -SOR⁶, -SO₂R⁶, -SO₂NR⁶R⁷, -NR⁶SO₂NR⁷R⁸, -OS₂NR⁷R⁸ und Pseudohalogen; und
**R⁵** Wasserstoff, Halogen, -CF₃, C₁₋₃-Alkyl oder -OR⁶; und
**R⁶, R⁷** und **R⁸** jeweils unabhängig voneinander Wasserstoff oder ein Rest ausgewählt aus der Gruppe bestehend aus gegebenenfalls einfach oder mehrfach substituiertem C₁₋₅-Alkyl, C₂₋₃-Alkenyl, C₂₋₅-Alkinyl, C₃₋₁₀-Cycloalkyl, Aryl, Heterocyclyl und Heteroaryl, wobei der/die Substituenten gleich oder verschieden sein können und ausgewählt werden aus der Gruppe bestehend aus C₃₋₁₀-Cycloalkyl, Aryl, Heterocyclyl, Heteroaryl, Halogen, -NO₂, -OR¹⁰, -C(=O)R¹⁰, -C(=O)OR¹⁰, -O(=O)NR¹⁰R¹¹, -NR¹⁰R¹¹, -NR¹⁰C(=O)R¹¹, -NR¹⁰C(=O)OR¹¹, -NR¹⁰C(=O)NR¹¹R¹², -NR¹⁰C(=O)ONR¹¹R¹², -NR¹⁰SO₂R¹¹, -N=CR¹⁰R¹¹, -SR¹⁰, -SOR¹⁰, -SO₂R¹⁰, -SO₂NR¹⁰R¹¹, -NR¹⁰SO₂NR¹¹R¹², -OSO₂NR¹⁰R¹¹ und Pseudohalogen; und
**L** eine Bindung oder ein Rest ausgewählt aus der Gruppe bestehend aus gegebenenfalls einfach oder mehrfach substituiertem C₁₋₁₆-Alkyl, C₂₋₁₆-Alkenyl, C₂₋₁₆-Alkinyl, C₃₋₁₀-Cycloalkyl, Aryl, Heterocyclyl und Heteroaryl, wobei der/die Substituent(en) gleich oder verschieden sein können und ausgewählt werden aus der Gruppe bestehend aus Halogen, -NO₂, -OR¹⁰, -C(=O)R¹⁰, -C(=O)OR¹⁰, -C(=O)NR¹⁰R¹¹, -NR¹⁰R¹¹, -NR¹⁰COR¹¹, -NR¹⁰C(=O)OR¹¹, -NR¹⁰C(=O)NR¹¹R¹², -NR¹⁰C(=O)ONR¹¹R¹², -NR¹⁰SO₂R¹¹, -N=CR¹⁰R¹¹, -SR¹⁰, -SOR¹⁰, -SO₂R¹⁰, -SO₂NR¹⁰R¹¹, -NR¹⁰SO₂NR¹¹R¹², -OSO₂NR¹⁰R¹¹ und Pseudohalogen; und
**Q₃** und **Q₄** unabhängig voneinander eine Bindung oder ein Rest ausgewählt aus der Gruppe bestehend aus gegebenenfalls einfach oder mehrfach substitutierten mono- oder bizyklische Heterocyclyl wobei der/die Substituent(en) gleich oder verschieden sein können und ausgewählt werden aus der Gruppe bestehend aus Methyl, Ethyl, Halogen, -NH₂, -OH und Pseudohalogen; und
**R⁹** ein Rest ausgewählt aus der Gruppe bestehend aus gegebenenfalls einfach oder mehrfach substituiertem C₁₋₁₆-Alkyl, C₂₋₁₆-Alkenyl, C₂₋₁₆-Alkinyl, C₃₋₁₀-Cycloalkyl, Aryl, Heterocyclyl und Heteroaryl, wobei der/die Substituent(en) gleich oder verschieden sein können und ausgewählt werden aus der Gruppe bestehend aus Halogen-, -NO₂, -OR¹⁰, -C(=O)R¹⁰, -C(=O)OR¹⁰, -C(=O)NR¹⁰R¹¹, -NR¹⁰R¹¹, -NR¹⁰COR¹¹, -NR¹⁰C(=O)OR¹¹, -NR¹⁰C(=O)NR¹¹R¹², -NR¹⁰C(=O)ONR¹¹R¹², -NR¹⁰SO₂R¹¹, -N=CR¹⁰R¹¹, -SR¹⁰, -SOR¹⁰, -SO₂R¹⁰, -SO₂NR¹⁰R¹¹, -NR¹⁰SO₂NR¹¹R¹², -OSO₂NR¹⁰R¹¹ und Pseudohalogen; und
**R¹⁰, R¹¹** und **R¹²** jeweils unabhängig voneinander Wasserstoff oder ein Rest ausgewählt aus der Gruppe bestehend aus gegebenenfalls einfach oder mehrfach substituiertem C₁₋₈-Alkyl, C₂₋₈-Alkenyl, C₂₋₉-Alkinyl, C₃₋₁₀-Cycloalkyl, Aryl, Heterocyclyl und Heteroaryl, wobei der/die Substituent(en) gleich oder verschieden sein können und ausgewählt werden aus der Gruppe bestehend aus Halogen, -NH₂, -OH und Pseudohalogen;
wobei sich Aryl auf monozyklische oder bizyklische Ringe mit 6-12 Kohlenstoffatomen bezieht;
wobei unter Heteroaryl mono- oder bizyklische Ringe zu verstehen sind, welche anstelle eines oder mehrerer Kohlenstoffatome ein oder mehrere, gleich oder verschiedene Heteroatome, ausgewählt aus Stickstoff- Schwefel- und Sauerstoffatomen, enthalten;
wobei sich Heterocyclyl auf 5-12 Kohlenstoffatome umfassende gesättigte oder ungesättigte, nicht aromatische mono-, bizyklische oder überbrückte bizyklische Ringe bezieht, welche anstelle eines oder mehrerer Kohlenstoffatome Heteroatome ausgewählt aus Stickstoff-Schwefel- und Saucrstoffatomen, enthalten;
gegebenenfalls in Form ihrer Tautomeren, ihrer Racemate, ihrer Enantiomerc, ihrer Diastercomere und ihrer Gemische, sowie gegebenenfalls ihrer pharmakologisch unbedenklichen Säureadditionssalze, bedeuten.

2. Verbindungen nach Anspruch 1, wobei
**X** -NR^{1a} oder Sauerstoff, und
**A** ausgewählt ist aus den Formeln (i) oder (ii) 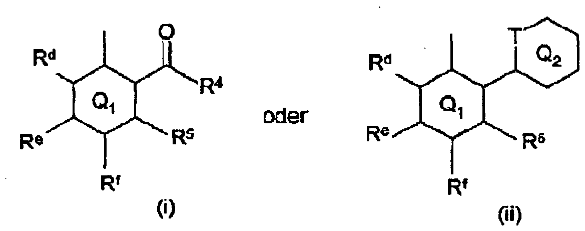 und
**Q₁** mono- oder bizyklische Arylverbindungen; und
**Q₂** monozyklische Heteroarylverbindungen; und
**T** N, O oder S, und
**R¹ und R^{1a}** Wasserstoff, und
**R³** Formel (iii), 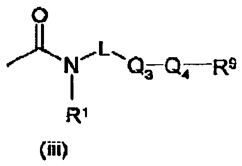
bedeuten und die übrigen Reste wie vorstehend erwähnt definiert sind.

3. Verbindungen nach Anspruch 1 oder 2, wobei
**Y** CH; und
**Q₁** monozyklische Arylverbindungen
bedeuten und die übrigen Reste wie vorstehend erwähnt definiert sind.

4. Verbindungen nach Anspruch 1-3, wobei
**R^{c}** ein Rest ausgewählt aus der Gruppe bestehend aus Wasserstoff -F, -Cl, Methyl und Ethyl
bedeutet und die übrigen Reste wie vorstehend erwähnt definiert sind.

5. Verbindungen nach Anspruch 1-4, wobei
**R^{a}** und **R^{b}** jeweils unabhängig voneinander Wasserstoff oder ein gegebenenfalls einfach oder mehrfach substituierter Rest ausgewählt aus der Gruppe bestehend aus C₁₋₂-Alkyl, C₂-Alkenyl, C₂-Alkinyl, C₃₋₆-Cycloalkyl, Aryl, Heterocyclyl und Heteroaryl, wobei
der/die Substituenten gleich oder verschieden sein können und ausgewählt werden aus der Gruppe bestehend aus Wasserstoff, Halogen, -NO₂, -OR⁶, -C(=O)R⁶, -C(=O)OR⁶, -C(=O)NR⁶R⁷, -NR⁶R⁷, -NR⁶C(=O)R⁷, -NR⁶C(=O)OR⁷, -NR⁶C(=O)NR⁷R⁸, -NR⁶SO₂R⁷, -N=CR⁶R⁷, -SR⁶, -SOR⁶, -SO₂R⁶, -SO₂NR⁶R⁷, -NR⁶, -SO₂NR⁷R⁸, -OSO₂NR⁷R⁸ und Pseudohalogen bedeuten und die übrigen Reste wie vorstehend erwähnt definiert sind.

6. Verbindungen nach Anspruch 1-5, wobei
**R^{a}** und **R^{b}** Wasserstoff
bedeuten und die übrigen Reste wie vorstehend erwähnt definiert sind.

7. Verbindungen - oder deren pharmazeutisch wirksamen Salze - nach Ansprüchen 1 bis 6 zur Verwendung als Arzneimittel.

8. Verbindungen- oder deren pharmazeutisch wirksamen Salze - nach Ansprüchen 1 bis 6 zur Herstellung eines Arzneimittels mit antiproliferativer Wirkung.

9. Verbindungen oder deren pharmazeutisch wirksamen Salze - nach Ansprüchen 1 bis 6 zur Herstellung eines Arzneimittels mit antiproliferativer Wirkung mit einem selektiven, kinaseinhibierendem Wirkmechanismus.

10. Verbindungen - oder deren pharmazeutisch wirksamen Salze - nach Ansprüchen 1 bis 6 zur Herstellung eines Arzneimittels mit antiproliferativer Wirkung mit einem PLK inhibierendem Wirkmechanismus.

11. Pharmazeutische Zubereitungen, enthaltend als Wirkstoff eine oder mehrere Verbindungen der allgemeinen Formel (1) gemäß einem der Ansprüche 1 bis 6 oder deren physiologisch verträgliche Salze gegebenenfalls in Kombination mit üblichen Hilfs- und/oder Trägerstoffen.

12. Verwendung einer Verbindung nach Anspruch 1 bis 6 zur Herstellung eines Arzneimittels zur Behandlung und/oder Prävention von Krebs, Infektionen, Entzündungs- und Autoimmunerkrankungen.

13. Pharmazeutische Präperation umfassend eine Verbindung der allgemeinen Formel (1) 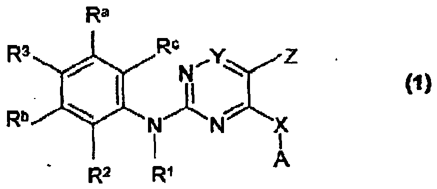 worin
**X** -NR^{1a}, O oder S, und
**Y** CH und
**Z** C₁₋₃-Fluoralkyl- und
**A** ausgewählt ist aus den Formeln (i) oder (ii) 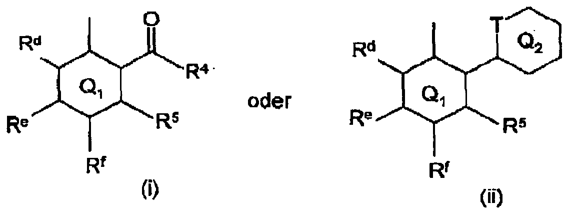 und
**Q₁** mono- oder bizyklische Arylverbindungen; und
**Q₂** mono- oder bizyklische Heteroarylverbindungen; und
**T** N, O oder S, und
**R¹** und **R^{1a}** Wasserstoff oder Methyl, und
**R²** ein Rest ausgewählt aus der Gruppe bestehend aus -Cl, -Br, -I, -OR⁶, -C(=O)R⁶, -C(=O)NR⁶R⁷, -NR⁶R⁷, -NR⁶C(=O)R⁷, -NR⁶SO₂R⁷, -N=CR⁶R⁷, -SR⁶, -SOR⁶, -SO₂R⁶, -SO₂NR⁶R⁷ und Pseudohalogen, oder ein gegebenenfalls einfach oder mehrfach substituierter Rest ausgewählt aus der Gruppe bestehend aus C₁₋₆-Alkyl, C₂₋₆-Alkenyl, C₂₋₆-Alkinyl, C₃₋₆-Cycloalkyl, Aryl, Heterocyclyl und Heteroaryl, wobei der/die Substituenten gleich oder verschieden sein können und ausgewählt werden aus der Gruppe bestehend aus Halogen, -NO₂, -OR⁶, -C(=O)R⁶, -C(=O)OR⁶, -C(=O)NR⁶R⁷, -NR⁶R⁷, -NR⁶C(=O)R⁷, -NR⁶C(=O)OR⁷, -NR⁶C(=O)NR⁷R⁸, -NR⁶SO₂R⁷, -N=CR⁶R⁷, -SR⁶, -SOR⁶, -SO₂R⁶, -SO₂NR⁶R⁷, -NR⁶SO₂NR⁷R⁸, -OSO₂NR⁷R⁸ und Pseudohalogen; und
**R^{a}, R^{b}, R^{c}, R^{d}, R^{e}** und **R^{f}** jeweils unabhängig voneinander ein Rest ausgewählt aus der Gruppe bestehend aus Wasserstoff, Halogen, -NO₂, -OR⁶, -C(=O)R⁶, -C(=O)OR⁶, -C(=O)NR⁶R⁷, -NR⁶R⁷, -NR⁶C(=O)R⁷, -NR⁶C(=O)OR⁷, -NR⁶C(=O)NR⁷R⁸, -NR⁶SO₂R⁷, -N=CR⁶R⁷, -SR⁶, -SOR⁶, -SO₂R⁶, -SO₂NR⁶R⁷, NR⁶SO₂NR⁷R⁸, -OSO₂NR⁷R⁸ und Pseudohalogen;
oder ein gegebenenfalls einfach oder mehrfach substituierter Rest ausgewählt aus der Gruppe bestehend aus C₁₋₆-Alkyl, C₂₋₆-Alkenyl, C₂₋₆-Alkinyl, C₃₋₆-Cycloalkyl, Aryl, Heterocyclyl und Heteroaryl, wobei der/die Substituenten gleich oder verschieden sein können und ausgewählt werden aus der Gruppe bestehend aus Wasserstoff, Halogen, -NO₂, -OR⁶, -C(=O)R⁶, -C(=O)OR⁶, -C(=O)NR⁶R⁷, NR⁶R⁷, -NR⁶C(=O)R⁷, -NR⁶C(=O)OR⁷, -NR⁶C(=O)NR⁷R⁸, -NR⁶SO₂R⁷, -N=CR⁶R, -SR⁶, -SOR⁶, -SO₂R⁶, -SO₂NR⁶R⁷, -NR⁶SO₂NR⁷R⁸, -OSO₂NR⁷R⁸ und Pseudohalogen; und
R³ ausgewählt aus den Formeln (iii) - (ix), 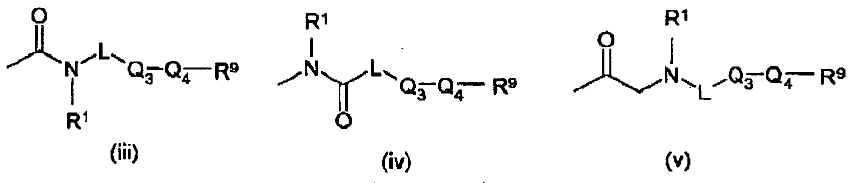 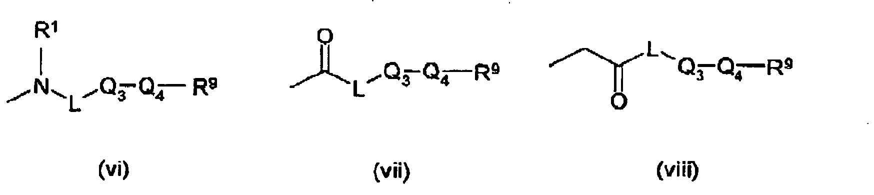 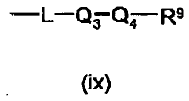 und
**R⁴** ein Rest ausgewählt aus der Gruppe bestehend aus Wasserstoff, -OR⁶, -C(=O)R⁶, -C(=O)OR⁶, -C(=O)NR⁶R⁷, -NR⁶R⁷, -NR⁶C(=O)R⁷, -NR⁶C(=O)OR⁷, -NR⁶C(=O)NR⁷R⁸, -NR⁶SO₂R⁷, -N=CR⁶R⁷, -SR⁶, -SOR⁶, -SO₂R⁶, -SO₂NR⁶R⁷, -NR⁶SO₂NR⁷R⁸, -OSO₂NR⁷R⁸ und Pseudohalogen, oder ein Rest ausgewählt aus der Gruppe bestehend aus gegebenenfalls einfach oder mehrfach substituiertem C₁₋₈-Alkyl, C₂₋₁₀-Alkcnyl, C₂₋₁₀-Alkinyl, C₃₋₈-Cycloalkyl, Aryl, Heterocyclyl und Heteroaryl, wobei der/die Substituent(en) gleich oder verschieden sein können und ausgewählt werden aus der Gruppe bestehend aus C₁₋₃-Alkyl, Halogen, -NO₂, -OR⁶, -C(=O)R⁶, -C(=O)OR⁶, -C(=O)NR⁶R⁷, -NR⁶R⁷, -NR⁶C(=O)R⁷, -NR⁶C(=O)OR⁷, -NR⁶C(=O)NR¹R⁸, -NR⁶SO₂R⁷, -N=CR⁶R⁷, -SR⁶, -SOR⁶, -SO₂R⁶, -SO₂NR⁶R⁷, -NR₆SO₂NR⁷R⁸, -OSO₂NR⁷R⁸ und Pseudohalogen; und
**R⁵** Wasserstoff, Halogen, -CF₃, C₁₋₃-Alkyl oder -OR⁶; und
**R⁶, R⁷** und **R⁸** jeweils unabhängig voneinander Wasserstoff oder ein Rest ausgewählt aus der Gruppe bestehend aus gegebenenfalls einfach oder mehrfach substituiertem C₁₋₅-Alkyl, C₂₋₅-Alkenyl, C₂₋₅-Alkinyl, C₃₋₁₀-Cycloalkyl, Aryl, Heterocyclyl und Heteroaryl, wobei der/die Substituenten gleich oder verschieden sein können und ausgewählt werden aus der Gruppe bestehend aus C₃₋₁₀-Cycloalkyl, Aryl, Heterocyclyl, Heteroaryl, Halogen, -NO₂, -OR¹⁰, -C(=O)R¹⁰, -C(=O)OR¹⁰, -C(=O)NR¹⁰R¹¹, -NR¹⁰R¹¹, -NR¹⁰C(=O)R¹¹, -NR¹⁰C(=O)OR¹¹, -NR¹⁰C(=O)NR¹¹R¹², -NR¹⁰C(=O)ONR¹¹R¹², -NR¹⁰SO₂R¹¹, -N=CR¹⁰R¹¹, -SR¹⁰, -SOR¹⁰, -SO₂R¹⁰, -SO₂NR¹⁰R¹¹, -NR¹⁰SO₂NR¹¹R¹², -OSO₂NR¹⁰R¹¹ und Pseudohalogen; und
L eine Bindung oder ein Rest ausgewählt aus der Gruppe bestehend aus gegebenenfalls einfach oder mehrfach substituiertem C₁₋₁₆-Alkyl, C₂₋₁₈-Alkenyl, C₂₋₁₆-Alkinyl, C₃₋₁₀-Cycloalkyl, Aryl, Heterocyclyl und Heteroaryl, wobei der/die Substituent(en) gleich oder verschieden sein können und ausgewählt werden aus der Gruppe bestehend aus Halogen, -NO₂, -OR¹⁰, -C(=O)R¹⁰, -C(=O)OR¹⁰, -C(=O)NR¹⁰R¹¹, -NR¹⁰R¹¹, -NR¹⁰COR¹¹, -NR¹⁰C(=O)OR¹¹, NR¹⁰C(-O)NR¹¹R¹², -NR¹⁰C(=O)ONR¹¹R¹², -NR¹⁰SO₂R¹¹, -N=CR¹⁰R¹¹, -SR¹⁰, -SOR¹⁰, -SO₂R¹⁰, -SO₂NR¹⁰R¹¹, -NR¹⁰SO₂NR¹¹R¹², -OSO₂NR¹⁰R¹¹ und Pseudohalogen; und
**Q₃** und **Q₄** unabhängig voneinander eine Bindung oder ein Rest ausgewählt aus der Gruppe bestehend aus gegebenenfalls einfach oder mehrfach substitutierten mono- oder bizyklische Heterocyclyl wobei der/die Substituent(en) gleich oder verschieden sein können und ausgewählt werden aus der Gruppe bestehend aus Methyl, Ethyl, Halogen, -NH₂, -OH und Pseudohalogen, und
**R⁹** ein Rest ausgewählt aus der Gruppe bestehend aus gegebenenfalls einfach oder mehrfach substituiertem C₁₋₁₆-Alkyl, C₂₋₁₆-Alkenyl, C₂₋₁₆-Alkinyl, C₃₋₁₀-Cycloalkyl, Aryl, Heterocyclyl und Heteroaryl, wobei der/die Substituent(en) gleich oder verschieden sein können und angewählt werden aus der Gruppe bestehend aus Halogen-, -NO₂, -OR¹⁰, -C(=O)R¹⁰, -C(=O)OR¹⁰, -C(=O)NR¹⁰R¹¹, -NR¹⁰R¹¹, -NR¹⁰COR¹¹, -NR¹⁰C(=O)OR¹¹, -NR¹⁰C(=O)NR¹¹R¹², -NR¹⁰C(=O)ONR¹¹R¹², -NR¹⁰SO₂R¹¹, -N=CR¹⁰R¹¹, -SR¹⁰, -SOR¹⁰, -SO₂R¹⁰, -SO₂NR¹⁰R¹¹, -NR¹⁰SO₂NR¹¹R¹², -OSO₂NR¹⁰R¹¹ und Pseudohalogen; und
**R¹⁰, R¹¹** und **R¹²** jeweils unabhängig voneinander Wasserstoff oder ein Rest ausgewählt aus der Gruppe bestehend aus gegebenenfalls einfach oder mehrfach substituiertem C₁₋₃-Alkyl, C₂₋₈-Alkenyl, C₂₋₃-Alkinyl, C₃₋₁₀-Cycloalkyl, Aryl, Heterocyclyl und Heteroaryl, wobei der/die Substituent(en) gleich oder verschieden sein können und ausgewählt werden aus der Gruppe bestehend aus Halogen, -NH₂, -OH und Pseudohalogen;
wobei sich Aryl auf monozyklische oder bizyklische Ringe mit 6-12 Kohlenstoffatomen bezieht;
wobei unter Heteroaryl mono- oder bizyklische Ringe zu verstehen sind, welche anstelle eines oder mehrerer Kohlenstoffatome ein oder mehrere, gleich oder verschiedene Heteroatome, ausgewählt aus Stickstoff- Schwefel- und Sauerstoffatomen, enthalten;
wobei sich Heterocyclyl auf 5-12 Kohlenstoffatome umfassende gesättigte oder ungesättigte, nicht aromatische mono-, bizyklische oder überbrückte bizyklische Ringe bezieht, welche anstelle eines oder mehrerer Kohlenstoffatome Heteroatome ausgewählt aus Stickstoff-Schwefel- und Sauerstoffatomen, enthalten;
gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze, und
mindestens eine weitere Wirksubstanz ausgewählt aus der Gruppe bestehend aus zytostatischen Wirksubstanzen, zytotoxische Wirksubstanzen, Steroide und Antikörper, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

## Claims

1. Compounds of general formula (1) 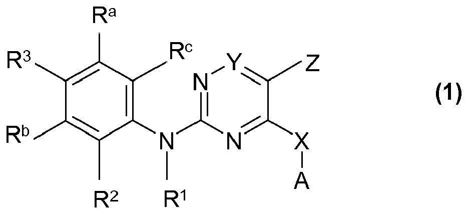 wherein
**X** denotes -NR^{1a}, O or S, and
**Y** denotes CH, and
**Z** denotes C₁₋₃-fluoroalkyl, and
**A** is selected from formulae (i) or (ii) 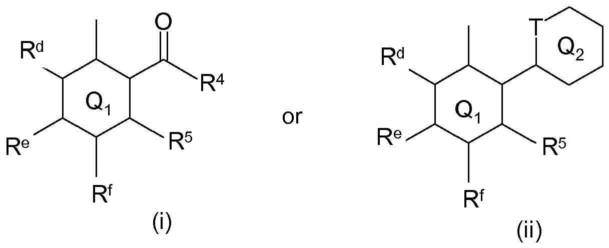 and
**Q₁** denotes mono- or bicyclic aryl compounds; and
Q₂ denotes mono- or bicyclic heteroaryl compounds; and
T denotes N, O or S, and
**R¹** and **R^{1a}** denote hydrogen or methyl, and
**R²** denotes a group selected from among -Cl, -Br, -I, -OR⁶, -C(=O)R⁶, -C(=O)NR⁶R⁷, -NR⁶R⁷, -NR⁶C(=O)R⁷, -NR⁶SO₂R⁷, -N=CR⁶R⁷, -SR⁶, -SOR⁶, -SO₂R⁶, -SO₂NR⁶R⁷ and pseudohalogen, or an optionally mono- or polysubstituted group selected from among C₁₋₆-alkyl, C₂₋₆-alkenyl, C₂₋₆-alkynyl, C₃₋₆-cycloalkyl, aryl, heterocyclyl and heteroaryl, wherein the substituent(s) may be identical or different and are selected from among halogen, -NO₂, -OR⁶, -C(=O)R⁶, -C(=O)OR⁶, -C(=O)NR⁶R⁷, -NR⁶R⁷, -NR⁶C(=O)R⁷, -NR⁶C(=O)OR⁷, -NR⁶C(=O)NR⁷R⁸, -NR⁶SO₂R⁷, -N=CR⁶R⁷, -SR⁶, -SOR⁶, -SO₂R⁶, -SO₂NR⁶R⁷, -NR⁶SO₂NR⁷R⁸, -OSO₂NR⁷R⁸ and pseudohalogen; and
**R^{a}, R^{b}, R^{c}, R^{d}, R^{e}** and **R^{f}** each independently of one another denote a group selected from among hydrogen, halogen, -NO₂, -OR⁶, -C(=O)R⁶, -C(=O)OR⁶, -C(=O)NR⁶R⁷, -NR⁶R⁷, -NR⁶C(=O)R⁷, -NR⁶C(=O)OR⁷, -NR⁶C(=O)NR⁷R⁸, -NR⁶SO₂R⁷, -N=CR⁶R⁷, -SR⁶, -SOR⁶, -SO₂R⁶, -SO₂NR⁶R⁷, -NR⁶SO₂NR⁷R⁸, -OSO₂NR⁷R⁸ and pseudohalogen; or an optionally mono- or polysubstituted group selected from among C₁₋₆-alkyl, C₂₋₆-alkenyl, C₂₋₆-alkynyl, C₃₋₆-cycloalkyl, aryl, heterocyclyl and heteroaryl, wherein the substituent(s) may be identical or different and are selected from among hydrogen, halogen, -NO₂, -OR⁶, -C(=O)R⁶, -C(=O)OR⁶, -C(=O)NR⁶R⁷, -NR⁶R⁷, -NR⁶C(=O)R⁷, -NR⁶C(=O)OR⁷, -NR⁶C(=O)NR⁷R⁸, -NR⁶SO₂R⁷, -N=CR⁶R⁷, -SR⁶, -SOR⁶, -SO₂R⁶, -SO₂NR⁶R⁷, -NR⁶SO₂NR⁷R⁸, -OSO₂NR⁷R⁸ and pseudohalogen; and
**R³** is selected from the formulae (iii) - (ix), 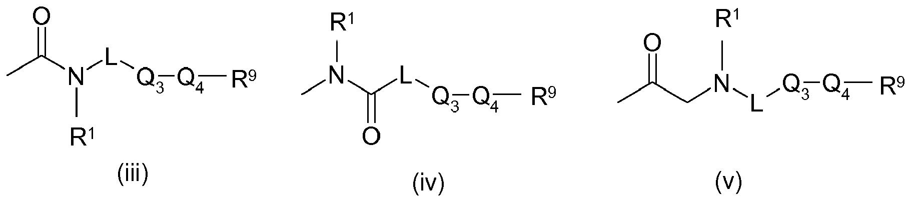 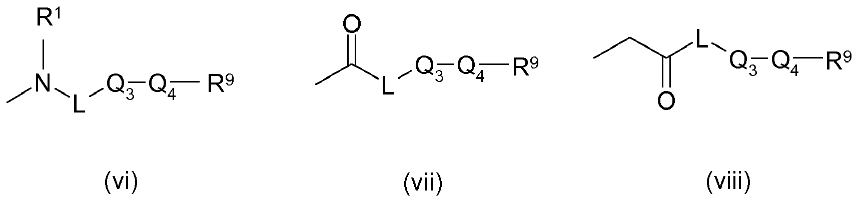 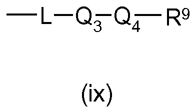 and
**R⁴** denotes a group selected from among hydrogen, -OR⁶, -C(=O)R⁶, -C(=O)OR⁶, -C(=O)NR⁶R⁷, -NR⁶R⁷, -NR⁶C(=O) R⁷, -NR⁶C(=O)OR⁷, -NR⁶C(=O)NR⁷R⁸, -NR⁶SO₂R⁷, -N=CR⁶R⁷, -SR⁶, -SOR⁶, -SO₂R⁶, -SO₂NR⁶R⁷, -NR⁶SO₂NR⁷R⁸, -OSO₂NR⁷R⁸ and pseudohalogen,
or denotes a group selected from among optionally mono- or polysubstituted C₁₋₈-alkyl, C₂₋₁₀-alkenyl, C₂₋₁₀-alkynyl, C₃₋₈-cycloalkyl, aryl, heterocyclyl and heteroaryl, wherein the substituent(s) may be identical or different and are selected from among C₁₋₈-alkyl, halogen, -NO₂, -OR⁶, -C(=O)R⁶, -C(=O)OR⁶, -C(=O)NR⁶R⁷, -NR⁶R⁷, -NR⁶C(=O)R⁷, -NR⁶C(=O)OR⁷, -NR⁶C(=O)NR⁷R⁸, -NR⁶SO₂R⁷, -N=CR⁶R⁷, -SR⁶, -SOR⁶, -SO₂R⁶, -SO₂NR⁶R⁷, -NR⁶SO₂NR⁷R⁸, -OSO₂NR⁷R⁸ and pseudohalogen; and
**R⁵** denotes hydrogen, halogen, -CF₃, C₁₋₃-alkyl or -OR⁶; and
**R⁶, R⁷** and **R⁸** each independently of one another denote hydrogen or a group selected from among optionally mono- or polysubstituted C₁₋₅-alkyl, C₂₋₅-alkenyl, C₂₋₅-alkynyl, C₃₋₁₀-cycloalkyl, aryl, heterocyclyl and heteroaryl, wherein the substituent(s) may be identical or different and are selected from among C₃₋₁₀-cycloalkyl, aryl, heterocyclyl, heteroaryl, halogen, -NO₂, -OR¹⁰, -C(=O)R¹⁰, -C(=O)OR¹⁰, -C(=O)NR¹⁰R¹¹, -NR¹⁰R¹¹, -NR¹⁰C(=O)R¹¹, -NR¹⁰C(=O)OR¹¹, -NR¹⁰C(=O)NR¹¹R¹², -NR¹⁰C(=O)ONR¹¹R¹², -NR¹⁰SO₂R¹¹, -N=CR¹⁰R¹¹, -SR¹⁰, -SOR¹⁰, -SO₂R¹⁰, -SO₂NR¹⁰R¹¹, -NR¹⁰SO₂NR¹¹R¹², -OSO₂NR¹⁰R¹¹ and pseudohalogen; and
**L** denotes a bond or a group selected from among optionally mono- or polysubstituted C₁₋₁₆-alkyl, C₂₋₁₆-alkenyl, C₂₋₁₆-alkynyl, C₃₋₁₀-cycloalkyl, aryl, heterocyclyl and heteroaryl, wherein the substituent(s) may be identical or different and are selected from among halogen, -NO₂, -OR¹⁰, -C(=O)R¹⁰, -C(=O)OR¹⁰, -C(=O)NR¹⁰R¹¹, -NR¹⁰R¹¹, -NR¹⁰COR¹¹, -NR¹⁰C(=O)OR¹¹, -NR¹⁰C(=O)NR¹¹R¹², -NR¹⁰C(=O)ONR¹¹R¹², -NR¹⁰SO₂R¹¹, -N=CR¹⁰R¹¹, -SR¹⁰, -SOR¹⁰, -SO₂R¹⁰, -SO₂NR¹⁰R¹¹, -NR¹⁰SO₂NR¹¹R¹², -OSO₂NR¹⁰R¹¹ and pseudohalogen; and
**Q₃** and **Q₄** independently of one another denote a bond or a group selected from among optionally mono- or polysubstituted mono- or bicyclic heterocyclyl, wherein the substituent(s) may be identical or different and are selected from among methyl, ethyl, halogen, -NH₂, -OH and pseudohalogen; and
**R⁹** denotes a group selected from among optionally mono- or polysubstituted C₁₋₁₆-alkyl, C₂₋₁₆-alkenyl, C₂₋₁₆-alkynyl, C₃₋₁₀-cycloalkyl, aryl, heterocyclyl and heteroaryl, wherein the substituent(s) may be identical or different and are selected from among halogen, -NO₂, -OR¹⁰, -C(=O)R¹⁰, -C(=O)OR⁶, -C(=O)NR¹⁰R¹¹, -NR¹⁰R¹¹, -NR¹⁰COR¹¹, -NR¹⁰C(=O)OR¹¹, -NR¹⁰C(=O)NR¹¹R¹², -NR¹⁰C(=O)ONR¹¹R¹², -NR¹⁰SO₂R¹¹, -N=CR¹⁰R¹¹, -SR¹⁰, -SOR¹⁰, -SO₂R¹⁰, -SO₂NR¹⁰R¹¹, -NR¹⁰SO₂NR¹¹R¹², -OSO₂NR¹⁰R¹¹ and pseudohalogen; and
**R¹⁰, R¹¹** and **R¹²** each independently of one another denote hydrogen or a group selected from among optionally mono- or polysubstituted C₁₋₈-alkyl, C₂₋₈-alkenyl, C₂₋₈-alkynyl, C₃₋₁₀-cycloalkyl, aryl, heterocyclyl and heteroaryl, while the substituent(s) may be identical or different and are selected from among halogen, -NH₂, -OH and pseudohalogen;
wherein aryl relates to monocyclic or bicyclic rings with 6-12 carbon atoms; wherein by heteroaryl are meant monocyclic or bicyclic rings which contain,
instead of one or more carbon atoms, one or more identical or different heteroatoms, selected from among nitrogen, sulphur and oxygen atoms;
wherein heterocyclyl relates to saturated or unsaturated, non-aromatic, mono-, bicyclic or bridged bicyclic rings comprising 5-12 carbon atoms, which contain, instead of one or more carbon atoms, heteroatoms selected from among nitrogen, sulphur and oxygen atoms;
optionally in the form of the tautomers, the racemates, the enantiomers, the diastereomers and the mixtures thereof, as well as optionally the pharmacologically acceptable acid addition salts thereof.

2. Compounds according to claim 1, wherein
**X** denotes -NR^{1a} or oxygen, and
**A** is selected from formulae (i) or (ii) 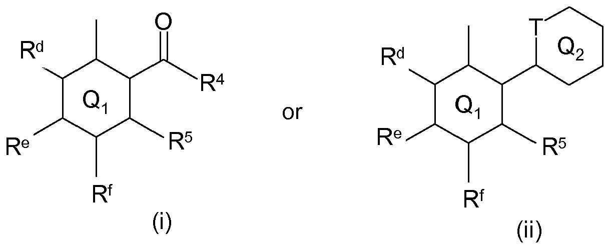 and
**Q₁** denotes mono- or bicyclic aryl compounds; and
**Q₂** denotes monocyclic heteroaryl compounds; and
**T** denotes N, O or S, and
**R¹** and **R^{1a}** denote hydrogen; and
**R³** denotes formula (iii), 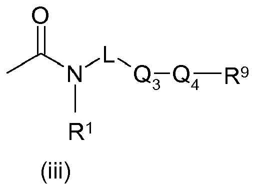
and the other groups are as hereinbefore defined.

3. Compounds according to claim 1 or 2, wherein
**Y** denotes CH; and
**Q₁** denotes monocyclic aryl compounds
and the other groups are as hereinbefore defined.

4. Compounds according to claims 1 - 3, wherein
**R^{c}** denotes a group selected from among hydrogen, -F, -Cl, methyl and ethyl
and the other groups are as hereinbefore defined.

5. Compounds according to claims 1 - 4, wherein
**R^{a}** and **R^{b}** each independently of one another denote hydrogen or an optionally mono- or polysubstituted group selected from among C₁₋₂-alkyl, C₂-alkenyl, C₂-alkynyl, C₃₋₆-cycloalkyl, aryl, heterocyclyl and heteroaryl, wherein the substituent(s) may be identical or different and are selected from among hydrogen, halogen, -NO₂, -OR⁶, -C(=O)R⁶, -C(=O)OR⁶, -C(=O)NR⁶R⁷, -NR⁶R⁷, -NR⁶C(=O)R⁷, -NR⁶C(=O)OR⁷, -NR⁶C(=O)NR⁷R⁸, -NR⁶SO₂R⁷, -N=CR⁶R⁷, -SR⁶, -SOR⁶, -SO₂R⁶, -SO₂NR⁶R⁷, -NR⁶, -SO₂NR⁷R⁸, -OSO₂NR⁷R⁸ and pseudohalogen
and the other groups are as hereinbefore defined.

6. Compounds according to claims 1-5, wherein
**R^{a}** and **R^{b}** denote hydrogen
and the other groups are as hereinbefore defined.

7. Compounds - or the pharmaceutically active salts thereof - according to claims 1 to 6 for use as medicaments.

8. Compounds - or the pharmaceutically active salts thereof - according to claims 1 to 6 for preparing a medicament with an antiproliferative activity.

9. Compounds - or the pharmaceutically active salts thereof - according to claims 1 to 6 for preparing a medicament with an antiproliferative activity with a selective, kinase-inhibiting mechanism of activity.

10. Compounds - or the pharmaceutically active salts thereof - according to claims 1 to 6 for preparing a medicament with an antiproliferative activity with a PLK- inhibiting mechanism of activity.

11. Pharmaceutical preparations, containing as active substance one or more compounds of general formula (1) according to one of claims 1 to 6 or the physiologically acceptable salts thereof, optionally in conjunction with conventional excipients and/or carriers.

12. Use of a compound according to claims 1 to 6 for preparing a medicament for the treatment and/or prevention of cancer, infections, inflammatory and autoimmune diseases.

13. Pharmaceutical preparation comprising a compound of general formula (1) 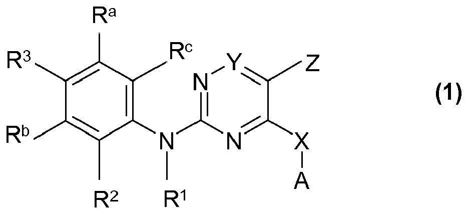 wherein
**X** denotes -NR^{1a}, O or S, and
**Y** denotes CH, and
**Z** denotes C₁₋₃-fluoroalkyl and
**A** is selected from formulae (i) or (ii) 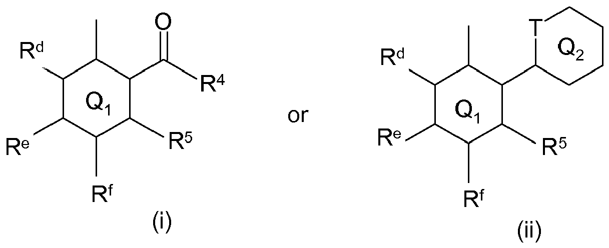 **and**
**Q₁** denotes mono- or bicyclic aryl compounds; and
**Q₂** denotes mono- or bicyclic heteroaryl compounds; and
**T** denotes N, O or S, and
**R¹** and **R^{1a}** denote hydrogen or methyl, and
**R²** denotes a group selected from among -Cl, -Br, -I, -OR⁶, -C(=O)R⁶, -C(=O)NR⁶R⁷, -NR⁶R⁷, -NR⁶C(=O)R⁷, -NR⁶SO₂R⁷, -N=CR⁶R⁷, -SR⁶, -SOR⁶, -SO₂R⁶, -SO₂NR⁶R⁷ and pseudohalogen, or an optionally mono- or polysubstituted group selected from among C₁₋₆-alkyl, C₂₋₆-alkenyl, C₂₋₆-alkynyl, C₃₋₆-cycloalkyl, aryl , heterocyclyl and heteroaryl, wherein the substituent(s) may be identical or different and are selected from among halogen, -NO₂, -OR⁶, -C(=O)R⁶, -C(=O)OR⁶, -C(=O)NR⁶R⁷, -NR⁶R⁷, -NR⁶C(=O)R⁷, -NR⁶C(=O)OR⁷, -NR⁶C(=O)NR⁷R⁸, -NR⁶SO₂R⁷, -N=CR⁶R⁷, -SR⁶, -SOR⁶, -SO₂R⁶, -SO₂NR⁶R⁷, -NR⁶SO₂NR⁷R⁸, -OSO₂NR⁷R⁸ and pseudohalogen; and
**R^{a}, R^{b}, R^{c}, R^{d}, R^{e}** and **R^{f}** each independently of one another denote a group selected from among hydrogen, halogen, -NO₂, -OR⁶, -C(=O)R⁶, -C(=O)OR⁶, -C(=O)NR⁶R⁷, -NR⁶R⁷, -NR⁶C(=O)R⁷, -NR⁶C(=O)OR⁷, -NR⁶C(=O)NR⁷R⁸, -NR⁶SO₂R⁷, -N=CR⁶R⁷, -SR⁶, -SOR⁶, -SO₂R⁶, -SO₂NR⁶R⁷, -NR⁶SO₂NR⁷R⁸, -OSO₂NR⁷R⁸ and pseudohalogen; or an optionally mono- or polysubstituted group selected from among C₁₋₆-alkyl, C₂₋₆-alkenyl, C₂₋₆-alkynyl, C₃₋₆-cycloalkyl, aryl, heterocyclyl and heteroaryl, wherein the substituent(s) may be identical or different and are selected from among hydrogen, halogen, -NO₂, -OR⁶, -C(=O)R⁶, -C(=O)OR⁶, -C(=O)NR⁶R⁷, -NR⁶R⁷, -NR⁶C(=O)R⁷, -NR⁶C(=O)OR⁷, -NR⁶C(=O)NR⁷R⁸, -NR⁶SO₂R⁷, -N=CR⁶R⁷, -SR⁶, -SOR⁶, -SO₂R⁶, -SO₂NR⁶R⁷, -NR⁶SO₂NR⁷R⁸, -OSO₂NR⁷R⁸ and pseudohalogen; and
**R³** is selected from the formulae (iii) - (ix), 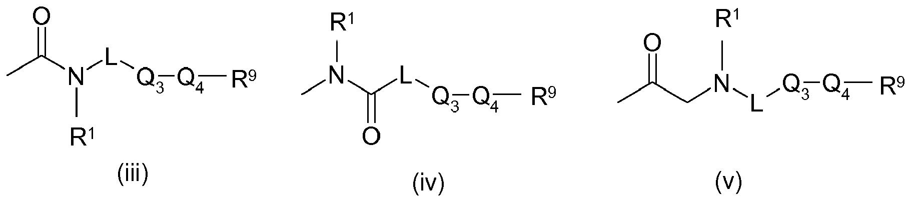 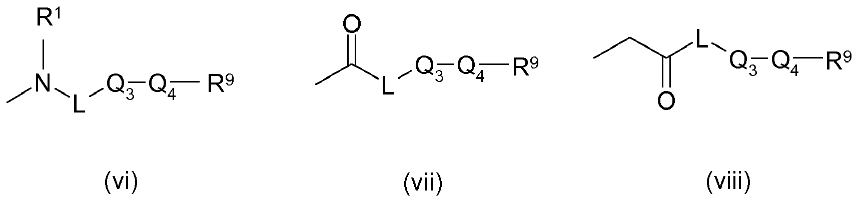 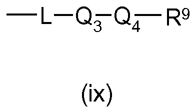 and
**R⁴** denotes a group selected from among hydrogen, -OR⁶, -C(=O)R⁶, -C(=O)OR⁶, -C(=O)NR⁶R⁷, -NR⁶R⁷, -NR⁶C(=O)R⁷, -NR⁶C(=O)OR⁷, -NR⁶C(=O)NR⁷R⁸, -NR⁶SO₂R⁷, -N=CR⁶R⁷, -SR⁶, -SOR⁶, -SO₂R⁶, -SO₂NR⁶R⁷, -NR⁶SO₂NR⁷R⁸, -OSO₂NR⁷R⁸ and pseudohalogen, or denotes a group selected from among optionally mono- or polysubstituted C₁₋₈-alkyl, C₂₋₁₀-alkenyl, C₂₋₁₀-alkynyl, C₃₋₈-cycloalkyl, aryl, heterocyclyl and heteroaryl, wherein the substituent(s) may be identical or different and are selected from among C₁₋₈-alkyl, halogen, -NO₂, -OR⁶, -C(=O)R⁶, -C(=O)OR⁶, -C(=O)NR⁶R⁷, -NR⁶R⁷, -NR⁶C(=O)R⁷, -NR⁶C(=O)OR⁷, -NR⁶C(=O)NR⁷R⁸, -NR⁶SO₂R⁷, -N=CR⁶R⁷, -SR⁶, -SOR⁶, -SO₂R⁶, -SO₂NR⁶R⁷, -NR⁶SO₂NR⁷R⁸, -OSO₂NR⁷R⁸ and pseudohalogen; and
**R⁵** denotes hydrogen, halogen, -CF₃, C₁₋₃-alkyl or -OR⁶; and
**R⁶, R⁷** and **R⁸** each independently of one another denote hydrogen or a group selected from among optionally mono- or polysubstituted C₁₋₅-alkyl, C₂₋₅-alkenyl, C₂₋₅-alkynyl, C₃₋₁₀-cycloalkyl, aryl, heterocyclyl and heteroaryl, wherein the substituent(s) may be identical or different and are selected from among C₃₋₁₀-cycloalkyl, aryl, heterocyclyl, heteroaryl, halogen, -NO₂, -OR¹⁰, -C(=O)R¹⁰, -C(=O)OR⁶, -C(=O)NR¹⁰R¹¹, -NR¹⁰R¹¹, -NR¹⁰C(=O)R¹¹, -NR¹⁰C(=O)OR¹¹, -NR¹⁰C(=O)NR¹¹R¹², -NR¹⁰C(=O)ONR¹¹R¹², -NR¹⁰SO₂R¹¹, -N=CR¹⁰R¹¹, -SR¹⁰, -SOR¹⁰, -SO₂R¹⁰, -SO₂NR¹⁰R¹¹, -NR¹⁰SO₂NR¹¹R¹², -OSO₂NR¹⁰R¹¹ and pseudohalogen; and
**L** denotes a bond or a group selected from among optionally mono- or polysubstituted C₁₋₁₆-alkyl, C₂₋₁₆-alkenyl, C₂₋₁₆-alkynyl, C₃₋₁₀-cycloalkyl, aryl, heterocyclyl and heteroaryl, wherein the substituent(s) may be identical or different and are selected from among halogen, -NO₂, -OR¹⁰, -C(=O)R¹⁰, -C(=O)OR¹⁰, -C(=O)NR¹⁰R¹¹, -NR¹⁰R¹¹, -NR¹⁰COR¹¹, -NR¹⁰C(=O)OR¹¹, -NR¹⁰C(=O)NR¹¹R¹², -NR¹⁰C(=O)ONR¹¹R¹², -NR¹⁰SO₂R¹¹, -N=CR¹⁰R¹¹, -SR¹⁰, -SOR¹⁰, -SO₂R¹⁰, -SO₂NR¹⁰R¹¹, -NR¹⁰SO₂NR¹¹R¹², -OSO₂NR¹⁰R¹¹ and pseudohalogen; and
**Q₃** and **Q₄** independently of one another denote a bond or a group selected from among optionally mono- or polysubstituted mono- or bicyclic heterocyclyl, while the substituent(s) may be identical or different and are selected from among methyl, ethyl, halogen, -NH₂, -OH and pseudohalogen; and
**R⁹** denotes a group selected from among optionally mono- or polysubstituted C₁₋₁₆-alkyl, C₂₋₁₆-alkenyl, C₂₋₁₆-alkynyl, C₃₋₁₀-cycloalkyl, aryl, heterocyclyl and heteroaryl, wherein the substituent(s) may be identical or different and are selected from among halogen, -NO₂, -OR¹⁰, -C(=O)R¹⁰, -C(=O)OR⁶, -C(=O)NR¹⁰R¹¹, -NR¹⁰R¹¹, -NR¹⁰COR¹¹, -NR¹⁰C(=O)OR¹¹, -NR¹⁰C(=O)NR¹¹R¹², -NR¹⁰C(=O)ONR¹¹R¹², -NR¹⁰SO₂R¹¹, -N=CR¹⁰R¹¹, -SR¹⁰, -SOR¹⁰, -SO₂R¹⁰, -SO₂NR¹⁰R¹¹, -NR¹⁰SO₂NR¹¹R¹², -OSO₂NR¹⁰R¹¹ and pseudohalogen; and
**R¹⁰, R¹¹** and **R¹²** each independently of one another denote hydrogen or a group selected from among optionally mono- or polysubstituted C₁₋₈-alkyl, C₂₋₈-alkenyl, C₂₋₈-alkynyl, C₃₋₁₀-cycloalkyl, aryl, heterocyclyl and heteroaryl, wherein the substituent(s) may be identical or different and are selected from among halogen, -NH₂, -OH and pseudohalogen;
wherein aryl relates to monocyclic or bicyclic rings with 6-12 carbon atoms;
wherein by heteroaryl are meant monocyclic or bicyclic rings which contain, instead of one or more carbon atoms, one or more identical or different heteroatoms, selected from among nitrogen, sulphur and oxygen atoms;
wherein heterocyclyl relates to saturated or unsaturated, non-aromatic, mono-, bicyclic or bridged bicyclic rings comprising 5-12 carbon atoms, which contain, instead of one or more carbon atoms, heteroatoms selected from among nitrogen, sulphur and oxygen atoms;
optionally in the form of the tautomers, the racemates, the enantiomers, the diastereomers and the mixtures thereof, as well as optionally the pharmacologically acceptable acid addition salts thereof, and
at least one other active substance selected from among cytostatic active substances, cytotoxic active substances, steroids and antibodies, optionally in the form of the tautomers, the racemates, the enantiomers, the diastereomers and the mixtures thereof, and optionally the pharmacologically acceptable acid addition salts thereof.

## Revendications

1. Composés de formule générale (1) 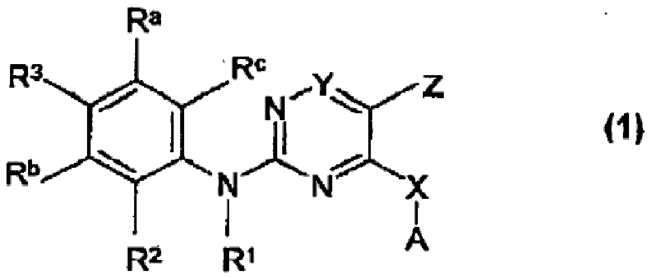 dans laquelle
X représente -NR^{1a}, 0 ou S, et
Y représente CH et
Z représente un groupe fluoroalkyle en C₁ à C₃ et
A est choisi parmi les formules (i) ou (ii) 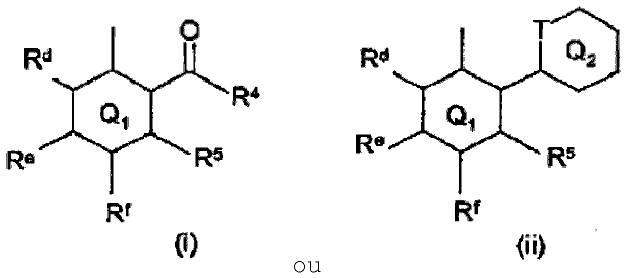 et
Q¹ représente des composés aryle mono- ou bicycliques ; et
Q² représente des composés hétéroaryle mono- ou bicycliques ; et
T représente N, 0 ou S, et
R¹ et R^{1a} représentent un atome d'hydrogène ou un groupe méthyle, et
R² représente un radical choisi dans le groupe constitué de -Cl, -Br, -I, -OR⁶, -C(=O)R⁶, - C(=O)NR⁶R⁷, -NR⁶R⁷, -NR⁶C(=O)R⁷, -NR⁶SO₂R⁷, -N=CR⁶R⁷, -SR⁶, -SOR⁶, -SO₂R⁶, -SO₂NR⁶R⁷ et un pseudohalogène ou un radical éventuellement mono- ou poly-substitué choisi dans le groupe constitué d'un groupe alkyle en C₁ à C₆, alcényle en C₂ à C₆, alcinyle en C₂ à C₆, cycloalkyle en C₃ à C₆, aryle, hétérocyclyle et hétéroaryle, dans lequel le/les substituant(s) peuvent être identiques ou différents et sont choisis dans le groupe constitué d'un atome d'halogène, -NO₂, -OR⁶, - C(=O)R⁶, -C(=O)OR⁶, -C(=O)NR⁶R⁷, -NR⁶R⁷, -NR⁶C(=O)R⁷, -NR⁶C(=O)OR⁷, -NR⁶C (=O)NR⁷R⁸, -NR⁶SO₂R⁷, -N=CR⁶R⁷, - SR⁶, -SOR⁶, -SO₂R⁶, -SO²NR⁶R⁷, -NR⁶SO₂NR⁷R⁸, - OSO₂NR⁷R⁸ et un pseudo-halogène ; et
R^{a}, R^{b}, R^{c}, R^{d}, R^{e} et R^{f} représentent respectivement, indépendamment les uns des autres, un radical choisi dans le groupe constitué d'un atome d'hydrogène, d'halogène, -NO₂, -OR⁶, -C(=O)R⁶, - C(=O)OR⁶, -C(=O)NR⁶R⁷, -NR⁶R⁷, -NR⁶C(=O)R⁷, - NR⁶C(=O)OR⁷, -NR⁶C (=O)NR⁷R⁸, -NR⁶SO₂R⁷, -N=CR⁶R⁷, - SR⁶, -SOR⁶, ₋SO₂R⁶, -SO₂NR⁶R⁷, -NR⁶SO₂NR⁷R⁸, - OSO₂NR⁷R⁸ et un pseudo-halogène ; ou un radical éventuellement mono- ou poly-substitué choisi dans le groupe constitué d'un groupe alkyle en C₁ à C₆, alcényle en C₂ à C₆, alcinyle en C₂ à C₆, cycloalkyle en C₃ à C₆, aryle, hétérocyclyle et hétéroaryle, dans lequel le/les substituants peuvent être identiques ou différents et sont choisis dans le groupe constitué d'un atome d'hydrogène, d'halogène, -NO₂, -OR⁶, -C(=O)R⁶, -C(=O)OR⁶, -C(=O)NR⁶R⁷, -NR⁶R⁷, -NR⁶C(=O)R⁷, - NR⁶C(=O)OR⁷, -NR⁶C(=O)NR⁷R⁸, -NR⁶SO₂R⁷, -N=CR⁶R⁷, - SR⁶, -SOR⁶, -SO₂R⁶, -SO²NR⁶R⁷, -NR⁶SO₂NR⁷R⁸, - OSO₂NR⁷R⁸ et un pseudo-halogène ; et
R³ est choisi parmi les formules (iii) à (ix), 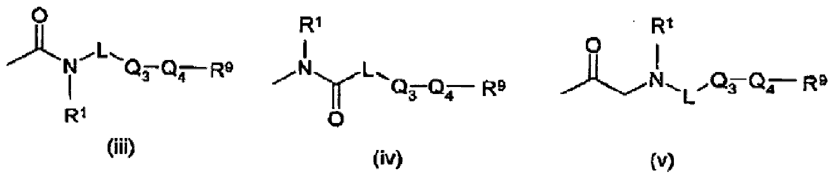 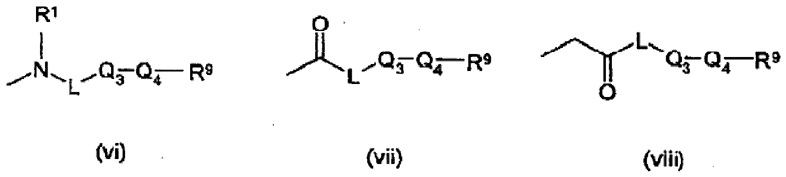 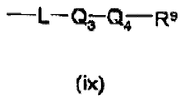 et
R⁴ représente un radical choisi dans le groupe constitué d'un atome d'hydrogène, -OR⁶, -C(=O)R⁶, -C(=O)OR⁶, -C(=O)NR⁶R⁷, -NR⁶R⁷, -NR⁶C(=O)R⁷, - NR⁶C(=O)OR⁷, -NR⁶C(=O)NR⁷R⁸, -NR⁶SO₂R⁷, -N=CR⁶R⁷, - SR⁶, -SOR⁶, -SO₂R⁶, -SO₂NR⁶R⁷, -NR⁶SO₂NR⁷R⁸,-OSO₂NR⁷R⁸ et un pseudo-halogène,
ou un radical choisi dans le groupe constitué des groupes éventuellement mono- ou poly-substitués alkyle en C₁ à C₈, alcényle en C₂ à C₁₀, alcinyle en C₂ à C₁₀, cycloalkyle en C₃ à C₈, aryle, hétérocyclyle et hétéroaryle, dans lequel le/les substituant(s) peuvent être identiques ou différents et sont choisis dans le groupe constitué d'un groupe alkyle en C₁ à C₈, un atome d'halogène, -NO₂, -OR⁶, -C(=O)R⁶, -C(=O)OR⁶, - C(=O)NR⁶R⁷, -NR⁶R⁷, -NR⁶C(=O)R⁷, -NR⁶C (=0) OR⁷, - NR⁶C(=O)NR⁷R⁸, -NR⁶SO₂R⁷, -N=CR⁶R⁷, -SR⁶, -SOR⁶, - SO₂R⁶, -SO₂NR⁶R⁷, -NR⁶SO₂NR⁷R⁸, -OSO₂NR⁷R⁸ et un pseudo-halogène ; et
R⁵ représente un atome d'hydrogène, d'halogène, -CF₃, un groupe alkyle en C₁ à C₃ ou -OR⁶ ; et
R⁶, R⁷ et R⁸ représentent respectivement, indépendamment les uns des autres, un atome d'hydrogène ou un radical choisi dans le groupe constitué des groupes éventuellement mono- ou poly-substitués alkyle en C₁ à C₅, alcényle en C₂ à C₅, alcinyle en C₂ à C₅, cycloalkyle en C₃ à C₁₀, aryle, hétérocyclyle et hétéroaryle, dans lequel le/les substituants peuvent être identiques ou différents et sont choisis dans le groupe constitué d'un groupe cycloalkyle en C₃ à C₁₀, aryle, hétérocyclyle, hétéroaryle, un atome d'halogène, - NO₂, -OR¹⁰, -C(=O)R¹⁰, -C(=O)OR¹⁰, -C(=O)NR¹⁰R¹¹, - NR¹⁰R¹¹, -NR¹⁰C(=0) R¹¹, -NR¹⁰C(=O)OR¹¹, - NR¹⁰C(=O)NR¹¹R¹², -NR¹⁰C(=O)ONR¹¹R¹², -NR¹⁰SO₂R¹¹, - N=CR¹⁰R¹¹, -SR¹⁰, -SOR¹⁰, -SO₂R¹⁰, -SO₂NR¹⁰R¹¹, - NR¹⁰SO₂NR¹¹R¹², -OSO₂NR¹⁰R¹¹ et un pseudo-halogène ; et
L représente une liaison ou un radical choisi dans le groupe constitué des groupes éventuellement mono- ou poly-substitués alkyle en C₁ à C₁₆, alcényle en C₂ à C₁₆, alcinyle en C₂ à C₁₆, cycloalkyle en C₃ à C₁₀, aryle, hétérocyclyle et hétéroaryle, dans lequel le/les substituant(s) peuvent être identiques ou différents et sont choisis dans le groupe constitué d'un atome d'halogène, -NO₂, -OR¹⁰, -C(=O)R¹⁰, -C(=O)OR¹⁰, - C(=O)NR¹⁰R¹¹, -NR¹⁰R¹¹, -NR¹⁰COR¹¹, -NR¹⁰C(=O)OR¹¹, - NR¹⁰C(=O)NR¹¹R¹², -NR¹⁰C(=O)ONR¹¹R¹², -NR¹⁰SO₂R¹¹, - N=CR¹⁰R¹¹, -SR¹⁰, -SOR¹⁰, -SO₂R¹⁰, -SO₂NR¹⁰R¹¹, - NR¹⁰SO₂NR¹¹R¹², -OSO₂NR¹⁰R¹¹ et un pseudo-halogène ; et
Q₃ et Q₄ représentent indépendamment l'un de l'autre, une liaison ou un radical choisi dans le groupe constitué d'un groupe hétérocyclyle mono- ou bicyclique éventuellement mono- ou poly-substitué, dans lequel le/les substituant(s) peuvent être identiques ou différents et sont choisis dans le groupe comprenant un groupe méthyle, éthyle, un atome d'halogène, -NH₂, -OH et un pseudo-halogène ; et
R⁹ représente un radical choisi dans le groupe constitué des groupes éventuellement mono- ou poly-substitués alkyle en C₁ à C₁₆, alcényle en C₂ à C₁₆, alcinyle en C₂ à C₁₆, cycloalkyle en C₃ à C₁₀, aryle, hétérocyclyle et hétéroaryle, dans lequel le/les substituant(s) peuvent être identiques ou différents et sont choisis dans le groupe constitué d'un atome d'halogène, -NO₂, -OR¹⁰, C(=O)R¹⁰, -C(=O)OR¹⁰, -C(=O)NR¹⁰R¹¹, -NR¹⁰R¹¹, - NR¹⁰COR¹¹, -NR¹⁰C(=O)OR¹¹, -NR¹⁰C(=O)NR¹¹R¹², - NR¹⁰C(=O)ONR¹¹R¹², -NR¹⁰SO₂R¹¹, -N=CR¹⁰R¹¹, -SR¹⁰, - SOR¹⁰, -SO₂R¹⁰, -SO₂NR¹⁰R¹¹, -NR¹⁰SO₂NR¹¹R¹², - OSO₂NR¹⁰R¹¹ et un pseudo-halogène ; et
R¹⁰, R¹¹ et R¹² représentent respectivement, indépendamment les uns des autres, un atome d'hydrogène ou un radical choisi dans le groupe constitué des groupes éventuellement mono- ou poly-substitués alkyle en C₁ à C₈, alcényle en C₂ à C₈, alcinyle en C₂ à C₈, cycloalkyle en C₃ à C₁₀, aryle, hétérocyclyle et hétéroaryle, dans lequel le/les substituant(s) peuvent être identiques ou différents et sont choisis dans le groupe comprenant un atome d'halogène, -NH₂, -OH et un pseudo-halogène ;
dans laquelle le groupe aryle se rapporte à des cycles monocycliques ou bicycliques comportant 6 à 12 atomes de carbone ;
dans laquelle les groupes hétéroaryle désignent des cycles mono- ou bicycliques qui, à la place d'un ou plusieurs atomes de carbone, contiennent un ou plusieurs hétéroatomes identiques ou différents, choisis parmi des atomes d'azote, de soufre et d'oxygène,
dans laquelle le groupe hétérocyclyle se rapporte à des cycles bicycliques saturés ou insaturés, non aromatiques, mono-, bicycliques ou pontés, comprenant 5 à 12 atomes de carbone, qui contiennent, à la place d'un ou plusieurs atomes de carbone, des hétéroatomes choisis parmi des atomes d'azote, de soufre et d'oxygène ;
éventuellement sous la forme de leurs tautomères, leurs racémates, leurs énantiomères, leurs diastéréomères et leurs mélanges, et éventuellement leurs sels d'addition d'acides pharmacologiquement inoffensifs.

2. Composés selon la revendication 1, dans lesquels
X représente -NR^{1a} ou un atome d'oxygène, et
A est choisi parmi les formules (i) ou (ii) 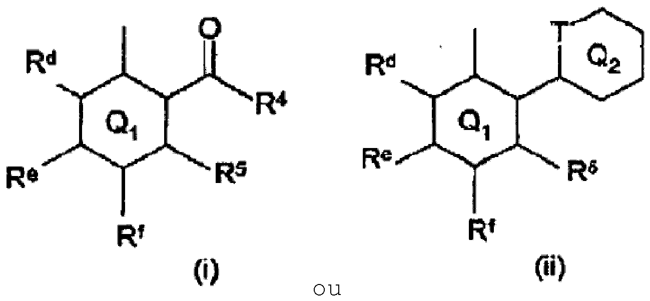 et
Q¹ représente des composés aryle mono- ou bicycliques ; et
Q² représente des composés hétéroaryle monocycliques ; et
T représente N, 0 ou S, et
R¹ et R^{1a} représentent un atome d'hydrogène; et
R³ représente la formule (iii) 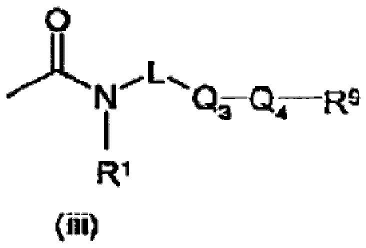
et les radicaux restants sont tels que définis précédemment.

3. Composés selon la revendication 1 ou 2, dans lesquels
Y représente CH ; et
Q₁ représente des composés aryle monocycliques
et les radicaux restants sont tels que définis précédemment.

4. Composés selon la revendication 1 à 3, dans lesquels
R^{c} est un radical choisi dans le groupe comprenant un atome d'hydrogène, -F, -Cl, un groupe méthyle et éthyle
et les radicaux restants sont tels que définis précédemment.

5. Composés selon la revendication 1 à 4, dans lesquels
R^{a} et R^{b} représentent respectivement, indépendamment les uns des autres, un atome d'hydrogène ou un radical éventuellement mono- ou poly-substitué choisi dans le groupe constitué d'un groupe alkyle en C₁ à C₂, alcényle en C₁ à C₂, alcinyle en C₂, cycloalkyle en C₃ à C₆, aryle, hétérocyclyle et hétéroaryle, dans lequel le/les substituants peuvent être identiques ou différents et sont choisis dans le groupe comprenant un atome d'hydrogène, d'halogène, -NO₂, -OR⁶, -C(=O)R⁶, - C(=O)OR⁶, -C(=O)NR⁶R⁷, -NR⁶R⁷, -NR⁶C(=O)R⁷, - NR⁶C(=O)OR⁷, -NR⁶C (=O)NR⁷R⁸, -NR⁶SO₂R⁷, -N=CR⁶R⁷, - SR⁶, -SOR⁶, -SO₂R⁶, -SO₂NR⁶R⁷, -NR⁶, -SO₂NR⁷R⁸, - OSO₂NR⁷R⁸ et un pseudo-halogène ;
et les radicaux restants sont tels que définis précédemment.

6. Composés selon la revendication 1 à 5, dans lesquels
R^{a} et R^{b} représentent un atome d'hydrogène
et les radicaux restants sont tels que définis précédemment.

7. Composés, ou leurs sels pharmaceutiquement efficaces, selon les revendications 1 à 6, pour leur utilisation comme médicaments.

8. Composés, ou leurs sels pharmaceutiquement efficaces, selon les revendications 1 à 6, pour la production d'un médicament ayant un effet antiprolifératif.

9. Composés, ou leurs sels pharmaceutiquement efficaces, selon les revendications 1 à 6, pour la production d'un médicament ayant un effet antiprolifératif et un mécanisme actif sélectif, inhibiteur de kinases.

10. Composés, ou leurs sels pharmaceutiquement efficaces, selon les revendications 1 à 6, pour la production d'un médicament ayant un effet antiprolifératif et un mécanisme actif inhibiteur de PLK.

11. Composition pharmaceutique contenant, comme substance active, un ou plusieurs composés de formule générale (1) selon l'une des revendications 1 à 6, ou leurs sels physiologiquement acceptables éventuellement en combinaison avec des adjuvants et/ou véhicules habituels.

12. Utilisation d'un composé selon la revendication 1 à 6, pour la production d'un médicament pour le traitement et/ou la prévention des cancers, des infections, des inflammations et des maladies auto-immunes.

13. Préparation pharmaceutique comprenant un composé de formule générale (1) 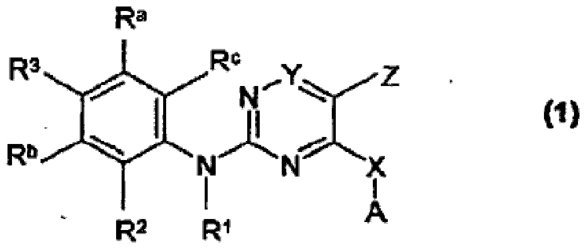 dans laquelle
X représente -NR^{1a}, 0 ou S, et
Y représente CH et
Z représente un groupe fluoroalkyle en C₁ à C₃ et
A est choisi parmi les formules (i) ou (ii) 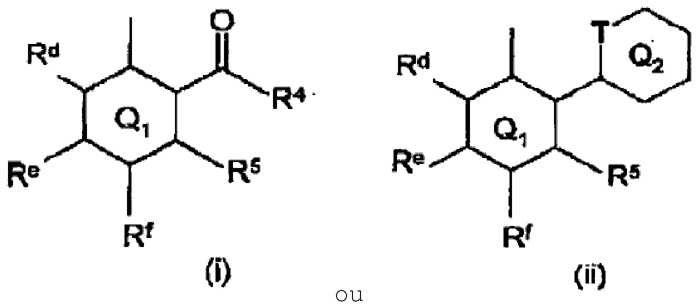 et
Q¹ représente des composés aryle mono- ou bicycliques ; et
Q² représente des composés hétéroaryle mono- ou bicycliques ; et
T représente N, 0 ou S, et
R¹ et R^{1a} représentent un atome d'hydrogène ou un groupe méthyle, et
R² représente un radical choisi dans le groupe constitué de -Cl, -Br, -I, -OR⁶, -C(=O)R⁶, C(=O)NR⁶R⁷, -NR⁶R⁷, -NR⁶C(=O)R⁷, -NR⁶SO₂R⁷, -N=CR⁶R⁷, -SR⁶, -SOR⁶, -SO₂R⁶, -SO₂NR⁶R⁷ et un pseudo-halogène ou un radical éventuellement mono- ou poly-substitué choisi dans le groupe constitué d'un groupe alkyle en C₁ à C₆, alcényle en C₂ à C₆, alcinyle en C₂ à C₆, cycloalkyle en C₃ à C₆, aryle, hétérocyclyle et hétéroaryle, dans lequel le/les substituants peuvent être identiques ou différents et sont choisis dans le groupe constitué d'un atome d'halogène, -NO₂, -OR⁶, -C(=O)R⁶, -C(=O)OR⁶, -C(=O)NR⁶R⁷, -NR⁶R⁷, -NR⁶C(=O)R⁷, -NR⁶C (=O)OR⁷, - NR⁶C(=O)NR⁷R⁸, -NR⁶SO₂R⁷, -N=CR⁶R⁷, -SR⁶, -SOR⁶, -SO₂R⁶, -SO₂NR⁶R⁷, -NR⁶SO₂NR⁷R⁸, - OSO₂NR⁷R⁸ et un pseudo-halogène ; et
R^{a}, R^{b}, R^{c}, R^{d}, R^{e} et R^{f} représentent respectivement, indépendamment les uns des autres, un radical choisi dans le groupe constitué d'un atome d'hydrogène, d'halogène, -NO₂, -OR⁶, -C(=O)R⁶, - C(=O)OR⁶, -C(=O)NR⁶R⁷, -NR⁶R⁷, -NR⁶C(=O)R⁷, - NR⁶C(=O)OR⁷, -NR⁶C (=O)NR⁷R⁸, -NR⁶SO₂R⁷, -N=CR⁶R⁷, - SR⁶, -SOR⁶, -SO₂R⁶, -SO₂NR⁶R⁷, -NR⁶SO₂NR⁷R⁸, - OSO₂NR⁷R⁸ et un pseudo-halogène ; ou un radical éventuellement mono- ou poly-substitué choisi dans le groupe constitué d'un groupe alkyle en C₁ à C₆, alcényle en C₂ à C₆, alcinyle en C₂ à C₆, cycloalkyle en C₃ à C₆, aryle, hétérocyclyle et hétéroaryle, dans lequel le/les substituants peuvent être identiques ou différents et sont choisis dans le groupe constitué d'un atome d'hydrogène, d'halogène, -NO₂, -OR⁶, -C(=O)R⁶, -C(=O)OR⁶, -C(=O)NR⁶R⁷, -NR⁶R⁷, -NR⁶C(=O)R⁷, - NR⁶C(=O)OR⁷, -NR⁶C(=O)NR⁷R⁸, -NR⁶SO₂R⁷, -N=CR⁶R⁷, - SR⁶, -SOR⁶, -SO₂R⁶, -SO₂NR⁶R⁷, -NR⁶SO₂NR⁷R⁸, - OSO₂NR⁷R⁸ et un pseudo-halogène ; et
R³ est choisi parmi les formules (iii) à (ix), 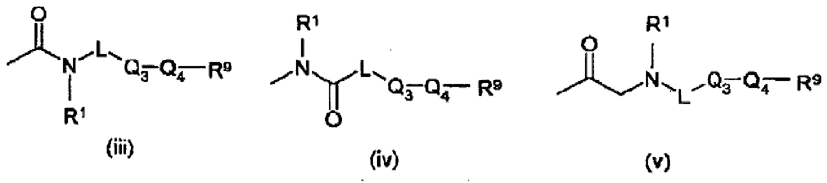 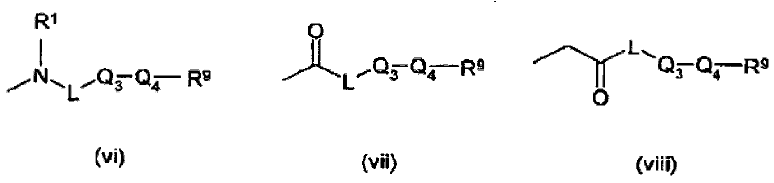 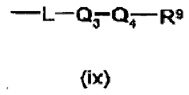 et
R⁴ représente un radical choisi dans le groupe constitué d'un atome d'hydrogène, -OR⁶, -C(=O)R⁶, -C(=O)OR⁶, -C(=O)NR⁶R⁷, -NR⁶R⁷, -NR⁶C(=O)R⁷, - NR⁶C(=O)OR⁷, -NR⁶C (=O)NR⁷R⁸, -NR⁶SO₂R⁷, -N=CR⁶R⁷, - SR⁶, -SOR⁶, -SO₂R⁶, -SO₂NR⁶R⁷, -NR⁶SO₂NR⁷R⁸, - OSO₂NR⁷R⁸ et un pseudo-halogène,
ou un radical choisi dans le groupe constitué des groupes éventuellement mono- ou poly-substitués alkyle en C₁ à C₈, alcényle en C₂ à C₁₀, alcinyle en C₂ à C₁₀, cycloalkyle en C₃ à C₈, aryle, hétérocyclyle et hétéroaryle, dans lequel le/les substituant(s) peuvent être identiques ou différents et sont choisis dans le groupe constitué d'un groupe alkyle en C₁ à C₈, un atome d'halogène, -NO₂, -OR⁶, -C(=O)R⁶, -C(=O)OR⁶, - C(=O)NR⁶R⁷, -NR⁶R⁷, -NR⁶C(=O)R⁷, -NR⁶C (=O)OR⁷, - NR⁶C(=O)NR⁷R⁸, -NR⁶SO₂R⁷, -N=CR⁶R⁷, -SR⁶, -SOR⁶, - SO₂R⁶, -SO²NR⁶R⁷, -NR⁶SO₂NR⁷R⁸, OSO₂NR⁷R⁸ et un pseudo-halogène ; et
R⁵ représente un atome d'hydrogène, d'halogène, -CF₃, un groupe alkyle en C₁ à C₃ ou -OR⁶ ; et
R⁶, R⁷ et R⁸ représentent respectivement, indépendamment les uns des autres, un atome d'hydrogène ou un radical choisi dans le groupe constitué des groupes éventuellement mono- ou poly-substitués alkyle en C₁ à C₅, alcényle en C₂ à C₅, alcinyle en C₂ à C₅, cycloalkyle en C₃ à C₁₀, aryle, hétérocyclyle et hétéroaryle, dans lequel le/les substituants peuvent être identiques ou différents et sont choisis dans le groupe constitué d'un groupe cycloalkyle en C₃ à C₁₀, aryle, hétérocyclyle, hétéroaryle, un atome d'halogène, -NO₂, -OR¹⁰, -C(=O)R¹⁰, -C(=O)OR¹⁰, -C(=O)NR¹⁰R¹¹, - NR¹⁰R¹¹, -NR¹⁰C(=O)R¹¹, -NR¹⁰C(=O)OR¹¹, - NR¹⁰C(=O)NR¹¹R¹², -NR¹⁰C(=O)ONR¹¹R¹², -NR¹⁰SO₂R¹¹, - N=CR¹⁰R¹¹, -SR¹⁰, -SOR¹⁰, -SO₂R¹⁰, -SO₂NR¹⁰R¹¹, - NR¹⁰SO₂NR¹¹R¹², -OSO₂NR¹⁰R¹¹ et un pseudo-halogène ; et
L représente une liaison ou un radical choisi dans le groupe constitué des groupes éventuellement mono- ou poly-substitués alkyle en C₁ à C₁₆, alcényle en C₂ à C₁₆, alcinyle en C₂ à C₁₆, cycloalkyle en C₃ à C₁₀, aryle, hétérocyclyle et hétéroaryle, dans lequel le/les substituant(s) peuvent être identiques ou différents et sont choisis dans le groupe constitué d'un atome d'halogène, -NO₂, -OR¹⁰, -C(=O)R¹⁰, -C(=O)OR¹⁰, - C(=O)NR¹⁰R¹¹, -NR¹⁰R¹¹, -NR¹⁰COR¹¹, -NR¹⁰C(=O)OR¹¹, - NR¹⁰C(=O)NR¹¹R¹², -NR¹⁰C(=O)ONR¹¹R¹², -NR¹⁰SO₂R¹¹, - N=CR¹⁰R¹¹, -SR¹⁰, -SOR¹⁰, -SO₂R¹⁰, -SO₂NR¹⁰R¹¹, - NR¹⁰SO₂NR¹¹R¹², -OSO₂NR¹⁰R¹¹ et un pseudo-halogène ; et
Q₃ et Q₄ représentent, indépendamment l'un de l'autre, une liaison ou un radical choisi dans le groupe constitué d'un groupe hétérocyclyle mono- ou bicyclique éventuellement mono- ou poly-substitué, dans lequel le/les substituant(s) peuvent être identiques ou différents et sont choisis dans le groupe constitué d'un groupe méthyle, éthyle, un atome d'halogène, -NH₂, -OH et un pseudo-halogène ; et
R⁹ représente un radical choisi dans le groupe constitué des groupes éventuellement mono- ou poly-substitués alkyle en C₁ à C₁₆, alcényle en C₂ à C₁₆, alcinyle en C₂ à C₁₆, cycloalkyle en C₃ à C₁₀, aryle, hétérocyclyle et hétéroaryle, dans lequel le/les substituant(s) peuvent être identiques ou différents et sont choisis dans le groupe constitué d'un atome d'halogène, -NO₂, -OR¹⁰, C(=O)R¹⁰, -C(=O)OR¹⁰, -C(=O)NR¹⁰R¹¹, -NR¹⁰R¹¹, - NR¹⁰COR¹¹, -NR¹⁰C(=O)OR¹¹, -NR¹⁰C(=O)NR¹¹R¹², - NR¹⁰C(=O)ONR¹¹R¹², -NR¹⁰SO₂R¹¹, -N=CR¹⁰R¹¹, -SR¹⁰, - SOR¹⁰, -SO₂R¹⁰, -SO₂NR¹⁰R¹¹, -NR¹⁰SO₂NR¹¹R¹², - OSO₂NR¹⁰R¹¹ et un pseudo-halogène ; et
R¹⁰ R¹¹ et R¹² représentent respectivement, indépendamment les uns des autres, un atome d'hydrogène ou un radical choisi dans le groupe constitué des groupes éventuellement mono- ou poly-substitués alkyle en C₁ à C₈, alcényle en C₂ à C₈, alcinyle en C₂ à C₈, cycloalkyle en C₃ à C₁₀, aryle, hétérocyclyle et hétéroaryle, dans lequel le/les substituant(s) peuvent être identiques ou différents et sont choisis dans le groupe constitué d'un atome d'halogène, -NH₂, -OH et un pseudo-halogène ;
dans laquelle le groupe aryle se rapporte à des cycles monocycliques ou bicycliques comportant 6 à 12 atomes de carbone ;
dans laquelle les groupes hétéroaryle désignent des cycles mono- ou bicycliques qui, à la place d'un ou plusieurs atomes de carbone, contiennent un ou plusieurs hétéroatomes identiques ou différents, choisis parmi des atomes d'azote, de soufre et d'oxygène,
dans laquelle le groupe hétérocyclyle se rapporte à des cycles bicycliques saturés ou insaturés, non aromatiques, mono-, bicycliques ou pontés, comprenant 5 à 12 atomes de carbone, qui contiennent, à la place d'un ou plusieurs atomes de carbone, des hétéroatomes choisis parmi des atomes d'azote, de soufre et d'oxygène ;
éventuellement sous la forme de leurs tautomères, leurs racémates, leurs énantiomères, leurs diastéréomères et leurs mélanges, et éventuellement leurs sels d'addition d'acides pharmacologiquement inoffensifs, et
au moins une autre substance active choisie dans le groupe comprenant des substances actives cytostatiques, des substances actives cytotoxiques, des stéroïdes et des anticorps, éventuellement sous la forme de leurs tautomères, leurs racémates, leurs énantiomères, leurs diastéréomères et leurs mélanges, et éventuellement leurs sels d'addition acide pharmacologiquement inoffensifs.
